(19) Europäisches Patentamt
European Patent Office
Office européen des brevets

(11) Veröffentlichungsnummer: **0 161 632 B1**

# (12) EUROPÄISCHE PATENTSCHRIFT

(45) Veröffentlichungstag der Patentschrift: **10.04.91**

(21) Anmeldenummer: **85105675.4**

(22) Anmeldetag: **09.05.85**

Die Akte enthält technische Angaben, die nach dem Eingang der Anmeldung eingereicht wurden und die nicht in dieser Patentschrift enthalten sind.

(51) Int. Cl.[5]: **C07D 487/04**, C07D 487/10, A61K 31/44, C07D 209/34, C07D 209/42, C07D 209/96, C07D 401/12, //(C07D487/12, 235:00,209:00)

## (54) Neue Pyrrolo-Benzimidazole, Verfahren zu ihrer Herstellung und diese Verbindungen enthaltende Arzneimittel sowie Zwischenprodukte.

(30) Priorität: **12.05.84 DE 3417643**
**20.12.84 DE 3446417**

(43) Veröffentlichungstag der Anmeldung:
**21.11.85 Patentblatt 85/47**

(45) Bekanntmachung des Hinweises auf die Patenterteilung:
**10.04.91 Patentblatt 91/15**

(84) Benannte Vertragsstaaten:
**AT BE CH DE FR GB IT LI LU NL SE**

(56) Entgegenhaltungen:
**EP-A- 0 098 448**
**GB-A- 1 158 532**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Hölck, Jens-Peter, Dr.rer.nat.**
**Ph. Brunnemer Weg 33**
**W-6800 Mannheim 31(DE)**
Erfinder: **Mertens, Alfred, Dr.**
**In der Schanz 31**
**W-6905 Schriesheim(DE)**
Erfinder: **Kampe, Wolfgang, Dr.rer.nat.**
**Zedernstrasse 49**
**W-6805 Heddesheim(DE)**
Erfinder: **Müller-Beckmann, Bernd, Dr.**
**Hochgewanne 46**
**W-6718 Grünstadt(DE)**
Erfinder: **Sponer, Gisbert, Dr. med. vet.**
**Lessingstrasse 13**
**W-6941 Laudenbach(DE)**
Erfinder: **Strein, Klaus, Prof. Dr. Dr.**
**Eichenstrasse 15**
**W-6944 Hemsbach(DE)**

EP 0 161 632 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Pyrrolo-Benzimidazole der allgemeinen Formel I

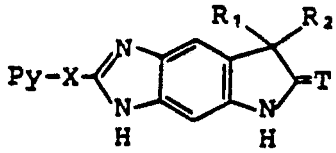

(I),

in welcher

- $R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,
- $R_2$ ein Wasserstoffatom, eine $C_1$-$C_6$-Alkyl-, $C_2$-$C_6$-Alkenyl-, oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe bedeutet oder mit $R_1$ zusammen eine $C_3$-$C_8$-Cycloalkylengruppe darstellt oder $R_1$ und $R_2$ zusammen eine $C_2$-$C_6$-Alkyliden- oder $C_3$-$C_8$-Cycloalkylidengruppe bilden,
- X einen Valenzstrich, eine $C_1$-$C_4$-Alkylengruppe oder die Vinylengruppe bedeutet,
- T gleich Sauerstoff oder Schwefel bedeutet,
- Py einen 2-, 3- oder 4-Pyridylrest darstellt, der gegebenenfalls am Ringheteroatom ein Sauerstoffatom trägt und/oder durch eine oder mehrere $C_1$-$C_6$-Alkyl-, $C_1$-$C_6$-Alkoxy-, Hydroxy-, Cyano- oder Nitrogruppen, sowie durch Halogen substituiert sein kann, deren Tautomere und deren physiologisch verträgliche Salze anorganischer und organischer Säuren und Verfahren zu ihrer Herstellung, sowie diese Verbindungen enthaltende Arzneimittel.

Da die Verbindungen der allgemeinen Formel I für den Fall, daß $R_1$ nicht gleich $R_2$ ist, ein asymmetrisches Kohlenstoffatom besitzen, sind auch Gegenstand der Erfindung die optisch aktiven Formen und racemischen Gemische dieser Verbindungen. Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenaggregation und verbessern die Mikrozirkulation.

Aus EP-A-0 098 448 sind Benzimidazol-Derivate bekannt, die in 2-Stellung durch Phenylgruppen substituiert sind. Die dort beschriebenen Verbindungen beeinflussen die Kontraktilität des Herzens. Sie unterscheiden sich jedoch strukturell von den Verbindungen der vorliegenden Erfindung im wesentlichen dadurch, daß sie ausschließlich bicyclische Derivate darstellen, während es sich bei den erfindungsgemäßen Verbindungen um tricyclische Derivate (Pyrrolo-Benzimidazole) handelt, die in 2-Stellung des Imidazolsystems durch die heterocyclische Gruppe Py-X-substituiert sind. Aus GB-1,158,532, FR-1,529,901 und Helv. Chim. Acta 20 , 373-386 (1937) sind Verbindungen bzw. die Herstellung von Verbindungen bekannt, die als Zwischenprodukte für die Herstellung der erfindungsgemäßen Verbindungen eingesetzt werden können.

In den nachstehend aufgeführten Formeln, insbesondere Formel I, können die Substituenten $R_1$ und $R_2$ gleich oder verschieden sein und Wasserstoff, eine geradkettige oder verzweigte Alkyl- oder Alkenylgruppe mit 1-6 bzw. 2-6 Kohlenstoffatomen, eine Cyanogruppe oder eine durch eine Hydroxy-, Alkyl-, Alkoxy-, Amino-, Alkylamino-, Dialkylamino-, oder Hydrazinogruppe substituierte Carbonylgruppe darstellen, wobei jeder der vorgenannten Alkylteile geradkettig oder verzweigt aus 1-6 bzw. 2-6 Kohlenstoffatomen zusammengesetzt sein kann. Insbesondere kommen jedoch Wasserstoff, die Methyl-, Ethyl-, Allyl-, Cyan-, Carboxy-, Acetyl-, Propionyl-, Methoxycarbonyl-, Ethoxycarbonyl-, Aminocarbonyl-, Methylaminocarbonyl-, Dimethylaminocarbonyl- und Hydrazinocarbonylgruppe für $R_1$ und $R_2$ in Frage.

Stellt nur $R_2$ ein Wasserstoffatom dar, so ist $R_1$ eine geradkettige Alkylgruppe mit 1-6 Kohlenstoffatomen oder eine verzweigte Alkylgruppe, eine Cycloalkyl- oder Alkenylgruppe mit 3-7 Kohlenstoffatomen, eine Cyanogruppe oder eine durch eine Alkyl-, Alkoxy-, Amino-, Alkylamino-, Dialkylamino- oder Hydrazinogruppe substituierte Carbonylgruppe. Bevorzugt in diesem Sinne sind die Methyl-, Ethyl-, Isopropyl-, Isobutyl-, Pentyl-, Cyclopentyl-, Cyclohexyl-, Allyl-, Cyan-, Acetyl-, Propionyl,-Methoxycarbonyl-, Ethoxycarbonyl-, Aminocarbonyl-, Methylaminocarbonyl-, Dimethylaminocarbonyl- und Hydrazinocarbonylgruppe.

$R_1$ und $R_2$ koennen zusammen mit dem C-Atom, an das sie gebunden sind, auch einen Cycloalkylring mit 3-8 Kohlenstoffatomen bilden, vorzugsweise handelt es sich dabei um die Spirocyclopropyl-,

Spirocyclobutyl-, Spirocyclopentyl- und Spirocyclohexylgruppe.

$R_1$ und $R_2$ können zusammen auch eine $C_2$-$C_6$-Alkyliden- oder $C_3$-$C_8$-Cycloalkylidengruppe bilden, vorzugsweise handelt es sich dabei um die Isopropylidengruppe.

Alkyl- oder Alkoxy-Substituenten des Pyridin-Rings koennen 1-6, vorzugsweise 1-4 C-Atome enthalten. Bevorzugt ist Methyl und Ethyl bzw. Methoxy und Ethoxy. Unter Halogen ist Fluor, Chlor und Brom, vorzugsweise Chlor zu verstehen.

Stellt Py einen 2-, 3- oder 4-Pyridylrest dar, der gegebenenfalls am Ringheteroatom ein Sauerstoffatom traegt und/oder durch eine oder mehrere Alkyl-, Alkoxy-, Hydroxy-, Cyano- oder Nitrogruppen, sowie durch Halogen substituiert sein kann und x ist ein Valenzstrich, so sind in diesem Sinne bevorzugt der 2-Pyridyl-, 2-(N-Oxy-pyridyl)-, 2-(5-n-Butyl-pyridyl)-, 3-Pyridyl-, 3-(N-Oxy-pyridyl)-, 3-(6-Methyl-pyridyl)-, 3-(6-Cyan-pyridyl)-, 3-(6-Nitro-pyridyl)-, 3-(6-Hydroxy-pyridyl)-, 3-(2-Methoxy-6-methyl-pyriayl)-, 4-Pyridyl-, 4-(N-Oxy-pyridyl)-, 4-(2-Methyl-pyridyl)-, 4-(2-Ethyl-pyridyl)-, 4-(2-Hydroxy-pyridyl)-, 4-(2-Methoxy-pyridyl)-, 4-(2-Nitro-pyridyl)-, 4-(2-Chlor-pyridyl)- und der 4-(3-Hydroxy-pyridyl)-rest.

Bedeutet X eine $C_1$-$C_4$ Alkylengruppe oder die Vinylengruppe und Py einen 2-, 3-oder 4-Pyridylrest, so sind in diesem Sinne besonders bevorzugt der 3-Pyridyl-methyl-, 3-Pyridyl-ethyl-, 3-Pyridyl-vinyl-, 4-Pyridyl-methyl-, 4-Pyridyl-ethyl und 4-Pyridyl-vinylrest.

Bevorzugte Pyrrolo-Benzimidazole der allgemeinen Formel I sind Verbindungen, in denen $R_1$ Wasserstoff, Methyl, Ethyl, 2-Propyl, 2-Methyl-propyl oder Cyclopentyl, $R_2$ Wasserstoff, Methyl, Ethyl, Ethoxycarbonyl oder Hydrazinocarbonyl, $R_1$ und $R_2$ zusammen mit dem C-Atom, an das sie gebunden sind, einen Cyclopentan-Ring oder zusammen einem Isopropyliden-Rest bilden, X eine Valenzbindung, eine Methylen-, Ethylen- oder Vinylengruppe, T Sauerstoff oder Schwefel und Py einen Pyridyl-N-oxid-Rest oder einen Pyridyl-Rest bedeuten, der ein- oder mehrfach durch Hydroxyl, Methoxy, Methyl oder Halogen substituiert sein kann.

Besonders bevorzugt sind Verbindungen der Formel I, in denen T Sauerstoff, X eine Valenzbindung, $R_1$ Wasserstoff oder Methyl, $R_2$ Methyl, Ethyl oder Ethoxycarbonyl und Py einen Pyridin-N-oxid-Rest oder einen Pyridyl-Rest darstellen, der durch Hydroxy oder Methyl substituiert sein kann.

Die Herstellung der Verbindungen der allgemeinen Formel I erfolgt zunaechst in der Weise, daß man entweder

a) o-Cyanbenzylnitril II

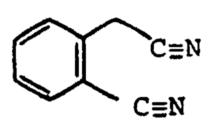

(II),

mit einer Verbindung der allgemeinen Formel III

$$R_1-Z \qquad (III)$$

oder IV

$$Z-R_3-Z \qquad (IV),$$

in denen $R_1$ die angegebene Bedeutung hat, $R_3$ eine $C_2$-$C_6$-Alkylengruppe ist und Z eine abspaltbare Gruppe darstellt, zu Verbindungen der allgemeinen Formel V alkyliert,

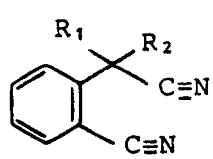

(V),

in welcher $R_1$ und $R_2$ die angegebene Bedeutung haben und anschließend in saurem Medium zu

Verbindungen der allgemeinen Formel VI cyclisiert,

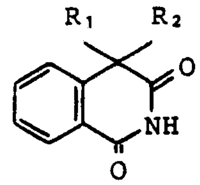

(VI),

in welcher $R_1$ und $R_2$ die oben angegebene Bedeutung haben,
oder
b) Isochinolin-1,3-dion mit einer Verbindung der allgemeinen Formel III oder IV zu einer Verbindung der allgemeinen Formel VI alkyliert
oder
c) Isochinolin-1,3-dion mit einer Verbindung der allgemeinen Formel VII

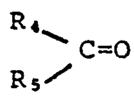

(VII),

in welcher $R_4$ und $R_5$ Wasserstoff und/oder Alkylgruppen sind oder $R_4$ zusammen mit $R_5$ eine $C_3$-$C_7$-Cycloalkylengruppe bildet, in Gegenwart einer Base zu Verbindungen der allgemeinen Formel VIII kondensiert

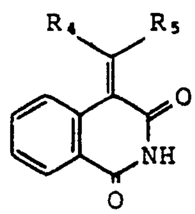

(VIII),

in denen $R_4$ und $R_5$ die oben angegebene Bedeutung besitzt, und gegebenenfalls anschließend Verbindungen der allgemeinen Formel VIII durch katalytische Hydrierung in Verbindungen der allgemeinen Formel VI ueberfuehrt, in denen $R_1$ oder $R_2$ gleich Wasserstoff ist.

Eine abspaltbare Gruppe Z der Verbindungen der Formel III bzw. IV ist in der Regel ein Halogenatom, wobei Chlor, Brom und Jod bevorzugt sind.

Die Cyclisierung einer Verbindung der Formel V findet in saurem Medium statt, vorzugsweise in Gegenwart von Mineralsaeuren wie Salzsaeure oder Schwefelsaeure.

Die Kondensation von Isochinolin-1,3-dion mit einer Verbindung der Formel VII geschieht in Gegenwart einer Base, vorzugsweise KOH oder NaOH.

Die katalytische Hydrierung von Verbindungen der Formel VIII geschieht vorzugsweise mit Pd/C in alkoholischen Medien.

Verbindungen der Formel VI sowie die voranstehend aufgefuehrten Verfahrensschritte sind in der deutschen Patentanmeldung p 34 10 168.3 beschrieben.

Die so erhaltenen Verbindungen der allgemeinen Formel VI werden in bekannter Weise durch Nitrieren in 7-Stellung in Verbindungen der allgemeinen Formel IX ueberfuehrt

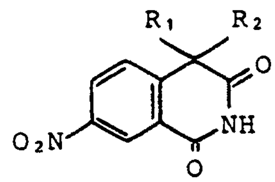

(IX),

aus denen durch Hoffmann-Abbau mit Hypohalogenid nach N.A. Joensson und P. Moses, Acta Chem. Scand. B 28, 225-232 (1974) Verbindungen der allgemeinen Formel X erhalten werden koennen,

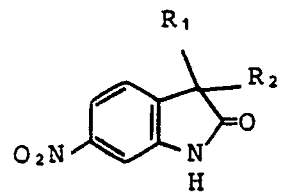

(X),

in denen $R_1$ und $R_2$ die oben angegebene Bedeutung haben.

Durch Reduktion der Nitrogruppe in eine Aminogruppe erhaelt man Verbindungen der allgemeinen Formel XI

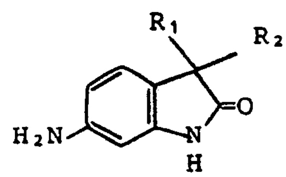

(XI),

in denen $R_1$ und $R_2$ die oben angegebene Bedeutung haben, die sich auch in bekannter Weise aus entsprechend substituierten Derivaten der 2,4-Diaminophenylessigsaeure durch Ringschluß (siehe dazu: R.C. Elderfield (Ed.), P.L. Julian, E.W. Meyer, H.C. Printy, Heterocycl. Comp. Vo. 3, 126-186, John Wiley & Sons 1952, New York) erhalten lassen.

Die so erhaltenen Verbindungen der allgemeinen Formel XI werden in bekannter Weise durch Acetylierung der Aminogruppe in 6-Stellung, Nitrierung in 5-Stellung, Abspaltung der Schutzgruppe und Reduktion der Nitrogruppe in 5-Stellung in ein 5,6-Diamino-indolin-2-on der allgemeinen Formel XII

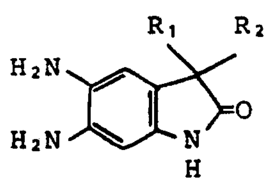

(XII),

in der $R_1$ und $R_2$ die oben angegebene Bedeutung haben, ueberfuehrt.

Die Herstellung der Verbindungen der allgemeinen Formel I kann auch in der Weise erfolgen, daß man

d) Verbindungen der allgemeinen Formel XII erhaelt, indem man ein am Stickstoff geschuetztes Oxindol-Derivat der allgemeinen Formel XIII

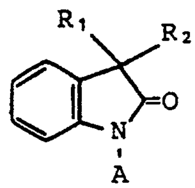

(XIII),

in der $R_1$ und $R_2$ gleich Wasserstoff bedeuten und A eine Schutzgruppe ist, z.B. eine Acetyl- oder Benzylschutzgruppe, mit Verbindungen der allgemeinen Formel III oder IV mit der oben angegebenen Bedeutung zu anderen Verbindungen der allgemeinen Formel XIII alkyliert, in der $R_1$ und $R_2$ die oben angegebene Bedeutung besitzen, jedoch nicht $R_1 = R_2 = H$ ist. Nach Abspaltung einer Benzylschutzgruppe z.B. durch Natrium in fluessigem Ammoniak oder einer Acetylschutzgruppe in saurem oder basischem Medium erhaelt man ein Indolin-2-on Derivat der allgemeinen Formel XIII in der A gleich Wasserstoff ist und $R_1$ und $R_2$ die oben angegebene Bedeutung besitzen, wobei jedoch nicht $R_1 = R_2$ = Wasserstoff bedeuten.

Die so erhaltenen Verbindungen der allgemeinen Formel XIII werden in bekannter Weise durch Nitrierung in 5-Stellung, anschließendes Reduzieren der Nitrogruppe zur Aminofunktion, Acetylierung der Aminogruppe in 5-Stellung, Nitrierung in 6-Stellung, Abspaltung der Schutzgruppe und Reduktion der Nitrogruppe in 5-Stellung in ein entsprechendes 5,6-Diamino-indolin-2-on Derivat der allgemeinen Formel XII mit der oben angefuehrten Bedeutung ueberfuehrt.

Das erfinderische Verfahren zur Herstellung von Verbindungen der Formel I erfolgt dadurch, daß man eine Verbindung der Formel XII mit einer Verbindung der Formel XIV

Py-X-CO-Y (XIV),

in der Py und X die angegebene Bedeutung besitzen und Y entweder Wasserstoff oder ein leicht abspaltbarer Rest darstellt, umsetzt und die erhaltenen Verbindungen zu einer Verbindung der Formel I oder dessen tautomeren Form cyclisiert, und eine erfindungsgemaeß erhaltene Verbindung der Formel I gewuenschtenfalls in eine andere Verbindung der Formel I oder dessen Tautomer umwandelt und/oder eine erhaltene Verbindung der allgemeinen Formel I und dessen Tautomer in ein physiologisch vertraegliches Saeureadditionssalz mit einer anorganischen oder organischen Saeure ueberfuehrt.

Unter Verbindungen der Formel XIV versteht man insbesondere Aldehyde, sowie Saeurehalogenide wie Saeurechloride, Carbonsaeureester wie Methyl- und Ethylester und andere aktivierte Carbonsaeurederivate, wie z.B. Anhydride.

Ist die Verbindung der Formel XIV ein Aldehyd findet die Umsetzung zur Schiff'schen Base mit Verbindungen der allgemeinen Formel XII vorzugsweise in alkoholischem Medium statt, die anschließende Cyclisierung und Oxidation zu den Verbindungen der allgemeinen Formel I erfolgt durch Erwaermen des Reaktionsansatzes zum Ruecktluß in Gegenwart von Luftsauerstoff und katalytischen Mengen Saeure, wie z.B. Toluolsulfonsaeure.

Ist die Verbindung der allgemeinen Formel XIV ein Carbonsaeurederivat, findet die Umsetzung mit Verbindung der allgemeinen Formel XII zum Amid in inerten Loesungsmitteln, vorzugsweise Methylenchlorid statt und die anschließende Cyclisierung zu Verbindungen der allgemeinen Formel I wird in saurem Medium, vorzugsweise in Gegenwart von Mineralsaeuren wie $H_2SO_4$ oder HC1 in alkoholischer Loesung, vorgenommen.

Verbindungen der allgemeinen Formel I werden auch erhalten, in dem man

e) ausgehend von Verbindungen der Formel XV

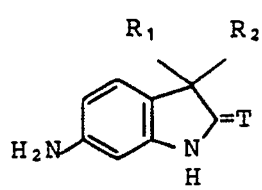

(XV)

oder XVI

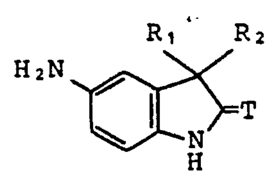

(XVI),

in denen $R_1$, $R_2$ und T die oben angegebene Bedeutung besitzen, durch Umsetzung mit Verbindungen

der allgemeinen Formel XIV, in denen Y einen leichtabspaltbaren Rest darstellt, nach allgemein bekannter Weise Verbindungen der Formel XVII

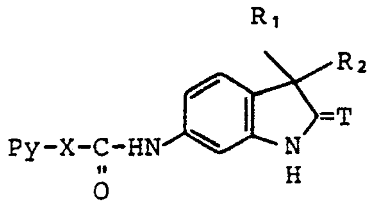

(XVII),

oder XVIII

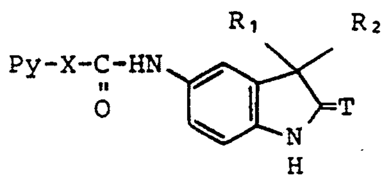

(XVIII),

in denen $R_1$, $R_2$, T, Py und X die oben angegebenen Bedeutungen besitzen, erhaelt und in bekannter Weise durch Nitrierung in Verbindungen der allgemeinen Formel XIX

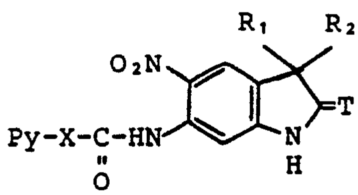

(XIX),

oder XX

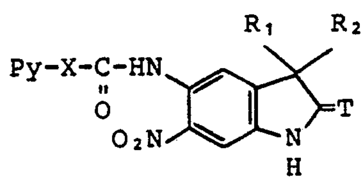

(XX),

in denen $R_1$, $R_2$, T, Py und X die oben angegebenen Bedeutungen besitzen, ueberfuehrt.

Nach Hydrierung der Nitrogruppe in den Verbindungen der allgemeinen Formel XIX bzw. XX zu den entsprechenden Aminverbindungen XXI

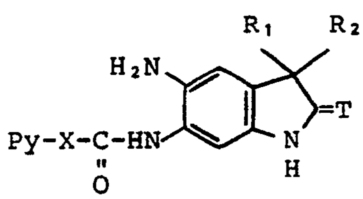

(XXI),

oder XXII

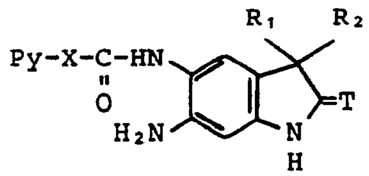 (XXII),

in denen $R_1$, $R_2$, T, Py und X die oben angegebene Bedeutung besitzen, erhaelt man durch Cyclisierung die gewuenschten Verbindungen der allgemeinen Formel I.

Verbindungen der Formel XVI erhaelt man z.B. durch Nitrierung von Verbindungen der allgemeinen Formel XXIII

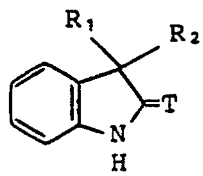 (XXIII),

in 5-Stellung und nachfolgende Reduktion der Nitro- zur Aminogruppe.

Die Herstellung der Verbindungen der allgemeinen Formel I kann auch in der Weise erfolgen, daß man f) in allgemein bekannter Weise Verbindungen der allgemeinen Formel XV bzw. XVI an der 6- bzw. 5-Aminofunktion acetyliert und durch nachfolgende Nitrierung und Abspaltung der Acetylschutzgruppen Verbindungen der allgemeinen Formel XXIV

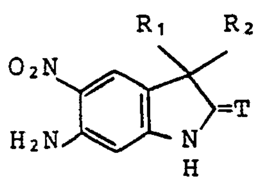 (XXIV) 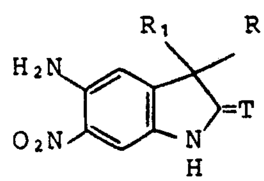 (XXV),

bzw. XXV

in denen $R_1$, $R_2$ und T die oben angegebene Bedeutung haben, bekommt. Durch Umsetzungen mit Verbindungen der allgemeinen Formel XIV lassen sich in der oben beschriebenen Weise ebenfalls Verbindungen der allgemeinen Formel XIX bzw. XX erhalten.

g) Verbindungen der allgemeinen Formel I oder deren tautomere Formen sowie die hierzu fuehrenden verschiedenen Zwischenprodukte lassen sich außer nach den oben beschriebenen Verfahren auch nach verschiedenen literaturbekannten Verfahren, z.B. aus Verbindungen der allgemeinen Formel XXVI

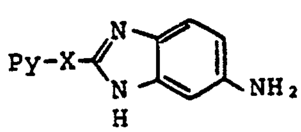 (XXVI) ------> (I),

mit Py und X in der oben angegebenen Bedeutung, herstellen (siehe hierzu R.C. Elderfield (Ed.), P.L. Julian, E.W. Meyer, H.C. Printy, Heterocycl. Comp. Vol. 3, 126-186, John Wiley Sons 1952, New York).

So werden z.B. Verbindungen der Formel XXVI mit einem reaktiven Carbonsäure-Derivat der Formel XXVII, vorzugsweise Säurechlorid,

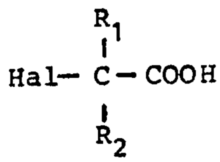

(XXVII)

acyliert und mit Lewis-Säuren ($AlCl_3$) cyclisiert (Stollé-Synthese), wobei Hal gleich Chlor, Brom oder Jod sein kann und $R_1$ und $R_2$ die oben angegebene Bedeutung besitzen. Nach dieser Methode können vorzugsweise die 7-monosubstituierten Verbindungen der Formel I hergestellt werden.

Nach einer anderen Variante werden Verbindungen der Formel XXVIII

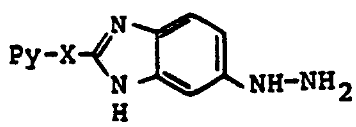

(XXVIII)

mit einem reaktiven Carbonsäure-Derivat der Formel XXIX, vorzugsweise Säurechlorid,

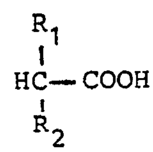

(XXIX)

in der $R_1$ und $R_2$ die oben angegebene Bedeutung besitzen, zu entsprechenden Hydraziden umgesetzt und diese dann unter alkalischen Bedingungen cyclisiert. (Brunner-Synthese)

Reaktive Carbonsäure-Derivate der Formeln XXVII und XXIX sind insbesondere Säurechloride, Anhydride oder Mesyl- und Tosylester.

Entsprechende Verbindungen der allgemeinen Formel I oder die hierzu fuehrenden Zwischenprodukte werden auch erhalten durch Umsetzung von Phenylhydrazin bzw. entsprechend substituierten Phenylhydrazinen nach der von K. Brunner, Monatsh. f. Chemie 18 , 95 (1897) beschriebenen Weise oder z.B. durch Alkylierung von 2,4-Dinitrochlorbenzol mit geeignet substituierten Malonsaeurederivaten, z.B. dem Natriumsalz des Methylmalonsaeurediethylesters, nach literaturbekannten Verfahren, anschließende Reduktion der Nitrogruppen und Ringschluß zu Verbindungen der allgemeinen Formel XV.

Die oben erwaehnte Hydrierung einer Nitrogruppe in den unter a) - g) genannten Verfahren zu Verbindungen der allgemeinen Formel I wird vorzugsweise in einem Loesungsmittel oder Loesungsmittelgemisch wie Wasser, Methanol, Ethanol, Eisessig, Essigsaeureethylester oder Dimethylformamid mit Wasserstoff in Gegenwart eines Katalysators wie Raney-Nickel, Platin oder Palladium/Kohle, mit Metallen wie Eisen, Zinn oder Zink in Gegenwart einer Saeure, mit Salzen wie Eisen(II)sulfat, Zinn(II)chlorid, Natriumsulfid, Natriumhydrogensulfit oder Natriumdithionit oder mit Hydrazin in Gegenwart von Raney-Nickel bei Temperaturen zwischen 0 und 100° C, vorzugsweise jedoch bei Raumtemperatur, durchgefuehrt.

Die in den oben in den Verfahren a) - g) genannten Cyclisierungen zu den gewuenschten Verbindungen der allgemeinen Formel I werden vorzugsweise in einem Loesungsmittel oder Loesungsmittelgemisch wie Ethanol, Isopropanol, Eisessig, Benzol, Toluol, Chlorbenzol, Glycol, Ethylenglycoldimethylether, Sulfolan oder Dimethylformamid bei Temperaturen zwischen 0° C und 220° C, vorzugsweise jedoch bei der Siedetemperatur des Reaktionsgemisches, gegebenenfalls in Gegenwart eines Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxid, Natriummethylat oder Kalium-tert.-butylat vorgenommen. Die Cyclisierung kann jedoch auch ohne Loesungsmittel und/oder Kondensationsmittel durchgefuehrt werden.

Die nachtraegliche Umwandlung einer Verbindung der Formel I in eine andere Verbindung der Formel I betrifft z.B. die Oxidation des Pyridyl-Restes in das entsprechende N-Oxid, was vorwiegend durch $H_2O_2$ in Essigsaeure geschieht, sowie die Hydrierung eines ungesaettigten Substituenten. Dies trifft insbesondere fuer die Hydrierung einer Vinyl-Verbindung (X = -CH=CH-) in die entsprechende Ethyl-Verbindung zu.

Die nachtraegliche Umwandlung einer Verbindung der Formel I, in der $R_1$ und $R_2$ gleich Wasserstoff

bedeuten, in eine andere Verbindung der Formel I betrifft z.B. auch die Umsetzung mit Verbindungen der allgemeinen Formel VII mit der oben angegebenen Bedeutung in Gegenwart einer Base wie Ammoniak oder Triethylamin in alkoholischer Loesung. Insbesondere trifft dieses zu fuer die Umwandlung von Verbindungen der allgemeinen Formel I in denen $R_1$ = $R_2$ = Wasserstoff bedeuten zu Verbindungen der allgemeinen Formel I in denen $R_1$ zusammen mit $R_2$ die Isopropylidengruppe, die Cyclopentyliden- oder Cyclohexylidengruppe darstellen, sowie gegebenenfalls deren Hydrierung zu den entsprechenden Verbindungen der allgemeinen Formel I, in denen $R_1$ oder $R_2$ gleich Wasserstoff ist.

Außerdem betrifft die nachtraegliche Umwandlung Verbindungen der allgemeinen Formel I in denen $R_1$ oder $R_2$ eine Carboxylgruppe oder ein reaktionsfaehiges Derivat wie z.B. Carbonsaeureester oder Saeurechlorid darstellt, die sich mit Hydrazin, Ammoniak, einem primaeren oder sekundaeren Amin oder einem reaktionsfaehigen Derivat hiervon zu neuen Verbindungen der allgemeinen Formel I, in denen $R_1$ oder $R_2$ eine durch eine Amino-, Alkylamino-, Dialkylamino- oder Hydrazinogruppe substituierte Carbonylgruppe ist, umsetzen lassen. Die nachtraegliche Umwandlung betrifft auch Verbindungen der allgemeinen Formel I in denen $R_1$ oder $R_2$ eine Aminocarbonylgruppe darstellt zu solchen in denen $R_1$ oder $R_2$ eine Cyangruppe ist, sowie die nachtraegliche Umwandlung einer Cyanogruppe in eine Carboxyl-, Aminocarbonyl- oder Alkoxycarbonylgruppe. Diese Umwandlungen werden alle nach allgemein ueblichen und literaturbekannten Methoden vorgenommen.

Die nachtraegliche Umwandlung zu Verbindungen der allgemeinen Formel I und den zu den Verbindungen der allgemeinen Formel I fuehrenden Zwischenprodukten in denen T ein Schwefelatom bedeutet, aus solchen in denen T ein Sauerstoffatom darstellt, wird nach literaturbekannten Verfahren mit einem das Schwefelatom uebertragenden Reagenz wie z.B. Phosphorpentasulfid oder 2,4-Bis (4-methoxyphenyl)-2,4-dithioxo-1,3,2,4-dithiadiphosphetan in einem geeigneten Loesungsmittel wie z.B. Tetrahydrofuran, Dioxan, Ethylenglycoldimethylether, Benzol, Toluol oder Pyridin bei Temperaturen zwischen 0° C und der Siedetemperatur des Reaktionsgemisches durchgefuehrt.

Die Verbindungen der allgemeinen Formel XII, XIX, XX, XXIV und XXV, in denen

$R_1$ ein Wasserstoffatom, eine Alkyl-, Alkenyl- oder eine Cycloalkylgruppe bedeutet,

$R_2$ ein Wasserstoffatom, eine Alkyl-, Alkenyl- oder Cyanogruppe, eine durch eine Hydroxy-, Alkyl-, Alkoxy-, Amino-, Alkylamino-, Dialkylamino- oder Hydrazinogruppe substituierte Carbonylgruppe bedeutet oder mit $R_1$ eine Cycloalkylengruppe bilden,

und soweit zutreffend

X einen Valenzstrich, eine $C_1$-$C_4$-Alkylengruppe oder die Vinylgruppe bedeutet,

T gleich Sauerstoff oder Schwefel bedeutet und

Py einen 2-, 3-, oder 4-Pyridylrest darstellt, der gegebenenfalls durch eine oder mehrere Alkyl-, Alkoxy-, Hydroxy-, Cyano- oder Nitrogruppen, sowie durch Halogen substituiert sein kann,

sind neu und ebenfalls Gegenstand der Erfindung.

Erfindungsgemaeße Verbindungen der Formel XII sind außer den in den Beispielen genannten folgende Verbindungen:

5,6-Diamino-3-acetyl-indolin-2-on
5,6-Diamino-3-acetyl-3-methyl-indolin-2-on
5,6-Diamino-3-allyl-indolin-2-on
5,6-Diamino-3-cyan-3-methyl-indolin-2-on
5,6-Diamino-3-cyclohexyl-indolin-2-on
5,6-Diamino-3-cyclopentyl-indolin-2-on
5,6-Diamino-3,3-diallyl-indolin-2-on
5,6-Diamino-3-ethoxycarbonyl-indolin-2-on
5,6-Diamino-3-ethoxycarbonyl-3-ethyl-indolin-2-on
5,6-Diamino-3-ethoxycarbonyl-3-methyl-indolin-2-on
5,6-Diamino-3-ethyl-indolin-2-on
5,6-Diamino-3-methoxycarbonyl-indolin-2-on
5,6-Diamino-3-methoxycarbonyl-3-methyl-indolin-2-on
5,6-Diamino-3-methyl-indolin-2-on
5,6-Diamino-3-(3-pentyl)-indolin-2-on
5,6-Diamino-3-(2-propyl)-indolin-2-on
5',6'-Diamino-spiro[cyclohexan-1,3'-indolin]-2'-on

Erfindungsgemaeße Verbindungen der Formel XXV sind außer den in den Beispielen genannten Verbindungen folgende:

5-Amino-6-nitro-3-acetyl-indolin-2-on
5-Amino-6-nitro-3-acetyl-3-methyl-indolin-2-on

5-Amino-6-nitro-3-allyl-indolin-2-on
5-Amino-6-nitro-3-cyan-3-methyl-indolin-2-on
5-Amino-6-nitro-3-cyclohexyl-indolin-2-on
5-Amino-6-nitro-3-cyclopentyl-indolin-2-on
5-Amino-6-nitro-3,3-diallyl-indolin-2-on
5-Amino-6-nitro-3-ethoxycarbonyl-indolin-2-on
5-Amino-6-nitro-3-ethoxycarbonyl-3-ethyl-indolin-2-on
5-Amino-6-nitro-3-ethoxycarbonyl-3-methyl-indolin-2-on
5-Amino-6-nitro-3-ethyl-indolin-2-on
5-Amino-6-nitro-3-methoxycarbonyl-indolin-2-on
5-Amino-6-nitro-3-methoxycarbonyl-3-methyl-indolin-2-on
5-Amino-6-nitro-3-methyl-indolin-2-on
5-Amino-6-nitro-3-(3-pentyl)-indolin-2-on
5-Amino-6-nitro-3-(2-propyl)-indolin-2-on
5-Amino-6-nitro-3-(2-methyl-propyl)-indolin-2-on
5'-Amino-6'-nitro-spiro[cyclohexan-1,3'-indolin]-2'-on
5'-Amino-6'-nitro-spiro[cyclopentan-1,3'-indolin]-2'-on
5-Amino-6-nitro-3,3-dimethyl-indolin-2-thion
5-Amino-6-nitro-3-ethyl-indolin-2-thion
5-Amino-6-nitro-3-methyl-indolin-2-thion

Erfindungsgemaeße Verbindungen der Formel XXIV sind außer den in den Beispielen genannten Verbindungen folgende:

6-Amino-5-nitro-indolin-2-on
6-Amino-5-nitro-3-acetyl-indolin-2-on
6-Amino-5-nitro-3-acetyl-3-methyl-indolin-2-on
6-Amino-5-nitro-3-allyl-indolin-2-on
6-Amino-5-nitro-3-cyan-3-methyl-indolin-2-on
6-Amino-5-nitro-3-cyclohexyl-indolin-2-on
6-Amino-5-nitro-3-cyclopentyl-indolin-2-on
6-Amino-5-nitro-3,3-diallyl-indolin-2-on
6-Amino-5-nitro-3-ethoxycarbonyl-indolin-2-on
6-Amino-5-nitro-3-ethoxycarbonyl-3-ethyl-indolin-2-on
6-Amino-5-nitro-3-ethoxycarbonyl-3-methyl-indolin-2-on
6-Amino-5-nitro-3-ethyl-indolin-2-on
6-Amino-5-nitro-3-methoxycarbonyl-indolin-2-on
6-Amino-5-nitro-3-methoxycarbonyl-3-methyl-indolin-2-on
6-Amino-5-nitro-3-methyl-indolin-2-on
6-Amino-5-nitro-3-(3-pentyl)-indolin-2-on
6-Amino-5-nitro-3-(2-propyl)-indolin-2-on
6'-Amino-5'nitro-spiro[cyclohexan-1,3'-indolin)-2-'-on
6-Amino-5-nitro-3,3-dimethyl-indolin-2-thion
6-Amino-5-nitro-3-ethyl-indolin-2-thion
6-Amino-5-nitro-3-methyl-indolin-2-thion

Erfindungsgemaeße Verbindungen der Formel XIX sind außer den in den Beispielen genannten Verbindungen folgende:

5-Nitro-6-(3-pyridinoylamino)-indolin-2-on
3-Acetyl-5-nitro-6-(3-pyridinoylamino)-indolin-2-on
3-Acetyl-5-nitro-6-(4-pyridinoylamino)-indolin-2-on
3-Acetyl-3-methyl-5-nitro-6-(3-pyridinoylamino)-indolin-2-on
3-Acetyl-3-methyl-5-nitro-6-(4-Pyridinoylamino)-indolin-2-on
3-Allyl-5-nitro-6-(3-pyridinoylamino)-indolin-2-on
3-Allyl-5-nitro-6-(4-pyridinoylamino)-indolin-2-on
3-Cyan-3-methyl-5-nitro-6-(3-pyridinoylamino)-indolin-2-on
3-Cyan-3-methyl-5-nitro-6-(4-pyridinoylamino)-indolin-2-on
3-Cyclohexyl-5-nitro-6-(3-pyridinoylamino)-indolin-2-on
3-Cyclohexyl-5-nitro-6-(4-pyridinoylamino)-indolin-2-on
3-Cyclopentyl-5-nitro-6-(3-pyridinoylamino)-indolin-2-on
3,3-Diallyl-5-nitro-6-(3-pyridinoylamino)-indolin-2-on

3,3-Diallyl-5-nitro-6-(4-pyridinoylamino)-indolin-2-on
3,3-Dimethyl-5-nitro-6-(2-pyridinoylamino)-indolin-2-on
3,3-Dimethyl-5-nitro-6-(2-(5-n-butyl-pyridinoylamino))-indolin-2-on
3,3-Dimethyl-5-nitro-6-(3-(6-cyan-pyridinoylamino))-indolin-2-on
3,3-Dimethyl-5-nitro-6-(3-(6-methyl-pyridinoylamino))-indolin-2-on
3,3-Dimethyl-5-nitro-6-(4-(2-methyl-pyridinoylamino))-indolin-2-on
3,3-Dimethyl-5-nitro-6-(4-(2-hydroxy-pyridinoylamino))-indolin-2-on
3-Ethoxycarbonyl-5-nitro-6-(3-pyridinoylamino)-indolin-2-on
3-Ethoxycarbonyl-5-nitro-6-(4-pyridinoylamino)-indolin-2-on
3-Ethoxycarbonyl-3-ethyl-5-nitro-6-(3-pyridinoylamino)-indolin-2-on
3-Ethoxycarbonyl-3-ethyl-5-nitro-6-(4-pyridinoylamino)-indolin-2-on
3-Ethoxycarbonyl-3-methyl-5-nitro-6-(3-pyridinoylamino)-indolin-2-on
3-Methoxycarbonyl-5-nitro-6-(3-pyridinoylamino)-indolin-2-on
3-Methoxycarbonyl-5-nitro-6-(4-pyridinoylamino)-indolin-2-on
3-Methoxycarbonyl-3-methyl-5-nitro-6-(3-pyridinoylamino)-indolin-2-on
3-Methoxycarbonyl-3-methyl-5-nitro-6-(4-pyridinoylamino)-indolin-2-on
3-(3-Pentyl)-5-nitro-6-(3-pyridinoylamino)-indolin-2-on
3-(3-Pentyl)-5-nitro-6-(4-pyridinoylamino)-indolin-2-on
3-(2-Propyl)-5-nitro-6-(3-pyridinoylamino)-indolin-2-on
3-(2-Methyl-propyl)-5-nitro-6-(3-pyridinoylamino)-indolin-2-on
3-(2-Methyl-propyl)-5-nitro-6-(4-pyridinoylamino)-indolin-2-on
5'-Nitro-6'-(3-pyridinoylamino)-spiro[cyclohexan-1,3'-indolin]-2'-on
5'-Nitro-6'-(4-pyridinoylamino)-spiro[cyclohexan-1,3'-indolin]-2'-on
5'-Nitro-6'-(3-pyridinoylamino)-spiro[cyclopentan-1,3'-indolin]-2'-on
5'-Nitro-6'-(4-pyridinoylamino)-spiro[cyclopentan-1,3'-indolin]-1'-on
3,3-Dimethyl-5-nitro-6-(2-pyridinoylamino)-indolin-2-thion
3,3-Dimethyl-5-nitro-6-(3-pyridinoylamino)-indolin-2-thion
3,3-Dimethyl-5-nitro-6-(4-pyridinoylamino)-indolin-2-thion
3-Ethyl-5-nitro-6-(2-pyridinoylamino)-indolin-2-thion
3-Ethyl-5-nitro-6-(3-pyridinoylamino)-indolin-2-thion
3-Ethyl-5-nitro-6-(4-pyridinoylamino)-indolin-2-thion
3-Methyl-5-nitro-6-(2-pyridinoylamino)-indolin-2-thion
3-Methyl-5-nitro-6-(3-pyridinoylamino)-indolin-2-thion
3-Methyl-5-nitro-6-(4-pyridinoylamino)-indolin-2-thion

Erfindungsgemaeße Verbindungen der allgemeinen Formel XX sind außer in den Beispielen genannten Verbindungen folgende:

6-Nitro-5-(3-pyridinoylamino)-indolin-2-on
6-Nitro-5-(4-pyridinoylamino)-indolin-2-on
3-Acetyl-6-nitro-5-(3-pyridinoylamino)-indolin-2-on
3-Acetyl-6-nitro-5-(4-pyridinoylamino)-indolin-2-on
3-Acetyl-3-methyl-6-nitro-5-(3-pyridinoylamino)-indolin-2-on
3-Acetyl-3-methyl-6-nitro-5-(4-pyridinoylamino)-indolin-2-on
3-Allyl-6-nitro-5-(3-pyridinoylamino)-indolin-2-on
3-Allyl-6-nitro-5-(4-pyridinoylamino)-indolin-2-on
3-Cyan-3-methyl-6-nitro-5-(3-pyridinoylamino)-indolin-2-on
3-Cyan-3-methyl-6-nitro-5-(4-pyridinoylamino)-indolin-2-on
3-Cyclohexyl-6-nitro-5-(3-pyridinoylamino)-indolin-2-on
3-Cyclohexyl-6-nitro-5-(4-pyridinoylamino)-indolin-2-on
3-Cyclopentyl-6-nitro-5-(3-pyridinoylamino)-indolin-2-on
3-Cyclopentyl-6-nitro-5-(4-pyridinoylamino)-indolin-2-on
3,3-Diallyl-6-nitro-5-(3-pyridinoylamino)-indolin-2-on
3,3-Diallyl-6-nitro-5-(4-pyridinoylamino)-indolin-2-on
3,3-Diethyl-6-nitro-5-(3-pyridinoylamino)-indolin-2-on
3,3-Diethyl-6-nitro-5-(4-pyridinoylamino)-indolin-2-on
3,3-Dimethyl-6-nitro-5-(2-pyridinoylamino)-indolin-2-on
3,3-Dimethyl-6-nitro-5-(3-pyridinoylamino)-indolin-2-on
3,3-Dimethyl-6-nitro-5-(2-(5-n-butyl-pyridinoylamino)-indolin-2-on
3,3-Dimethyl-6-nitro-5-(3-(6-cyan-pyridinoylamino))-indolin-2-on

3,3-Dimethyl-6-nitro-5-(3-(6-methyl-pyridinoylamino))-indolin-2-on
3,3-Dimethyl-6-nitro-5-(4-(2-methyl-pyridinoylamino))-indolin-2-on
3,3-Dimethyl-6-nitro-5(4-(2-hydroxy-pyridinoylamino))-indolin-2-on
3-Ethoxycarbonyl-6-nitro-5-(3-pyridinoylamino)-indolin-2-on
3-Ethoxycarbonyl-6-nitro-5-(4-pyridinoylamino)-indolin-2-on
3-Ethoxycarbonyl-3-ethyl-6-nitro-5-(3-pyridinoylamino)-indolin-2-on
3-Ethoxycarbonyl-3-ethyl-6-nitro-5-(4-pyridinoylamino)-indolin-2-on
3-Ethoxycarbonyl-3-methyl-6-nitro-5-(3-pyridinoylamino)-indolin-2-on
3-Ethoxycarbonyl-3-methyl-6-nitro-5-(4-pyridinoylamino)-indolin-2-on
3-Ethyl-6-nitro-5-(3-pyridinoylamino)-indolin-2-on
3-Ethyl-6-nitro-5-(4-pyridinoylamino)-indolin-2-on
3-Methoxycarbonyl-6-nitro-5-(3-pyridinoylamino)-indolin-2-on
3-Methoxycarbonyl-6-nitro-5-(4-pyridinoylamino)-indolin-2-on
3-Methoxycarbonyl-3-methyl-6-nitro-5-(3-pyridinoylamino)-indolin-2-on
3-Methoxycarbonyl-3-methyl-6-nitro-5-(4-pyridinoylamino)-indolin-2-on
3-Methyl-6-nitro-5-(3-pyridinoylamino)-indolin-2-on
3-Methyl-6-nitro-5-(4-pyridinoylamino)-indolin-2-on
3-(3-Pentyl)-6-nitro-5-(3-pyridinoylamino)-indolin-2-on
3-(3-Pentyl)-6-nitro-5-(4-pyridinoylamino)-indolin-2-on
3-(2-Propyl)-6-nitro-5-(3-pyridinoylamino)-indolin-2-on
3-(2-Propyl)-6-nitro-5-(4-pyridinoylamino)-indolin-2-on
3-(2-Methyl-propyl)-6-nitro-5-(3-pyridinoylamino)-indolin-2-on
3-(2-Methyl-propyl)-6-nitro-5-(4-pyridinoylamino)-indolin-2-on
6'-Nitro-5'-(3-pyridinoylamino)-spiro[cyclohexan-1,3'-indolin]-2'-on
6'-Nitro-5'-(4-pyridinoylamino)-spiro[cyclohexan-1,3'-indolin]-2'-on
6'-Nitro-5'-(3-pyridinoylamino)-spiro[cyclopentan-1,3'-indolin]-2'-on
6'-Nitro-5'-(4-pyridinoylamino)-spiro[cyclopentan-1,3'-indolin]-2'-on
3,3-Dimethyl-6-nitro-5-(2-pyridinoylamino)-indolin-2-thion
3,3-Dimethyl-6-nitro-5-(3-pyridinoylamino)-indolin-2-thion
3,3-Dimethyl-6-nitro-5-(4-pyridinoylamino)-indolin-2-thion
3-Ethyl-6-nitro-5-(2-pyridinoylamino)-indolin-2-thion
3-Ethyl-6-nitro-5-(3-pyridinoylamino)-indolin-2-thion
3-Ethyl-6-nitro-5-(4-pyridinoylamino)-indolin-2-thion
3-Methyl-6-nitro-5-(2-pyridinoylamino)-indolin-2-thion
3-Methyl-6-nitro-5-(3-pyridinoylamino)-indolin-2-thion
3-Methyl-6-nitro-5-(4-pyridinoylamino)-indolin-2-thion

Zur Ueberfuehrung der Verbindungen der allgemeinen Formel I bzw. deren tautomeren Formen in ihre pharmakologisch unbedenklichen Salze, setzt man diese vorzugsweise in einem organischen Loesungsmittel mit der aequivalenten Menge einer anorganischen oder organischen Saeure, z.B. Salzsaeure, Bromwasserstoffsaeure, Phosphorsaeure, Schwefelsaeure, Essigsaeure, Citronensaeure, Weinsaeure, Maleinsaeure, Fumarsaeure, Benzoesaeure oder Cyclohexylsulfaminsaeure um.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komblexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Sterinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks-und Sueßstoffe enthalten.

Die erfindungsgemaeßen Verbindungen der Formel I werden ueblicherweise in Mengen von 10-500 mg pro Tag bezogen auf 75 kg Koerpergewicht appliziert. Bevorzugt ist es, 2-3 mal pro Tag 1-2 Tabletten mit

einem Wirkstoffgehalt von 5-200 mg zu verabreichen. Die Tabletten koennen auch retardiert sein, wodurch nur noch 1 mal pro Tag 1-2 Tabletten mit 10-500 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Bevorzugt im Sinne der vorliegenden Erfindung sind außer den in den Beispielen genannten Verbindungen die folgenden und deren Tautomere:

2-(3-Pyridyl)-6,7-dihydro-3H,5H-pyrrolo[2,3-f]benzimidazol-6-on
7-Acetyl-2(3-pyridyl)-6,7-dihydro-3H,5H-pyrrolo[2,3-f]benzimidazol-6-on
7-Acetyl-2-(4-pyridyl)-6,7-dihydro-3H,5H-pyrrolo[2,3-f]benzimidazol-6-on
7-Acetyl-7-methyl-2-(3-pyridyl)-6,7-dihydro-3H,5H-pyrrolo[2,3-f]benzimidazol-6-on
7-Acetyl-7-methyl-2-(4-pyridyl)-6,7-dihydro-3H,5H-pyrrolo[2,3-f]-benzimidazol-6-on
7-Allyl-2-(3-pyridiyl)-6,7-dihydro-3H,5H-pyrrolo[2,3-f]benzimidazol-6-on
7-Allyl-2-(4-pyridyl)-6,7-dihydro-3H,5H-pyrrolo[2,3-f]benzimidazol-6-on
7-Aminocarbonyl-7-methyl-2-(3-pyridyl)-6,7-dihydro-3H,5H-pyrrolo[2,3-f]benzimidazol-6-on
7-Aminocarbonyl-7-methyl-2-(4-pyridyl)-6,7-dihydro-3H,5H-pyrrolo[2,3-f]benzimidazol-6-on
7-Cyan-7-methyl-2-(3-pyridyl)-6,7-dihydro-3H,5H-pyrrolo[2,3-f]benzimidazol-6-on
7-Cyan-7-methyl-2-(4-pyridyl)-6,7-dihydro-3H,5H-pyrrolo[2,3-f]benzimidazol-6-on
7-Cyclohexyl-2-(3-pyridyl)-6,7-dihydro-3H,5H-pyrrolo[2,3-f]-benzimidazol-6-on
7-Cyclohexyl-2-(4-pyridyl)-6,7-dihydro-3H,5H-pyrrolo[2,3-f]-benzimidazol-6-on
7-Cyclopentyl-2-(3-pyridyl)-6,7-dihydro-3H,5H-pyrrolo[2,3-f]-benzimidazol-6-on
7,7-Diallyl-2-(3-pyridyl)-6,7-dihydro-3H,5H-pyrrolo[2,3-f]-benzimidazol-6-on
7,7-Diallyl-2-(4-pyridyl)-6,7-dihydro-3H,5H-pyrrolo[2,3-f]-benzimidazol-6-on
7,7-Diethyl-2-(3-pyridyl)-6,7-dihydro-3H,5H-pyrrolo[2,3-f]-benzimidazol-6-on
7,7-Diethyl-2-(4-N-oxy-pyridyl)-6,7-dihydro-3H,5H-pyrrolo[2,3-f]benzimidazol-6-on
7,7-Diethyl-2-(4-(2-methyl-pyridyl)-6,7-dihydro-3H,5H-pyrrolo[2,3-f]benzimidazol-6-on
7,7-Diethyl-2-(4-pyridyl-ethyl)-6,7-dihydro-3H,5H-pyrrolo[2,3-f]benzimidazol-6-on
7,7-Dimethyl-2-(2-N-oxy-pyridyl)-6,7-dihydro-3H,5H-pyrrolo[2,3-f]benzimidazol-6-on
7,7-Dimethyl-2-(2-(5-n-butyl-pyridyl))-6,7-dihydro-3H,5H-pyrrolo[2,3-f]benzimidazol-6-on
7,7-Dimethyl-2-(3-(6-cyan-pyridyl))-6,7-dihydro-3H,5H-pyrrolo[2,3-f]benzimidazol-6-on
7,7-Dimethyl-2-(3-(6-hydroxy-pyridyl))-6,7-dihydro-3H,5H-pyrrolo[2,3-f]benzimidazol-6-on
7,7-Dimethyl-2-(3-(6-nitro-pyridyl))-6,7-dihydro-3H,5H-pyrrolo[2,3-f]benzimidazol-6-on
7,7-Dimethyl-2-(4-(2-ethyl-pyridyl))-6,7-dihydro-3H,5H-pyrrolo[2,3-f]benzimidazol-6-on
7,7-Dimethyl-2-(4-(2-methoxy-pyridyl))-6,7-dihydro-3H,5H-pyrrolo[2,3-f]benzimidazol-6-on
7,7-Dimethyl-2-(4-(2-nitro-pyridyl))-6,7-dihydro-3H,5H-pyrrolo[2,3-f]benzimidazol-6-on
7,7-Dimethyl-2-(3-pyridyl-methyl)-6,7-dihydro-3H,5H-pyrrolo[2,3-f]benzimidazol-6-on
7,7-Dimethyl-2-(3-pyridyl)-ethyl)-6,7-dihydro-3H,5H-pyrrolo[2,3-f]benzimidazol-6-on
7,7-Dimethyl-2-(4-pyridyl-vinyl)-6,7-dihydro-3H,5H-pyrrolo[2,3-f]benzimidazol-6-on
7-Ethoxycarbonyl-2-(3-pyridyl)-6,7-dihydro-3H,5H-pyrrolo[2,3-f]benzimidazol-6-on
7-Ethoxycarbonyl-2-(4-pyridyl)-6,7-dihydro-3H,5H-pyrrolo[2,3-f]benzimidazol-6-on
7-Ethoxycarbonyl-7-ethyl-2-(3-pyridyl)-6,7-dihydro-3H,5H-pyrrolo[2,3-f]benzimidazol-6-on
7-Ethoxycarbonyl-7-ethyl-2-(4-pyridyl)-6,7-dihydro-3H,5H-pyrrolo[2,3-f]benzimidazol-6-on
7-Ethoxycarbonyl-7-methyl-2-(3-pyridyl)-6,7-dihydro-3H,5H-pyrrolo[2,3-f]benzimidazol-6-on
7-Ethyl-2-(3-N-oxy-pyridyl)-6,7-dihydro-3H,5H-pyrrolo[2,3-f]benzimidazol-6-on
7-Ethyl-2-(4-N-oxy-pyridyl)-6,7-dihydro-3H,5H-pyrrolo[2,3-f]benzimidazol-6-on
7-Hydrazinocarbonyl-7-methyl-2-(3-pyridyl)-6,7-diyhdro-3H,5H-pyrrolo[2,3-f]benzimidazol-6-on
7-Isopropyliden-2-(3-pyridyl)-6,7-dihydro-3H,5H-pyrrolo[2,3-f]benzimidazol-6-on
7-Methoxycarbonyl-2-(3-pyridyl)-6,7-dihydro-3H,5H-pyrrolo[2,3-f]benzimidazol-6-on
7-Methoxycarbonyl-2-(4-pyridyl)-6,7-dihydro-3H,5H-pyrrolo[2,3-f]benzimidazol-6-on
7-Methoxycarbonyl-7-methyl-2-(3-pyridyl)-6,7-dihydro-3H,5H-pyrrolo[2,3-f]benzimidazol-6-on
7-Methoxycarbonyl-7-methyl-2-(4-pyridyl)-6,7-dihydro-3H,5H-pyrrolo[2,3-f]benzimidazol-6-on
7-Methyl-2-(3-N-oxy-pyridyl)-6,7-dihydro-3H,5H-pyrrolo[2,3-f]benzimidazol-6-on
7-(2-Methyl-propyl)-2-(3-pyridyl)-6,7-dihydro-3H,5H-pyrrolo[2,3-f]benzimidazol-6-on
7-(2-Methyl-propyl)-2-(4-pyridyl)-6,7-dihydro-3H,5H-pyrrolo[2,3-f]benzimidazol-6-on
7-(3-Pentyl)-2-(3-pyridyl)-6,7-dihydro-3H,5H-pyrrolo[2,3-f]benzimidazol-6-on
7-(3-Pentyl)-2-(4-pyridyl)-6,7-dihydro-3H,5H-pyrrolo[2,3-f]benzimidazol-6-on
7-(2-Propyl)-2-(3-pyridyl)-6,7-dihydro-3H,5H-pyrrolo[2,3-f]benzimidazol-6-on
7-(2-Propyl)-2-(4-(2-methyl-pyridyl))-6,7-dihydro-3H,5H-pyrrolo[2,3-f]benzimidazol-6-on
2'-(3-Pyridyl)-spiro[cyclopropan-1,7'-6',7'-diyhdro-3'H,5'H-pyrrolo[2',3'-f]benzimidazol-6'-on

2'-(3-Pyridyl)-spiro[cyclopentan-1,7'-6',7'-dihydro-3'H,5'H-pyrrolo[2',3'-f]benzimidazol-6'-on
2'-(3-Pyridyl)-spiro[cyclohexan-1,7'-6',7'-dihydro-3'H,5'H-pyrrolo[2',3'-f]benzimidazol-6'-on
2'-(4-Pyridyl)-spiro[cyclohexan-1,7'-6',7'-dihydro-3'H,5'-pyrrolo[2',3'-f]benzimidazol-6'-on
7,7-Dimethyl-2-(2-pyridyl)-6,7-dihydro-3H,5H-pyrrolo[2,3-f]benzimidazol-6-thion
7,7-Dimethyl-2-(3-pyridyl)-6,7-dihydro-3H,5H-pyrrolo[2,3-f]benzimidazol-6-thion
7,7-Dimethyl-2-(3-(6-methyl-pyridyl))-6,7-dihydro-3H,5H-pyrrolo[2,3-f]benzimidazol-6-thion
7,7-Dimethyl-2-(4-(2-methyl-pyridyl))-6,7-dihydro-3H,5H-pyrrolo[2,3-f]benzimidazol-6-thion
7-Ethyl-2-(2-pyridyl)-6,7-dihydro-3H,5H-pyrrolo[2,3-f]benzimidazol-6-thion
7-Ethyl-2-(3-Pyridyl)-6,7-dihydro-3H,5H-pyrrolo[2,3-f]benzimidazol-6-thion
7-Ethyl-2-(4-pyridyl)-6,7-dihydro-3H,5H-pyrrolo[2,3-f]benzimidazol-6-thion
7-Methyl-2-(2-pyridyl)-6,7-dihydro-3H,5H-pyrrolo[2,3-f]benzimidazol-6-thion
7-Methyl-2-(3-pyridyl)-6,7-dihydro-3H,5H-pyrrolo[2,3-f]benzimidazol-6-thion
7-Methyl-2-(4-pyridyl)-6,7-dihydro-3H,5H-pyrrolo[2,3-f]benzimidazol-6-thion

Beispiel 1

7,7-Dimethyl-2-(4-pyridyl)-6,7-dihydro-3H,5H-pyrrolo[2,3-f]benzimidazol-6-on x 4 $H_2O$

Zu einer Loesung von 5,6-Diamino-3,3-dimethyl-indolin-2-on (2.9 g, 0.015 mol) in Methylenchlorid (30 ml) mit Triethylamin (4.4 ml, 0.032 mol) gibt man portionsweise Isonicotinsaeurechlorid Hydrochlorid (3 g, 0.017 mol) und laeßt ueber Nacht weiterruehren. Es wird im Vakuum zur Trockene eingeengt, der Rueckstand mehrfach mit Wasser durchgeruehrt, abgesaugt und die erhaltenen Kristalle in einer Loesung von Ethanol (100 ml) mit konz. HCl (10 ml) ca. 12 h zum Rueckfluß erhitzt. Die erhaltene Mischung wird im Vakuum zur Trockne eingeengt, mit Wasser (20 ml) durchgeruehrt und mit waessriger $NH_3$-Loesung neutral gestellt. Anschließend wird abgesaugt, mit Wasser nachgewaschen, getrocknet und aus Wasser mit 30 % Ethanol umkristallisiert.

Ausbeute 1.9 g (36 % d.Th.), Mp. 215° C

Analog zu Beispiel 1 erhaelt man:

| | Bezeichnung | Ausbeute [%] | Mp [°C] Lösungsm. |
|---|---|---|---|
| a) | 7,7-Dimethyl-2-(2-pyridyl)-6,7-dihydro-3H, 5H-pyrrolo[2,3-f]benzimidazol-6-on x 0,3 $H_2O$ aus 5,6-Diamino-3,3-dimethyl-indolin-2-on und Picolinsaeurechlorid | 79 | 182-187 Wasser |
| b) | 7,7-Dimethyl-2-(3-pyridyl)-6,7-dihydro-3H, 5H-pyrrolo[2,3-f]benzimidazol-6-on x 3 $H_2O$ aus 5,6-Diamino-3,3-dimethyl-indolin-2-on und Nicotinsaeurechlorid Hydrochlorid | 38 | 331-335 Dioxan/ Wasser 2:1 |
| c) | 7,7-Dimethyl-2-(4-(2-methyl-pyridyl))-6,7-dihydro-3H,5H-pyrrolo[2,3-f]benzimidazol-6-on x 0.6 $H_2O$ aus 5,6-Diamino-3,3-dimethyl-indolin-2-on und 2-Methylisonicotinsaeurechlorid | 43 | 311-313 Aceton |
| d) | 7,7-Dimethyl-2-(4-(2-hydroxy-pyridyl))-6,7-dihydro-3H,5H-pyrrolo[2,3-f]benzimidazol-6-on x 2 $H_2O$ aus 5,6-Diamino-3,3-dimethyl-indolin-2-on und 2-Hydroxyisonicotinsaeurechlorid | 23 | >360 Wasser |

| | Bezeichnung | Ausbeute [%] | Mp [°C] Lösungsm. |
|---|---|---|---|
| e) | 7,7-Dimethyl-2-(4-(2-chlor-pyridyl))-6,7-dihydro-3H,5H-pyrrolo[2,3-f]benzimidazol-6-on<br>aus<br>5,6-Diamino-3,3-dimethyl-indolin-2-on<br>und<br>2-Chlorisonicotinsaeurechlorid | 42 | 341-344<br>Essigester |
| f) | 7,7-Dimethyl-2-(3-pyridyl-vinyl)-6,7-dihydro-3H,5H-pyrrolo[2,3-f]benzimidazol-6-on<br>x 0,75 $H_2O$<br>aus<br>5,6-Diamino-3,3-dimethyl-indolin-2-on<br>und<br>3-Pyridylacrylsaeurechlorid-Hydrochlorid | 17 | 203-207<br>Wasser + 5 % $CH_3OH$ |
| g) | 7,7-Dimethyl-2-(4-pyridyl-ethyl)-6,7-dihydro-3H,5H-pyrrolo[2,3-f]benzimidazol-6-on<br>x 0,6 $H_2O$<br>aus<br>5,6-Diamino-3,3-dimethyl-indolin-2-on<br>und<br>4-Pyridylpropionsaeurechlorid-Hydrochlorid | 35 | 150-154<br>Wasser |

| Bezeichnung | Ausbeute [%] | Mp [°C] Lösungsm. |
|---|---|---|
| h) 2'-(4-Pyridyl)-spiro[cyclopentan-1,7'-6',7'-dihydro-3'H,5'H-pyrrolo[2',3'-f]benzimidazol]-6'-on x 0.3 $H_2O$<br>aus<br>5',6'-Diamino-spiro[cyclopentan-1,3'-indolin]-2'-on<br>und<br>Isonicotinsaeurechlorid-Hydrochlorid | 48 | >365 Ethanol |
| i) 7,7-Dimethyl-2-(3-(6-methyl-pyridyl))-6,7-dihydro-3H,5H-pyrrolo[2,3-f]benzimidazol--6-on<br>aus<br>5,6-Diamino-3,3-dimethyl-indolin-2-on<br>und<br>6-Methylnicotinsaeurechlorid | 52 | >360 Essig-ester |

Die Ausgangsstoffe fuer die in Beispiel 1 beanspruchten Verbindungen koennen wie folgt hergestellt werden.

4,4-Dimethyl-2H,4H-isochinolin-1,3-dion

a) Cyan-o-tolunitril (142 g, 1 mol) werden zusammen mit Benzyltributylammoniumbromid (10.8 g, 0.03 mol) in konz. NaOH-Loesung (700 ml) gegeben und Methyljodid (185 ml, 2 mol) unter Eiskuehlung zugetropft. Es wird 2 h nachgeruehrt, vom Kristallisat abgesaugt, mit Wasser nachgewaschen und getrocknet. Man erhaelt 170 g (100 % d.Th.) o-Cyano-α,α-dimethyl-phenylessigsaeurenitril. Mp 84-88° C. Das erhaltene Produkt wird in 90proz. $H_2SO_4$ (1500 ml) eingetragen, 3 h lang geruehrt, auf Eis gegossen, das Kristallisat mit Wasser nachgewaschen und getrocknet. Man erhaelt 167 g (88 % d.Th.) der Titelverbindung. Mp 119-120° C.

b) KOH (17.73 g, 0.32 mol) wurde in 26.5 ml Wasser und 106 ml Ethanol geloest. In dieser Loesung wurde 1,3(2H,4H)-Isochinolindion (25.55 g, 0.16 mol) unter Erwaermung geloest. Zu der auf Raumtemperatur gekuehlten Loesung wurde Methyliodid (45.44 g, 0.32 mol) zugetropft.

Nach 1 h bei Raumtemperatur wurde noch 1 h bei 80° C weitergeruehrt. Der groeßte Teil des Ethanols wurde abdestilliert, der Rueckstand mit 300 ml heißem Wasser versetzt, abgekuehlt und die Kristalle abgesaugt. Die Kristalle wurden in wenig 2 H NaOH geloest und mit gesaettigter $NH_4Cl$-Loesung ausgefaellt und abgesaugt. Durch Behandeln mit Aktivkohle und umkristallisieren aus Ethanol erhaelt man das reine Produkt 17 g (57 %).

In analoger Weise koennen auf dem Weg a oder b die folgenden Verbindungen erhalten werden:

| Bezeichnung | Ausbeute [%] | Mp [°C] Lösungsm. |
|---|---|---|
| Spiro[cyclopentan-1,4'-2'H,4'H-isochinolin]-1',3'-dion<br>aus<br>Cyan-o-tolunitril<br>und<br>1,4-Dibrombutan | 90 | 136-138 Ethanol |

c)

4,4-Dimethyl-7-nitro-2H,4H-isochinolin-1,3-dion

Zu einer Loesung von 4,4-Dimethyl-2H,4H-isochinolin-1,3-dion (195 g, 1 mol) in konz. $H_2SO_4$ (1000 ml) wird eine Loesung von rauchender $HNO_3$ (46.2 ml, 1.1 mol) in konz. $H_2SO_4$ bei 20°C zugetropft. Es wird eine Stunde bei RT nachgeruehrt, auf Eis gegossen, die Kristalle abgesaugt, gut mit Wasser nachgewaschen und getrocknet. Es wird aus Ethanol umkristallisiert.
Ausbeute: 206 g (85 % d.TH.), Mp 211-214°C

In analoger Weise koennen nach dem Weg c die folgenden Verbindungen erhalten werden:

| Bezeichnung | Ausbeute [%] | Mp [°C] Lösungsm. |
|---|---|---|
| 7'-Nitro-spiro[cyclopentan-1,4'-2'H,4'H-isochinolin]-1',3'-dion<br>aus<br>Spiro[cyclopentan-1,4'-2'H,4'H-isochinolin]-1',3'-dion | 90 | 225-227 Ethanol |

d)

3,3-Dimethyl-6-nitro-indolin-2-on

Zu einer Loesung von NaOH (210 g, 5.25 mol) in Wasser (1700 ml) wird bei 0°C Brom (54 ml, 1.05 mol) zugetropft und anschließend 4,4-Dimethyl-7-nitro-2H,4H-isochinolin-1,3-dion (81.7 g, 0.35 mol) eingetragen. Nach 1 h Ruehren bei RT wird ca. 1 h auf 80°C erhitzt und nach dem Abkuehlen mit Essigsaeure sauer gestellt. Es wird abgesaugt, gut mit Wasser nachgewaschen und getrocknet.
Ausbeute 49 g (68 % d.Th.), Mp 241-242°C.

Analog zu d lassen sich folgende Verbindungen synthetisieren:

| Bezeichnung | Ausbeute [%] | Mp [°C] Lösungsm. |
|---|---|---|
| 6'-Nitro-spiro[cyclopentan-1,3'-indolin]-2'-on aus 7'-nitro-spiro[cyclopentan-1,4'-2H',4H'-isochinolin]-1',3'-dion | 82 | Ethanol 226-228 |

e)

6-Amino-3,3-dimethyl-indolin-2-on

Eine Suspension von 6-Nitro-3,3-dimethyl-indolin-2-on (146 g, 0.71 mol) in Methanol (3.5 Liter) mit Eisessig (300 ml) werden an 10proz. Pd/C (16 g) bei 40° C unter gutem Ruehren hydriert. Die anschließend klare Loesung wird vom Katalysator abgesaugt und eingeengt. Ausbeute 125 g (100 % d.Th.), Mp 185-190° C.

Analog zu e lassen sich folgende Verbindungen erhalten:

| Bezeichnung | Ausbeute [%] | Mp [°C] Lösungsm. |
|---|---|---|
| 6'-Amino-spiro[cyclopentan-1,3'-indolin]-2'-on aus 6'-Nitro-spiro[cyclopentan-1,3'-indolin]-2'-on | 98 | 165-170 Essigester |

f)

6-Acetamido-3,3-dimethyl-indolin-2-on

Zu einer Suspension von 6-Amino-3,3-dimethyl-indolin-2-on (32 g, 0.18 mol) in Essigester (500 ml) wird unter Kuehlung Essigsaeureanhydrid (20.4 g, 0.2 mol) zugetropft und ca. 1 h bei RT nachgeruehrt. Das entstandene Produkt wird abgesaugt mit Essigester gut nachgewaschen und getrocknet.
Ausbeute: 37.8 g (96 % d.Th.), Mp 275-277° C.

Nach f erhaelt man in analoger Weise folgende Verbindungen:

| Bezeichnung | Ausbeute [%] | Mp [°C] Lösungsm. |
|---|---|---|
| 6'-Acetamido-spiro[cyclopentan-1,3'-indolin]-2'-on aus 6'-Amino-spiro[cyclopentan-1,3'-indolin]-2'-on | 75 | 263-265 Ethanol |

g)

6-Acetamido-3,3-dimethyl-5-nitro-indolin-2-on

Zu einer Loesung von 6-Acetamido-3,3-dimethyl-indolin-2-on (35 g, 0.16 mol) in konz. $H_2SO_4$ (200 ml) wird unter Kuehlung eine Loesung von rauchender $HNO_3$ (7.6 ml, 0.18 mol) in konz. $H_2SO_4$ (7.6 ml) zugetropft. Es wird eine Stunde nachgeruehrt, auf Eis gegossen, die Kristalle abgesaugt, gut mit Wasser nachgewaschen und getrocknet.
Ausbeute: 39 g (92 % d.Th.), Mp 276-280 ° C.

In analoger Weise koennen nach g die folgenden Verbindungen erhalten werden:

| Bezeichnung | Ausbeute [%] | Mp [°C] Lösungsm. |
|---|---|---|
| 6'-Acetamido-5'-nitro-spiro[cyclopentan-1,3'-indolin]-2'-on aus 6'-Acetamido-5'-nitro-spiro[cyclopentan-1,3'-indolin]-2'-on | 83 | 290-292 Ethanol |

h)

6-Amino-3,3-dimethyl-5-nitro-indolin-2-on

Eine Loesung von 6-Acetamido-3,3-dimethyl-5-nitro-indolin-2-on (36.2 g, 0.14 mol) in Ethanol (180 ml) mit konz. NaOH (18 ml) wird ca. 2 h am Rueckfluß erhitzt, anschließend im Vakuum eingeengt, auf pH 6 eingestellt und im Eisbad gekuehlt. Die erhaltenen Kristalle werden abgesaugt, mit Wasser gewaschen und getrocknet. Ausbeute 29.5 g (97 % d.Th.), Mp 247-248 ° C.

Analog zu h lassen sich folgende Verbindungen erhalten:

| Bezeichnung | Ausbeute [%] | Mp [°C] Lösungsm. |
|---|---|---|
| 6'-Amino-5'-nitro-spiro[cyclopentan-1,3'-indolin]-2'-on aus 6'-Acetamido-5-nitro-spiro[cyclopentan-1,3'-indolin]-2'-on | 87 | 300-303 Ethanol |

i)

5,6-Diamino-3,3-dimethyl-indolin-2-on

Eine Loesung von 6-Amino-3,3-dimethyl-5-nitro-indolin-2-on (18.7 g, 0.085 mol) in Methanol (200 ml) werden an 10proz. Pd/C (1.9 g) bei 40° C hydriert. Es wird vom Katalysator abgesaugt, eingeengt und aus Ethanol kristallisiert.
Ausbeute: 15.6 g (96 % d.Th.), Mp 245-247° C.

Nach der Vorschrift i lassen sich in analoger Weise folgende Verbindungen erhalten:

| Bezeichnung | Ausbeute [%] | Mp [°C] Lösungsm. |
|---|---|---|
| 5',6'-Diamino-spiro[cyclopentan-1,3'-indolin]-2'-on aus 6'-Amino-5'-nitro-spiro[cyclopentan-1,3'-indolin]-2'-on | 100 | 255-256 Ethanol |

Beispiel 2

7,7-Dimethyl-2-(4-pyridyl-methyl)-6,7-dihydro-3H,5H-pyrrolo-[2,3-f]benzimidazol-6-on x 1 $H_2O$

Eine Suspension von 5,6-Diamino-3,3-dimethyl-indolin-2-on (7.3 g, 0.038 mol) in 4-Pyridylessigsaeuremethylester (11.6 g, 0.077 mol) wird unter $N_2$-Atmosphaere ca. 16 h auf 180° C unter Ruehren erhitzt. Anschließend wird der ueberschuessige Ester im Vakuum abdestilliert und der erhaltene Rueckstand an Kieselgel (Laufmittel: Dichlormethan/$NH_3$-ges. Methanol 20:1) aufgetrennt. Ausbeute: 1.8 g (16 % d.Th.), Mp 333-337° C (Wasser/Methanol 10:1)

Beispiel 3

7,7-Dimethyl-2-(3-(2-methoxy-6-methyl-pyridyl))-6,7-dihydro-3H,5H-pyrrolo[2,3-f]benzimidazol-6-on

Eine Loesung von 5,6-Diamino-3,3-dimethyl-indolin-2-on (3.8 g, 0.02 mol) mit 2-Methoxy-6-methyl-

pyridin-3-aldehyd (3 g, 0.02 mol) und Toluolsulfonsaeure (0.4 g, 0.002 mol) in Ethanol (50 ml) wird ca. 1 h zum Rueckfluß erhitzt, wobei nach 30 min durch ein Einleitungsrohr Luft durch die Reaktionsmischung geleitet wird. Nach dem Abkuehlen wird von der ausgefallenen Substanz abgesaugt und das Filtrat im Vakuum eingeengt, mit Wasser verruehrt und mit Dichlormethan extrahiert. Die organische Phase wird eingeengt und der Rueckstand aus Ether kristallisiert. Die vereinigten Rohprodukte werden nochmals aus Essigester umkristallisiert. Ausbeute: 1.2 g (19 % d.Th.), Mp 296-298° C.

Analog zu Beispiel 3 erhaelt man:

| | Bezeichnung | Ausbeute [%] | Mp [°C] Loesungsm. |
|---|---|---|---|
| a) | 7,7-Dimethyl-2-(2-pyridyl)-6,7-dihydro-3H,5H-pyrrolo[2,3-f]benzimidazol-6-on x 0.3 $H_2O$ aus 5,6-Diamino-3,3-dimethyl-indolin-2-on und Pyridin-2-aldehyd | 25 | 182-187 Wasser |
| b) | 7,7-Dimethyl-2-(4-pyridyl)-6,7-dihydro-3H,5H-pyrrolo[2,3-f]benzimidazol-6-on x 4 $H_2O$ aus 5,6-Diamino-3,3-dimethyl-indolin-2-on und Pyridin-4-aldeyhd | 22 | 215 Wasser/ Ethanol |

Beispiel 4

7,7-Dimethyl-2-(4-N-oxy-pyridyl)-6,7-dihydro-3H,5H-pyrrolo-[2,3-f]benzimidazol-6-on x 3 $H_2O$

Eine Loesung von 7,7-Dimethyl-2-(4-pyridyl)-6,7-dihydro-3H, 5H-pyrrolo[2,3-f]benzimidazol-6-on (3.9 g, 0.014 mol) in Eisessig (50 ml) mit 30proz. $H_2O_2$ (20 ml) wird 2 Tage lang bei 50° C geruehrt und anschließend mit Wasser verduennt. Die ausgefallene Substanz wird abgesaugt und aus Dioxan/Wasser 1:1 umkristallisiert. Ausbeute 1.4 g (34 % d.Th.), Mp 260-262° C.

Analog zu Beispiel 4 erhaelt man:

| | Bezeichnung | Ausbeute [%] | Mp [°C] Loesungsm. |
|---|---|---|---|
| a) | 7,7-Dimethyl-2-(3-N-oxy-pyridyl)-6,7-dihydro-3H,5H-pyrrolo[2,3-f]benzimidazol-6-on x 1.25 $H_2O$ aus 7,7-Dimethyl-2-(3-pyridyl)-6,7-dihydro-3H,5H-pyrrolo[2,3-f]benzimidazol-6-on | 12 | 355-358 Wasser |
| b) | 7-Methyl-2-(4-N-oxy-pyridyl)-6,7-dihydro-3H,5H-pyrrolo[2,3-f]benzimidazol-6-on aus 7-Methyl-2-(4-pyridyl)-6,7-dihydro-3H,5H-pyrrolo[2,3-f]benzimidazol-6-on | 11 | > 300 Essigester |

Beispiel 5

2-(4-Pyridyl)-6,7-dihydro-3H,5H-pyrrolo[2,3-f]benzimidazol-6-on-Hydrochlorid

0.2 g (0.67 mmol) 6-Isonicotinoylamino-5-nitro-indolin-2-on wurden in 20 ml Eisessig suspendiert, mit 80 mg 10proz. Pd/Kohle versetzt und bei Normaldruck hydriert. Nach der Hydrierung wurde vom Katalysator abgetrennt, eingeengt und der Rueckstand in Alkohol geloest, filtriert und mit ethanolischer Salzsaeure angesaeuert. Durch Absaugen und Nachwaschen mit Ethanol erhaelt man 0.12 g (62.5 %)Produkt. Mp >300 °C, MS (Trimethylsilylderivat) : $M^+$ 394.466 $\frac{m}{z}$ 73, 75, 379, 451

Analog zu Beispiel 5 erhaelt man:

| Bezeichnung | Ausbeute [%] | Mp [°C] Loesungsm. |
|---|---|---|
| a) 7-Methyl-2-(4-pyridyl)-6,7-dihydro-3H,5H-pyrrolo[2,3-f]benzimidazol-6-on aus 6-Isonicotinoylamino-3-methyl-5-nitro-indolin-2-on | 76 | 315-318 Ethanol/ Wasser |
| b) 7-Methyl-2-(3-pyridyl)-6,7-dihydro-3H,5H-pyrrolo[2,3-f]benzimidazol-6-on aus 3-Methyl-6-nicotinoylamino-5-nitro-indolin-2-on | 32 | >300 Dioxan/ Methanol |
| c) 7-Ethyl-2-(4-pyridyl)-6,7-dihydro-3H,5H-pyrrolo[2,3-f]benzimidazol-6-on aus 3-Ethyl-6-isonicotinoylamino-5-nitro-indolin-2-on | 36 | 270-272 Essigester/ Methanol |
| d) 7-Ethyl-2-(3-pyridyl)-6,7-dihydro-3H,5H-pyrrolo[2,3-f]benzimidazol-6-on aus 3-Ethyl-6-nicotinoylamino-5-nitro-indolin-2-on | 63 | >300 Ethanol/ Wasser |

| Bezeichnung | Ausbeute [%] | Mp. [°C] Loesungsm. |
|---|---|---|
| e) 7-(2-Propyl)-2-(4-pyridyl)-6,7-dihydro-3H,5H-pyrrolo[2,3-f]benzimidazol-6-on aus 6-Isonicotinoylamino-3-(2-propyl)-5-nitro-indolin-2-on | 66 | 215-220 Ethanol/ Wasser |
| f) 7-Cyclopentyl-2-(4-pyridyl)-6,7-dihydro-3H,5H-pyrrolo[2,3-f]benzimidazol-6-on aus 3-Cyclopentyl-6-isonicotinoylamino-5-nitro-indolin-2-on | 75 | 200-204 Dioxan/ Methanol |

Die Ausgangsstoffe fuer die in Beispiel 5 beanspruchten Verbindungen koennen wie folgt hergestellt

werden.

a)

<u>6-Isonicotinoylamino-5-nitro-indolin-2-on</u>

5.5 g (21.7 mmol) 6-Isonicotinoylamino-indolin-2-on wurden portionsweise in 20 ml konzentrierten Schwefelsaeure geloest, mit Eis gekuehlt und 2.19 g (21.7 mmol) $KNO_3$ in konzentrierter $H_2SO_4$ geloest langsam zugetropft. Die Loesung wurde nach 2 Stunden auf Eis gegossen, neutralisiert und abgesaugt. Der Rueckstand wurde in 2 N Salzsaeure weitgehend geloest, filtriert, mit Floridin behandelt, abgesaugt und neutral gestellt. Man erhaelt 4.5 g (70 %) der Titelverbindung. Mp >300 °C

In analoger Weise werden die folgenden Verbindungen erhalten:

| Bezeichnung | Ausbeute [%] | Mp [°C] Lösungsm. |
|---|---|---|
| 6-Isonicotinoyl amino-3-methyl-5-nitro-indolin-2-on-Sulfat<br>aus<br>6-Isonicotinoylamino-3-methyl-indolin-2-on | 80 | >300<br>Wasser |
| 3-Methyl-6-nicotinoylamino-5-nitro-indolin-2-on<br>aus<br>3-Methyl-6-nicotinoylamino-indolin-2-on | 98 | 242-246<br>Ethanol |

| Bezeichnung | Ausbeute [%] | Mp. [°C] Lösungsm. |
|---|---|---|
| 3-Ethyl-6-isonicotinoylamino-5-nitro-indolin-2-on aus 3-Ethyl-6-isonicotinoylamino-indolin-2-on | 70 | 232-235 Ethanol |
| 3-Ethyl-6-nicotinoylamino-5-nitro-indolin-2-on aus 3-Ethyl-6-nicotinoylamino-indolin-2-on | 75 | 245-247 Ethanol |
| 6-Isonicotinoylamino-3-(2-propyl)-5-nitro-indolin-2-on-Sulfat aus 6-Isonicotinoylamino-3-(2-propyl)-indolin-2-on | 83 | 294 Wasser |
| 3-Cyclopentyl-6-isonicotinoylamino-5-nitro-indolin-2-on aus 3-Cyclopentyl-6-isonicotinoylamino-indolin-2-on | 85 | 186-190 Methanol |

b)

6-Isonicotinoylamino-indolin-2-on

5.0 g (27.1 mmol) 6-Amino-indolin-2-on-Hydrochlorid (Helv. Chim. Acta 20 , 373, 1937) wurden in 100 ml Methylenchlorid suspendiert und mit 3.7 ml (27.1 mmol) Triethylamin versetzt und 10 min geruehrt. Unter Eiskuehlung wurden 6.73 g (37.8 mmol) Isonicotinsaeurechlorid-Hydrochlorid und 5.24 ml (37.8 mmol) Triethylamin zugegeben. Anschließend wurden nochmals 3.7 ml (27.1 mmol) Triethylamin zugetropft. Nach 3 h wurde das Methylenchlorid abdestilliert, der Rueckstand mit Wasser durchgearbeitet und abgesaugt. Der Rueckstand wurde mit heißem Ethanol ausgeruehrt. Man erhaelt 5.6 g (82 %) der Titelverbindung. Mp 315-320 °C

In analoger Weise wird die folgende Verbindung erhalten:

| Bezeichnung | Ausbeute [%] | Mp [°C] Lösungsm. |
| --- | --- | --- |
| 6-Isonicotinoylamino-3-methyl-indolin-2-on aus 6-Amino-3-methyl-indolin-2-on-Hydrochlorid | 74 | >300 Ethanol |
| 3-Methyl-6-nicotinoylamino-indolin-2-on aus 6-Amino-3-methyl-indolin-2-on-Hydrochlorid | 88 | >300 Ethanol |
| 3-Ethyl-6-isonicotinoylamino-indolin-2-on aus 6-Amino-3-ethyl-indolin-2-on-Hydrochlorid | 50 | 258-260 Ethanol |

| Bezeichnung | Ausbeute [%] | Mp [°C] Lösungsm. |
| --- | --- | --- |
| 3-Ethyl-6-nicotinoylamino-indolin-2-on aus 6-Amino-3-ethyl-indolin-2-on-Hydrochlorid | 72 | 240-242 Ethanol |
| 6-Isonicotinoylamino-3-[2-propyl)-indolin-2-on aus 6-Amino-3-(2-propyl)indolin-2-on | 65 | 223-225 Ethanol |
| 3-Cyclopentyl-6-isonicotinoylamino-indolin-2-on aus 6-Amino-3-cyclopentyl-indolin-2-on | 95 | 178-180 Ethanol |

Beispiel 6

7,7-Diethyl-2-(4-pyridyl)-6,7-dihydro-3H,5H-pyrrolo[2,3-f]benzimidazol-6-on x $CH_3OH$

Zu einer Loesung von 5,6-Diamino-3,3-diethyl-indolin-2-on (4.4 g, 0.02 mol) in Methylenchlorid (100 ml) mit Triethylamin (8.4 ml) gibt man portionsweise Isonicotinsaeurechlorid Hydrochlorid (5.4 g, 0.03 mol), nach ca. 2 h gibt man Eisessig hinzu und die erhaltenen Kristalle werden abgesogen, nachgewaschen und getrocknet. Anschließend wird in einer Mischung von Ethanol (100 ml) mit konz. HCl (20 ml) ca. 20 h zum Rueckfluß erhitzt, im Vakuum eingedampft, der Rueckstand mit $NH_3$-Loesung digeriert, abgesaugt und getrocknet. Anschließend wird an Kieselgel (Laufmittel: Methylenchlorid/$NH_3$-gesaettigtes Methanol 15:1) gereinigt und aus Methanol kristallisiert.

Ausbeute: 2.4 g (35 % d.Th.), Mp. 216-219 °C

Analog zu Beispiel 6 erhaelt man:

| Bezeichnung | Ausbeute [%] | Mp. [°C] Lösungsm. |
|---|---|---|
| 7,7-Dimethyl-2-(4-pyridyl)-6,7-dihydro-3H,5H-pyrrolo[2,3-f]benzimidazol-6-on x 4 $H_2O$<br>aus<br>5,6-Diamino-3,3-dimethyl-indolin-2-on<br>und<br>Isonicotinsaeurechlorid Hydrochlorid | 41 | 215 Ethanol/ Wasser |

Die Ausgangsstoffe fuer die in Beispiel 6 beanspruchten Verbindungen koennen wie folgt hergestellt werden:

a)

5,6-Diamino-3,3-diethyl-indolin-2-on

Eine Loesung von 5-Amino-3,3-diethyl-6-nitro-indolin-2-on (10 g, 0.041 mol) in Ethanol (150 ml) wird an 10proz. Pd/C (0.6 g) bei RT hydriert. Es wird vom Katalysator abgesaugt, eingeengt und aus Ethanol umkristallisiert.

Ausbeute: 8.5 g (97 % d.Th.), Mp. 167-173 °C.

Nach der Vorschrift a lassen sich folgende Verbindungen in analoger Weise erhalten:

| Bezeichnung | Ausbeute [%] | Mp. [°C] Loesungsm. |
|---|---|---|
| 5,6-Diamino-3,3-dimethyl-indolin-2-on<br>aus<br>5-Amino-3,3-dimethyl-6-nitro-indolin-2-on | 98 | 255-256 Ethanol |

b)

5-Amino-3,3-diethyl-6-nitro-indolin-2-on

Eine Loesung von 5-Acetamido-3,3-diethyl-6-nitro-indolin-2-on (72 g, 0.25 mol) in Ethanol (500 ml) mit konz. HCl (100 ml) wird ca. 3 h am Rueckfluß erhitzt, mit Wasser (1000 ml) verduennt, abgesaugt und die erhaltenen Kristalle mit waessrigem Ethanol nachgewaschen und getrocknet.
Ausbeute: 54.7 g (89 % d.Th.), Mp. 267-272 °C.

Analog zu b lassen sich folgende Verbindungen erhalten:

| Bezeichnung | Ausbeute [%] | Mp. [°C] Loesungsm. |
|---|---|---|
| 5-Amino-3,3-dimethyl-6-nitro-indolin-2-on aus 5-Acetamido-3,3-dimethyl-6-nitro-indolin-2-on | 82 | 247 - 250 Ethanol/ Wasser |

c)

5-Acetamido-3,3-diethyl-6-nitro-indolin-2-on

Zu einer Loesung von 5-Acetamido-3,3-diethyl-indolin-2-on (84 g, 0.34 mol) in Essigsaeureanhydrid (800 ml) wird unter Kuehlung rauchende $HNO_3$ (24 ml) getropft und bei RT ca. 2 h nachgeruehrt. Es wird vorsichtig in Eiswasser gegeben und die erhaltenen Kristalle abgesaugt, mit Wasser nachgewaschen und getrocknet.
Ausbeute: 72 g (72 % d.Th.), Mp. 182-184 °C.

In analoger Weise koennen nach Vorschrift c die folgenden Verbindungen erhalten werden:

| Bezeichnung | Ausbeute [%] | Mp. [°C] Loesungsm. |
|---|---|---|
| 5-Acetamido-3,3-dimethyl-6-nitro-indolin-2-on aus 5-Acetamido-3,3-dimethyl-indolin-2-on | 73 | 225 - 228 Dichlor-methan |

d)

5-Acetamido-3,3-diethyl-indolin-2-on

Eine Suspension von 3,3-Diethyl-5-nitro-indolin-2-on (86 g, 0.372 mol) in Ethanol (700 ml) wird an 10proz. Pd/C (4 g) unter gutem Ruehren hydriert. Anschließend wird die klare Loesung abgesaugt und eine Probe aus Ethanol kristallisiert (Mp. 188-190 °C), die Hauptmenge der Loesung wird vorsichtig mit Essigsaeureanhydrid (50 ml) versetzt und im Vakuum eingedampft. Der Rueckstand wird mit Essigester

digeriert, abgesaugt, gewaschen und getrocknet. Kristallisation aus Essigester.
Ausbeute: 85 g (94 % d.Th.), Mp. 196-197 °C.

Nach d erhaelt man in analoger Weise folgende Verbindungen:

| Bezeichnung | Ausbeute [%] | Mp. [°C] Loesungsm. |
|---|---|---|
| 5-Acetamido-3,3-dimethyl-indolin-2-on aus 5-Nitro-3,3-dimethyl-indolin-2-on | 92 | 265 - 266 Essigester |

e)

3,3-Diethyl-5-nitro-indolin-2-on

Zu einer Loesung von 3,3-Diethyl-indolin-2-on (86 g, 0.462 mol) in 80proz. $H_2SO_4$ (500 ml) wird unter Kuehlung eine Loesung von rauchender $HNO_3$ (17 ml) in 80proz. $H_2SO$ (200 ml) zugetropft. Es wird ca. 30 min nachgeruehrt, auf Eis gegossen, abgesaugt, mit Wasser nachgewaschen und getrocknet.
Ausbeute: 86 g (79 % d.Th.), Mp. 174-176 °C.

In analoger Weise koennen nach e die folgenden Verbindungen erhalten werdend:

| Bezeichnung | Ausbeute [%] | Mp. [°C] Loesungsm. |
|---|---|---|
| 3,3-Dimethyl-5-nitro-indolin-2-on aus 3,3-Dimethyl-indolin-2-on | 78 | Ethanol |

Beispiel 7

7-Isopropyliden-2-(4-pyridyl)-6,7-dihydro-3H,5H-pyrrolo[2,3-f]benzimidazol-6-on

In eine Suspension von 2-(4-Pyridyl)-6,7-dihydro-3H,5H-pyrrolo[2,3-f]benzimidazol-6-on (0.4 g, 1.6 mmol) in Ethanol (20 ml) mit Aceton (10 ml) wird bis zur Saettigung Ammoniak eingeleitet und 2 h bei 60 °C geruehrt. Anschließend wird noch einmal Aceton (10 ml) zugesetzt und 4 h bei 60 °C geruehrt, dann wird eingeengt, in Ethanol aufgenommen, angesaeuert und vom Niederschlag abgesogen. Der erhaltene Rueckstand wird in Wasser geloest, neutral gestellt, von den Kristallen abgesaugt und aus Isopropanol/Essigester umkristallisiert.
Ausbeute: 40 mg (9 % d.Th.), Mp. >300 °C.

Beispiel 8

7-Ethoxycarbonyl-7-methyl-2-(4-pyridyl)-6,7-dihydro-3H,5H-pyrrolo[2,3-f]benzimidazol-6-on x HCl

Eine Loesung von 5-Amino-3-ethoxycarbonyl-3-methyl-6-(4-pyridinoylamino)-indolin-2-on (10.85 g, 32.2 mmol) in Ethanol (700 ml) mit konz. HCl (50 ml) wird ca. 12 h zum Rueckfluß erhitzt. Anschließend wird abgekuehlt, etwas eingeengt, von den Kristallen abgesaugt und aus Ethanol umkristallisiert.
Ausbeute: 9.6 g (79 % d.Th.), Mp. 288-290 °C.

Die Ausgangsstoffe fuer die in Beispiel 8 beanspruchten Verbindungen koennen wie folgt hergestellt werden:

a)

5-Amino-3-ethoxycarbonyl-3-methyl-6-(4-pyridinoylamino)-indolin-2-on

Eine Loesung von 3-Ethoxycarbonyl-3-methyl-5-nitro-6-(4-pyridinoylamino)-indolin-2-on (12.4 g, 32.2 mmol) in Ethanol (500 ml) wird an 10proz. Pd/C (1 g) bei Raumtemperatur hydriert. Es wird vom Katalysator abgesaugt, eingeengt und die erhaltene Substanz sofort weiter eingesetzt.

b)

3-Ethoxycarbonyl-3-methyl-5-nitro-6-(4-pyridinoylamino)-indolin-2-on

Zu einer Loesung von 3-Ethoxycarbonyl-3-methyl-6-(4-pyridinoylamino)-indolin-2-on (13 g, 0.038 mol) in konz. $H_2SO_4$ (90 ml) wird unter Kuehlung eine Loesung von Kaliumnitrat (3.9 g, 0.038 mol) in konz. $H_2SO_4$ - (30 ml) zugetropft. Es wird ca. eine Stunde nachgeruehrt, auf Eis gegossen, unter Kuehlung mit konz. $NH_3$-Loesung auf pH 8 gestellt, die Kristalle abgesaugt und aus Ethanol/Methylenchlorid umkristallisiert.
Ausbeute: 12.81 g (82 % d.Th.), Mp. 217-219 °C.

c)

3-Ethoxycarbonyl-3-methyl-6-(4-pyridinoylamino)-indolin-2-on

Zu einer gekuehlten Loesung von 6-Amino-3-ethoxycarbonyl-3-methyl-indolin-2-on (13 g, 0.048 mol) in Methylenchlorid (500 ml) werden Triethylamin (22.1 ml) und Pyridin-4-carbonsaeurechlorid Hydrochlorid (9.4 g, 0.053 mol) gegeben und ca. 2 h bei RT geruehrt. Anschließend wird zur Trockne eingeengt mit Wasser verruehrt, abgesaugt und der Rueckstand aus Ethanol/Methylenchlorid umkristallisiert. Ausbeute: 11.9 g (73 % d.Th.), Mp. 222-224 °C.

d)

6-Amino-3-ethoxycarbonyl-3-methyl-indolin-2-on

Eine Loesung von Methyl-(2,4-dinitro-phenyl)malonsaeurediethylester (27 g, 0.079 mol) in Ethanol (800 ml) wird an 10proz. Pd/C (1 g) bei RT hydriert. Anschließend wird vom Katalysator abgesaugt, mit ethanolischer HCl angesaeuert, eingeengt und durch Zusatz von Isopropanol auskristallisiert. Ausbeute: 13.19 g (71 % d-Th.), Mp. 248 °C.

Beispiel 9

7-Hydrazinocarbonyl-7-methyl-2-(4-pyridyl)-6,7-dihydro-3H,5H-pyrrolo[2,3-f]benzimidazol-6-on

Eine Loesung von 7-Ethoxycarbonyl-7-methyl-2-(4-pyridyl)-6,7-dihydro-3H,5H-pyrrolo[2,3-f]-benzimidazol-6-on Hydrochlorid (1 g, 2.7 mmol) in Ethanol (30 ml) mit Hydrazin-Hydrat (6 ml) wird ca. 8 h bei 80°C geruehrt. Anschließend wird abgesaugt, mit einer Loesung von Methanol (1000 ml) mit Methylenchlorid (500 ml) aufgekocht, etwas eingeengt und die Kristalle abgesaugt, mit Methanol nachgewaschen und getrocknet.
Ausbeute: 0.6 g (70 % d.Th.), Mp. >300°C.

Beispiel 10

7,7-Dimethyl-2-(4-pyridyl)-6,7-dihydro-3H,5H-pyrrolo[2,3-f]benzimidazol-6-on x 4 $H_2O$

Eine Loesung von 3,3-Dimethyl-5-nitro-6-(4-pyridinoylamino)-indolin-2-on (68 g, 0.209 mol) in Ethanol (3000ml) mit Triethylamin (30 ml) wird an 10proz. Pd/C hydriert. Anschließend wird vom Katalysator abgesaugt, eingeengt und das Rohprodukt in Ethanol (1000 ml) mit konz. HCl (200 ml) ca. 6 h am Rueckfluß erhitzt, eingeengt, mit Wasser verduennt und mit Ammoniakwasser auf pH 8 gestellt. Das entstandene Kristallisat wird abgesaugt, gewaschen und aus Ethanol/Wasser kristallisiert.
Ausbeute: 52.3 g (71 % d.Th.), Mp. 215°C.

Analog zu Beispiel 10 erhaelt man:

| | Bezeichnung | Ausbeute [%] | Mp. [°C] Loesungsm. |
|---|---|---|---|
| a) | 7,7-Dimethyl-2-(3-pyridyl)-6,7-dihydro-3H,5H-pyrrolo[2,3-f]benzimidazol-6-on x 3 $H_2O$ aus 3,3-Dimethyl-5-nitro-6-(3-pyridinoylamino)indolin-2-on | 45 | 331-335 Dioxan/ Wasser |

Die Ausgangsstoffe aus Beispiel 10 werden wie folgt hergestellt:

a)

3,3-Dimethyl-5-nitro-6-(4-pyridinoylamino)-indolin-2-on

Eine Loesung von 6-Amino-3,3-dimethyl-5-nitro-indolin-2-on (48.3 g, 0.22 mol) in Pyridin (400 ml) wird portionsweise mit Isonicotinsaeurechlorid Hydrochlorid (78.8 g, 0.44 mol) versetzt und ca. 2 h nachgeruehrt. Anschließend wird auf Wasser gegossen, neutral gestellt, abgesaugt und aus Ethanol umkristallisiert.
Ausbeute: 68.1 g (95 % d.Th.), Mp. 225-230°C.

Man erhaelt nach Vorschrift a) in analoger Weise folgende Verbindungen:

| Bezeichnung | Ausbeute [%] | Mp [°C] Loesungsm. |
|---|---|---|
| 3,3-Dimethyl-5-nitro-6-(3-pyridinoyl-amino)indolin-2-on<br>aus<br>6-Amino-3,3-dimethyl-5-nitro-indolin-2-on<br>und<br>Nicotinsaeurechlorid Hydrochlorid | 95 | 225-230<br>Ethanol |

Beispiel 11

7-(2-Methyl-propyl)-2-(4-pyridyl)-6,7-dihydro-3H,5H-pyrrolo[2,3-f]benzimidazol-6-on x 1.5 $H_2O$

Zu einer Loesung von 5,6-Diamino-3-(2-methyl-propyl)-indolin-2-on (1.3 g, 0.006 mol) in Methylenchlorid (40 ml) mit Triethylamin (3 ml) gibt man portionsweise Isonicotinsaeurechlorid Hydrochlorid (1.6 g, 0.009 mol), extrahiert nach ca. 2 h mit Wasser und engt die organische Phase ein. Der feste Rueckstand wird in Ethanol (70 ml) mit konz. HCl (10 ml) ueber Nacht am Rueckfluß erhitzt, eingeengt, mit waessrigem Ammoniak durchgeruehrt und mit Methylenchlorid/Methanol extrahiert. Die organische Phase wird eingeengt und an Kieselgel (Laufmittel: Methylenchlorid/$NH_3$-gesaettigtes Methanol 9:1) gereinigt.
Ausbeute: 0.65 g (35 % d.Th.), Mp. 200-202° C.

Ausgangsstoffe für Beispiel 11 werden in folgender Weise erhalten:

a)

5,6-Diamino-3-(2-methyl-propyl)indolin-2-on

Eine Loesung von 6-Amino-3-(2-methyl-propyl)-5-nitro-indolin-2-on (1.5 g, 0.006 mol) in Methanol (50 ml) wird an 10proz. Pd/C (0.3 g) hydriert. Es wird vom Katalysator abgesaugt, eingeengt und aus Ethanol umkristallisiert. Ausbeute: 1.3 g (99 % d.Th.)

b)

6-Amino-3-(2-methyl-propyl)-5-nitro-indolin-2-on

Eine Loesung von 6-Acetamido-3-(2-methyl-propyl)-5-nitro-indolin-2-on (2.9 g, 0.01 mol) in Ethanol (50 ml) mit konz. HCl (3 ml) wird ca. 30 min am Rueckfluß erhitzt, eingeengt und an Kieselgel (Laufmittel: Methylenchlorid/Methanol $NH_3$ gesaettigt 20:1) gereinigt.
Ausbeute: 1.5 g (60 % d.Th.)

c)

6-Acetamido-3-(2-methyl-propyl)-5-nitro-indolin-2-on

Eine Loesung von 6-Acetamido-3-(2-methyl-propyl)indolin-2-on (4.2 g, 0.017 mol) in Acetanhydrid (50 ml) wird unter Kuehlung mit rauchender $HNO_3$ (0.8 ml, 0.019 mol) versetzt und ca. 30 min nachgeruehrt. Anschließend wird vorsichtig auf Eis gegossen und die Kristalle abgesaugt, mit Wasser gewaschen und getrocknet.
Ausbeute: 3.2 g (66 % d.Th.), Mp. 192-197 °C.

d)

6-Acetamido-3-(2-methyl-propyl)indolin-2-on

Eine Loesung von 6-Acetamido-3-isopropyliden-indolin-2-on (6.4 g, 0.026 mol) in Methanol (100 ml) wird an 10proz. Pd/C (0.6 g) hydriert. Anschließend wird vom Katalysator abgesaugt und die Loesung zur Trockene eingeengt.
Ausbeute: 5.5 g (84 % d.Th.), Mp. 214-216 °C.

e)

6-Acetamido-3-isopropyliden-indolin-2-on

Zu einer Suspension von 6-Acetamido-indolin-2-on (9 g, 0.047 mol) in Ethanol (50 ml) mit Isobutyraldehyd (3.4 g, 0.047 mol) wird eine Loesung von NaOH (1.9 g) in Wasser (2 ml) getropft. Nach ca. 5 h wird im Vakuum zur Trockne eingeengt und an Kiesel gel (Laufmittel: Methylenchlorid/Methanol $NH_3$ gesaettigt) gereinigt.
Ausbeute: 7.6 g (65 % d.Th.) Schaum, Mp. 93 °C.

Beispiel 12

7,7-Dimethyl-2-(4-pyridyl)-6,7-dihydro-3H,5H-pyrrolo[2,3-f]benzimidazol-6-thion x 2 $H_2O$

Eine Loesung von 7,7-Dimethyl-2-(4-pyridyl)-6,7-dihydro-3H,5H-pyrrolo[2,3-f]benzimidazol-6-on (2.0 g, 7.2 mmol) in Pyridin (35 ml) wird mit $P_4S_{10}$ (4 g) unter gutem Ruehren ca. 5 h auf 100 °C erhitzt. Anschließend wird mit Eiswasser zersetzt, alkalisch gestellt, die gewuenschte Substanz extrahiert und an Kieselgel (Laufmittel; Methylenchlorid/$NH_3$) gesaettigtes Methanol 20:1) gereinigt.
Ausbeute: 1.7 g (72 % d.Th.), Mp. 205-220 °C.

Beispiel 13

7,7-Dimethyl-2-(4-(3-hydroxy-pyridyl))-6,7-dihydro-3H,5H-pyrrolo[2,3-f]benzimidazol-6-on

Zu einer Mischung von 3-Hydroxy-pyridin-4-carbonsaeure (2.8 g, 0.02 mol) in DMF (30 ml) mit wasserfreiem $CaSO_4$ (3 g) und Hydroxybenzotriazol (3.4 g, 0.025 mol) wird unter Ruehren bei 0 °C eine Loesung von DCC (5.2 g, 0.025 mol) in DMF (20 ml) gegeben. Anschließend wird mit 5,6-Diamino-3,3-dimethyl-indolin-2-on (2.8 g, 0.015 mol) versetzt und nach kurzem Nachruehren eingeengt, mit Wasser digeriert und abgesaugt. Der Rueckstand wird mit Ethanol (200 ml) und konz. HCl (40 ml) 2 d am Rueckfluß erhitzt, nach dem Abkuehlen abgesaugt und das Filtrat im Vakuum eingeengt, mit Ammoniakwasser digeriert und abgesaugt. Es wird saeulenchromatographisch an Kieselgel (Laufmittel: Methylenchlorid/Methanol/Essigsaeure 10:1:1) gereinigt.
Ausbeute: 1.4 g (24 % d.Th.), Mp. >300 °C.

Analog zu Beispiel 13 erhaelt man:

| | Bezeichnung | Ausbeute [%] | Mp. [°C] Loesungsm. |
|---|---|---|---|
| a) | 7,7-Dimethyl-2-(4-(2-hydroxy-pyridyl))-6,7-dihydro-3H,5H-pyrrolo[2,3-f]benzimidazol-6-on x 2 $H_2O$<br>aus<br>5,6-Diamino-3,3-dimethyl-indolin-2-on<br>und<br>2-Hydroxy-pyridin-4-carbonsaeure | 22 | >360<br>Wasser |

Beispiel 14

7,7-Dimethyl-2-(4-(pyridyl)-6,7-dihydro-3H,5H-pyrrolo[2,3-f]benzimidazol-6-on

Eine Loesung von 3,3-Dimethyl-6-nitro-5-(4-pyridinoylamino)indolin-2-on (33 g, 0.1 mol) in Ethanol (1300 ml) mit Triethylamin (13 ml) wird an 10proz. Pd/C hydriert. Anschließend wird vom Katalysator abgesaugt, eingeengt und in Ethanol (500 ml) mit konz. HCl (100 ml) ca. 6 h am Rueckfluß erhitzt. Es wird eingeengt, mit Ammoniakwasser digeriert und das entstandene Produkt abgesaugt, mit Wasser gewaschen, aus Ethanol/Wasser kristallisiert und aus Methanol umkristallisiert.
Ausbeute: 19.5 g (70 % d.Th.), Mp. 285-288° C.

Die Ausgangsverbindungen werden wie folgt hergestellt:

a)

3,3-Dimethyl-6-nitro-5-(4-pyridinoylamino)indolin-2-on

Zu einer Loesung von 3,3-Dimethyl-5-(5-pyridinoylamino)indolin-2-on (5.5 g, 0.02 mol) in Eisessig (50 ml) wird unter Kuehlung rauchende $HNO_3$ (2.5 ml) getropft und bei RT ca. 2 h nachgeruehrt. Es wird vorsichtig auf Eis gegossen, neutralisiert und die erhaltenen Kristalle abgesaugt, mit Wasser gewaschen und getrocknet.
Ausbeute: 5.2 g (81 % d.Th.), Mp. 310-318° C (aus Methanol)

b)

3,3-Dimethyl-5-(4-pyridinoylamino)indolin-2-on

Eine Suspension von 5-Amino-3,3-dimethyl-indolin-2-on (5.0 g, 0.028 mol) in Methylenchlorid (100 ml) mit Triethylamin (14 ml) wird unter Kuehlung mit Isonicotinsaeurechlorid Hydrochlorid (7.0 g, 0.04 mol) versetzt. Anschließend wird eingeengt, mit Wasser digeriert, abgesaugt und aus Ethanol umkristallisiert.
Ausbeute: 5.8 g (73 % d.Th.), Mp. >300° C.

c)

5-Amino-3,3-dimethyl-indolin-2-on

Eine Suspension von 5-Nitro-indolin-2-on (100 g, 0.48 mol) in Methanol (2500 ml) mit Eisessig (200 ml) wird an 10proz. Pd/C (10 g) bei 40 ° C unter gutem Ruehren hydriert. Die anschließende klare Loesung wird vom Katalysator abgesaugt und eingeengt.
Ausbeute: 82 g (96 % d.Th.), Mp. 185-191 ° C (HCl-Salz aus Methanol).

d)

3,3-Dimethyl-5-nitro-indolin-2-on

Zu einer Loesung von 3,3-Dimethyl-indolin-2-on (100 g, 0.62 mol) in 80proz. $H_2SO_4$ (500 ml) wird unter Kuehlung eine Loesung von rauchender $HNO_3$ (22 ml) in 80proz. $H_2SO_4$ (200 ml) zugetropft. Es wird ca. 30 min nachgeruehrt, auf Eis gegossen, abgesaugt, mit Wasser nachgewaschen und getrocknet.
Ausbeute: 100 g (78 % d.Th.), Mp. 192-196 ° C (Essigester/Heptan).

Beispiel 15

7,7-Dimethyl-2-(4-pyridyl)-6,7-dihydro-3H,5H-pyrrolo[2,3-f]benzimidazol-6-on x 4 $H_2O$

Zu einer Loesung von 5-Amino-3,3-dimethyl-6-nitro-indolin-2-on (5 g, 0.023 mol) in Pyridin (50 ml) wird portionsweise Isonicotinsaeurechlorid Hyrochlorid (8 g, 0.04 mol) gegeben und ca. 2 h nachgeruehrt. Anschließend wird auf Wasser gegossen, mit Ammoniakwasser digeriert, abgesaugt, mit Wasser gewaschen und der Rueckstand in Ethanol (300 ml) mit Triethylamin (3 ml) an 10proz. Pd/C hydriert. Es wird vom Katalysator abgesaugt, eingeengt und das Rohprodukt in Ethanol (100 ml) mit konz. HCl (20 ml) ca. 6 h am Rueckfluß erhitzt, eingeengt, neutral gestellt und das erhaltene Produkt abgesaugt, gewaschen und aus Ethanol/Wasser kristallisiert.
Ausbeute: 5.5 g (69 % d.Th.), Mp. 215 ° C.

Analog zu Beispiel 15 erhaelt man:

| | Bezeichnung | Ausbeute [%] | Mp. [°C] Loesungsm. |
|---|---|---|---|
| a) | 7,7-Dimethyl-2-(2-pyridyl)-6,7-dihydro-3H,5H-pyrrolo[2,3-f]benzimidazol-6-on x 0.3 $H_2O$ aus 5-Amino-3,3-dimethyl-6-nitro-indolin-2-on und Picolinsaeurechlorid | 60 | 182-187 Wasser |
| b) | 7,7-Dimethyl-2-(3-pyridyl)-6,7-dihydro-3H,5H-pyrrolo[2,3-f]benzimidazol-6-on x 3 $H_2O$ aus 5-Amino-3,3-dimethyl-6-nitro-indolin-2-on und Nicotinsaeurechlorid Hydrochlorid | 55 | 331-335 Dioxan/ Wasser |

| | Bezeichnung | Ausbeute [%] | Mp. [°C] Loesungsm. |
|---|---|---|---|
| c) | 7,7-Diethyl-2-(4-pyridyl)-6,7-dihydro-3H,5H-pyrrolo[2,3-f]benzimidazol-6-on x $CH_3OH$ aus 5-Amino-3,3-diethyl-6-nitro-indolin-2-on und Isonicotinsaeurechlorid Hydrochlorid | 59 | 216-219 Methanol |

Beispiel 16

7,7-Dimethyl-2-(4-pyridyl)-6,7-dihydro-3H,5H-pyrrolo[2,3-f]benzimidazol-6-on x 4 $H_2O$

Zu einer Loesung von 6-Amino-5-nitro-3,3-dimethyl-indolin-2-on (221 mg, 1 mmol) in DMF (2 ml) mit Pyridin (0.5 ml) und Isonicotinsaeure (123 mg, 4 mmol) gibt man $POCl_3$ (0.15 ml), laeßt ca. 3 h Nachruehren, gießt auf Eiswasser und saugt die erhaltenen Kristalle ab. Anschließend wird in Ethanol (10 ml) mit Triethylamin (0.2 ml) an Pd/C hydriert, vom Katalysator abgesaugt, eingeengt und ca. 6 h in einer Loesung von Ethanol (5 ml) mit konz. HCl (1 ml) zum Rueckfluß erhitzt. Es wird eingeengt, mit Ammoniakwasser digeriert und aus Ethanol/Wasser umkristallisiert.
Ausbeute: 220 mg (63 % d.Th.), Mp. 215 °C.

Analog zu Beispiel 16 erhaelt man:

| | Bezeichnung | Ausbeute [%] | Mp. [°C] Loesungsm. |
|---|---|---|---|
| a) | 7,7-Diethyl-2-(4-pyridyl)-6,7-dihydro-3H,5H-pyrrolo[2,3-f]benzimidazol-6-on x $CH_3OH$ aus 5-Amino-6-nitro-3,3-diethyl-indolin-2-on und Isonicotinsaeure | 62 | 216-219 Methanol |
| b) | 7,7-Dimethyl-2-(3-(6-methyl-pyridyl))-6,7-dihydro-3H,5H-pyrrolo[2,3-f]benzimidazol-6-on aus 6-Amino-5-nitro-3,3-dimethyl-indolin-2-on und 6-Methylnicotinsaeure | 55 | >360 Essigester |

| | Bezeichnung | Ausbeute [%] | Mp. [°C] Loesungsm. |
|---|---|---|---|
| c) | 7,7-Dimethyl-2-(4-(2-methyl-pyridyl))-6,7-dihydro-3H,5H-pyrrolo[2,3-f]benzimidazol-6-on x 0.6 $H_2O$ aus 6-Amino-5-nitro-3,3-dimethyl-indolin-2-on und 2-Methylisonicotinsaeure | 45 | 311-313 Aceton |
| d) | 7,7-Dimethyl-2-(4-pyridyl-ethyl)-6,7-dihydro-3H,5H-pyrrolo[2,3-f]benzimidazol-6-on x 0.6 $H_2O$ aus 6-Amino-5-nitro-3,3-dimethyl-indolin-2-on und 4-Pyridylpropionsaeure | 43 | 150-154 Wasser |

## Ansprüche

1. Verbindungen der Formel I

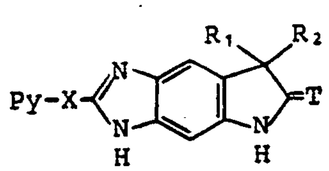

(I),

in welcher

$R_1$ ein Wasserstoffatom, eine $C_1$-$C_6$ Alkyl-, $C_2$-$C_6$ Alkenyl- oder eine $C_3$-$C_7$ Cycloalkylgruppe bedeutet,

$R_2$ ein Wasserstoffatom, eine $C_1$-$C_6$ Alkyl-, $C_2$-$C_6$ Alkenyl-, oder Cyangruppe, eine durch eine Hydroxy-, Alkyl-, Alkoxy-, Amino-, Alkylamino-, Dialkylamino- oder Hydrazinogruppe substituierte Carbonylgruppe bedeutet wobei die vorgenannten Alkylteile 1-6 C-Atome enthalten, oder mit $R_1$ zusammen eine $C_3$-$C_8$ Cycloalkylengruppe darstellt oder $R_1$ und $R_2$ zusammen eine $C_2$-$C_6$ Alkyliden- oder $C_3$-$C_8$ Cycloalkylidengruppe bilden,

X einen Valenzstrich, eine $C_1$-$C_4$-Alkylengruppe oder die Vinylengruppe bedeutet,

T gleich Sauerstoff oder Schwefel bedeutet,

Py einen 2-, 3- oder 4-Pyridylrest darstellt, der gegebenenfalls am Ringheteroatom ein Sauerstoffatom traegt und/oder durch eine oder mehrere $C_1$-$C_6$ Alkyl-, $C_1$-$C_6$ Alkoxy-, Hydroxy-, Cyano- oder Nitrogruppen, sowie durch Halogen substituiert sein kann,

deren Tautomere und deren physiologisch vertraegliche Salze anorganischer und organischer Saeuren.

2. Verbindungen gemäß Anspruch 1, in denen $R_1$ Wasserstoff, Methyl, Ethyl, 2-Propyl, 2-Methyl-propyl oder Cyclopentyl, $R_2$ Wasserstoff, Methyl, Ethyl, Ethoxycarbonyl oder Hydrazinocarbonyl, $R_1$ und $R_2$ zusammen mit dem C-Atom, an das sie gebunden sind, einen Cyclopentan-Ring oder zusammen einen

Isopropyliden-Rest bilden, X eine Valenzbindung, eine Methylen-, Ethylen- oder Vinylgruppe, T Sauerstoff oder Schwefel und Py einen Pyridylen-N-oxid-Rest oder einen Pyridyl-Rest bedeuten, der ein- oder mehrfach durch Hydroxyl, Methoxy, Methyl oder Halogen substituiert sein kann, deren Tautomere und deren physiologisch verträgliche Salze.

**3.** Verbindungen gemäß Anspruch 1 oder 2, in denen T Sauerstoff, X eine Valenzbindung, $R_1$ Wasserstoff oder Methyl, $R_2$ Methyl, Ethyl oder Ethoxycarbonyl und Py einen Pyridin-N-oxid-Rest oder einen Pyridyl-Rest darstellen, der durch Hydroxy oder Methyl substituiert sein kann, deren Tautomere und deren physiologisch verträgliche Salze.

**4.** Verbindungen gemäß Anspruch 1, ausgewählt hiervon:
7,7 Dimethyl-2-(4-pyridyl)-6,7-dihydro-3H,5H-pyrrolo[2,3-f]benzimidazol-6-on
7,7-Dimethyl-2-(-N-oxy-pyridyl)-6,7-dihydro-3H,5H-pyrrolo[2,3-f]benzimidazol-6-on
7-Ethoxycarbonyl-7-methyl-2-(4-pyridyl)-6,7-dihydro-3H,5H-pyrrolo[2,3-f]benzimidazol-6-on
7,7-Dimethyl-2-(4-(2-methyl-pyridyl))-6,7-dihydro-3H,5H-pyrrolo[2,3-f]benzimidazol-6-on

**5.** Verfahren zur Herstellung von Verbindungen der Formel I

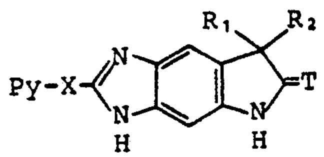

(I),

in welcher

$R_1$ ein Wasserstoffatom, eine $C_1$-$C_6$ Alkyl-, $C_2$-$C_6$ Alkenyl- oder eine $C_3$-$C_7$ Cycloalkylgruppe bedeutet,

$R_2$ ein Wasserstoffatom, eine $C_1$-$C_6$ Alkyl-, $C_2$-$C_6$ Alkenyl-, oder Cyangruppe, eine durch eine Hydroxy-, Alkyl-, Alkoxy-, Amino-, Alkylamino-, Dialkylamino- oder Hydrazinogruppe substituierte Carbonylgruppe bedeutet wobei die vorgenannten Alkylteile 1-6 C-Atome enthalten, oder mit $R_1$ sammen eine $C_3$-$C_8$ Cycloalkylengruppe darstellt oder $R_1$ und $R_2$ zusammen eine $C_2$-$C_6$ Alkyliden- oder $C_3$-$C_8$ Cyloalkylidengruppe bilden,

X einen Valenzstrich, eine $C_1$-$C_4$-Alkylengruppe oder die Vinylengruppe bedeutet,

T gleich Sauerstoff oder Schwefel bedeutet,

Py einen 2-, 3- oder 4-Pyridylrest darstellt, der gegebenenfalls am Ringheteroatom ein Sauerstoffatom traegt und/oder durch eine oder mehrere $C_1$-$C_6$ Alkyl-, $C_1$-$C_6$ Alkoxy-, Hydroxy-, Cyano- oder Nitrogruppen, sowie durch Halogen substituiert sein kann,

deren Tautomere und deren physiologisch vertraegliche Salze anorganischer und organischer Saeuren, dadurch gekennzeichnet, daß man in an sich bekannter Weise

a) eine Verbindung der Formel XII

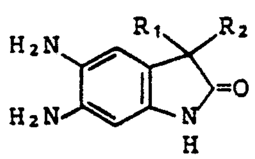

(XII),

in der $R_1$ und $R_2$ die oben angegebene Bedeutung besitzen, mit einer Verbindung der Formel XIV

Py-X-CO-Y (XIV),

in der Py und X die angegebene Bedeutung besitzen und Y entweder Wasserstoff oder einen leicht

abspaltbaren Rest darstellt,
umsetzt und die erhaltene Verbindung in saurem Medium cyclisiert, oder
b) eine Verbindung der Formel XIX

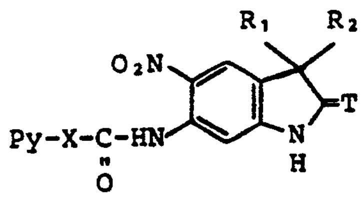 (XIX),

oder der Formel XX

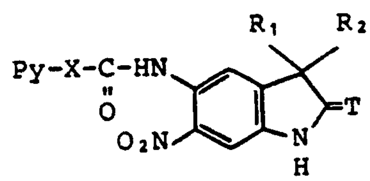 (XX),

in denen $R_1$, $R_2$, T, Py und X die oben angegebenen Bedeutungen besitzen, reduziert und anschließend cyclisiert oder
c) eine Verbindung der Formel XXVI

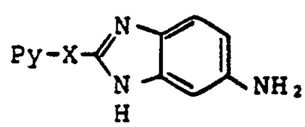 (XXVI)

mit einem reaktiven Carbonsäure-Derivat der Formel XXVII

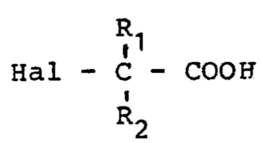 (XXVII)

acyliert und cyclisiert,
wobei $R_1$, $R_2$, Py, X und Hal die angegebenen Bedeutungen haben, oder
d) eine Verbindung der Formel XXVIII

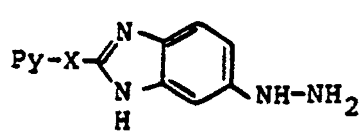 (XXVIII)

mit einem reaktiven Carbonsäure-Derivat der Formel XXIX

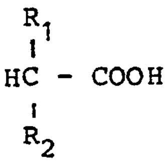

(XXIX)

zu entsprechenden Hydraziden umgesetzt und diese dann unter alkalischen Bedingungen cyclisiert, wobei $R_1$, $R_2$, Py und X die angegebenen Bedeutungen haben und anschließend gewünschtenfalls erhaltene Verbindungen der Formel I in andere Verbindungen der Formel I überführt sowie gewünschtenfalls die Verbindungen mit anorganischen oder organischen Säuren in physiologisch verträgliche Salze überführt.

**6.** Arzneimittel, enthaltend eine Verbindung gemäß Anspruch 1, 2 oder 3 neben üblichen Träger- und Hilfsstoffen.

**7.** Verwendung von Verbindungen gemäß Anspruch 1, 2,oder 3 zur Herstellung von Arzneimitteln zur Prophylaxe bzw. Behandlung von Herz- und Kreislauferkrankungen.

**8.** Verbindungen der Formel XXX in der

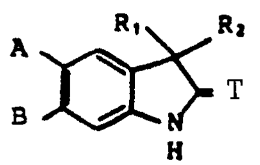

(XXX)

$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet

$R_2$ ein Wasserstoffatom, eine $C_1$-$C_6$-Alkyl-, $C_2$-$C_6$-Alkenyl-, oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino-oder Hydrazinogruppe substituierte Carbonylgruppe bedeutet oder mit $R_1$ zusammen eine $C_3$-$C_8$-Cycloalkylengruppe darstellt oder $R_1$ und $R_2$ zusammen eine $C_2$-$C_6$-Alkyliden- oder $C_3$-$C_8$-Cycloalkylidengruppe bilden,

A ein Wasserstoffatom, $NH_2$, $NO_2$ oder Py-X-CO-NH- und

B ein Wasserstoffatom, $NH_2$, $NO_2$ oder Py-X-CO-NH- bedeuten, wobei

X einen Valenzstrich, eine $C_1$-$C_4$-Alkylengruppe oder die Vinylengruppe bedeutet und

Py einen 2-, 3- oder 4-Pyridylrest darstellt, der gegebenenfalls am Ringheteroatom ein Sauerstoffatom trägt und/oder durch eine oder mehrere $C_1$-$C_6$-Alkyl-, $C_1$-$C_6$-Alkoxy-, Hydroxy-, Cyano- oder Nitrogruppen sowie durch Halogen substituiert sein kann, und

T gleich Sauerstoff oder Schwefel bedeutet,
mit der Maßgabe, daß A und B nur gleichzeitig $NH_2$ bedeuten dürfen, und
mit der maßgabe, daß wenn $R_1$ Wasserstoff und $R_2$ Wasserstoff oder eine Alkylgruppe darstellt, oder wenn $R_1$ Wasserstoff, eine Alkyl- oder Alkoxygruppe substituierte Carbonylgruppe darstellt, A nicht Wasserstoff oder eine Aminogruppe ist.

Patentansprüche für folgenden Vertragsstaat: AT

**1.** Verbindungen der Formel I

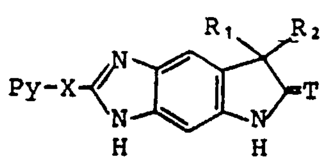

(I),

in welcher

$R_1$ ein Wasserstoffatom, eine $C_3$-$C_6$ Alkyl-, $C_2$-$C_6$ Alkenyl- oder eine $C_3$-$C_7$ Cycloalkylgruppe bedeutet,

$R_2$ ein Wasserstoffatom, eine $C_1$-$C_6$ Alkyl-, $C_2$-$C_6$ Alkenyl-, oder Cyangruppe, eine durch eine Hydroxy-, Alkyl-, Alkoxy-, Amino-, Alkylamino-, Dialkylamino- oder Hydrazinogruppe substituierte Carbonylgruppe bedeutet wobei die vorgenannten Alkylteile 1-6 C-Atome enthalten, oder mit $R_1$ zusammen eine $C_3$-$C_8$ Cycloalkylengruppe darstellt oder $R_1$ und $R_2$ zusammen eine $C_2$-$C_6$ Alkyliden- oder $C_3$-$C_8$ Cycloalkylidengruppe bilden,

X einen Valenzstrich, eine $C_1$-$C_4$-Alkylengruppe oder die Vinylengruppe bedeutet,

T gleich Sauerstoff oder Schwefel bedeutet,

Py einen 2-, 3- oder 4-Pyridylrest darstellt, der gegebenenfalls am Ringheteroatom ein Sauerstoffatom traegt und/oder durch eine oder mehrere $C_1$-$C_6$ Alkyl-, $C_1$-$C_6$ Alkoxy-, Hydroxy-, Cyano- oder Nitrogruppen, sowie durch Halogen substituiert sein kann,
deren Tautomere und deren physiologisch vertraegliche Salze anorganischer und organischer Saeuren.

**2.** Verbindungen gemäß Anspruch 1, in denen $R_1$ Wasserstoff, Methyl, Ethyl, 2-Propyl, 2-Methyl-propyl oder Cyclopentyl, $R_2$ Wasserstoff, Methyl, Ethyl, Ethoxycarbonyl oder Hydrazinocarbonyl, $R_1$ und $R_2$ zusammen mit dem C-Atom, an das sie gebunden sind, einen Cyclopentan-Ring oder zusammen einen Isopropyliden-Rest bilden, X eine Valenzbindung, eine Methylen-, Ethylen- oder Vinylgruppe, T Sauerstoff oder Schwefel und Py einen Pyridylen-N-oxid-Rest oder einen Pyridyl-Rest bedeuten, der ein- oder mehrfach durch Hydroxyl, Methoxy, Methyl oder Halogen substituiert sein kann, deren Tautomere und deren physiologisch verträgliche Salze.

**3.** Verbindungen gemäß Anspruch 1 oder 2, in denen T Sauerstoff, X eine Valenzbindung, $R_1$ Wasserstoff oder Methyl, $R_2$ Methyl, Ethyl oder Ethoxycarbonyl und Py einen Pyridin-N-oxid-Rest oder einen Pyridyl-Rest darstellen, der durch Hydroxy oder Methyl substituiert sein kann, deren Tautomere und deren physiologisch verträgliche Salze.

**4.** Verbindungen gemäß Anspruch 1, ausgewählt hiervon:
7,7 Dimethyl-2-(4-pyridyl)-6,7-dihydro-3H,5H-pyrrolo[2,3-f]benzimidazol-6-on
7,7-Dimethyl-2-(4-N-oxy-pyridyl)-6,7-dihydro-3H,5H-pyrrolo[2,3-f]benzimidazol-6-on
7-Ethoxycarbonyl-7-methyl-2-(4-pyridyl)-6,7-dihydro-3H,5H-pyrrolo[2,3-f]benzimidazol-6-on
7,7-Dimethyl-2-(4-(2-methyl-pyridyl))-6,7-dihydro-3H,5H-pyrrolo[2,3-f]benzimidazol-6-on

**5.** Verfahren zur Herstellung von Verbindungen der Formel I

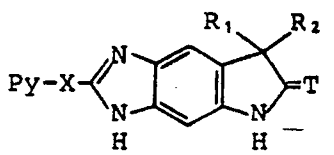

(I),

in welcher

$R_1$ ein Wasserstoffatom, eine $C_1$-$C_6$ Alkyl-, $C_2$-$C_6$ Alkenyl- oder eine $C_3$-$C_7$ Cycloalkylgruppe bedeutet,

$R_2$ ein Wasserstoffatom, eine $C_1$-$C_6$ Alkyl-, $C_2$-$C_6$ Alkenyl-, oder Cyangruppe, eine durch eine Hydroxy-, Alkyl-, Alkoxy-, Amino-, Alkylamino-, Dialkylamino- oder Hydrazinogruppe substituierte Carbonylgruppe bedeutet wobei die vorgenannten Alkylteile 1-6 C-Atome enhalten, oder mit $R_1$ zusammen eine $C_3$-$C_8$ Cycloalkylengruppe darstellt oder $R_1$ und $R_2$ zusammen eine ($C_2$-$C_6$) Alkyliden- oder $C_3$-$C_8$ Cycloalkylidengruppe bilden,

X einen Valenzstrich, eine $C_1$-$C_4$-Alkylengruppe oder die Vinylengruppe bedeutet,

T gleich Sauerstoff oder Schwefel bedeutet,

Py einen 2-, 3- oder 4-Pyridylrest darstellt, der gegebenenfalls am Ringheteroatom ein Sauerstoffatom traegt und/oder durch eine oder mehrere $C_1$-$C_6$ Alkyl-, $C_1$-$C_6$ Alkoxy-, Hydroxy-, Cyano- oder Nitrogruppen, sowie durch Halogen substituiert sein kann,
deren Tautomere und deren physiologisch vertraegliche Salze anorganischer und organischer Saeuren, dadurch gekennzeichnet, daß man in an sich bekannter Weise
a) eine Verbindung der Formel XII

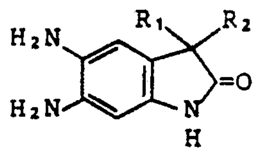

(XII),

in der $R_1$ und $R_2$ die oben angegeben Bedeutung besitzen, mit einer Verbindung der Formel XIV

Py-X-CO-Y (XIV),

in der Py und X die angegebene Bedeutung besitzen und Y entweder Wasserstoff oder einen leicht abspaltbaren Rest darstellt,
umsetzt und die erhaltene Verbindung in saurem Medium cyclisiert, oder
b) eine Verbindung der Formel XIX

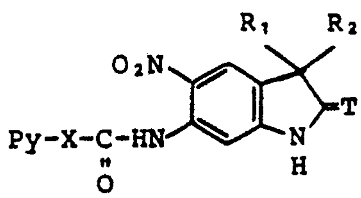

(XIX),

oder der Formel XX

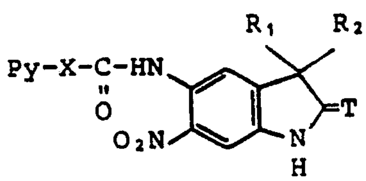

(XX),

in denen $R_1$, $R_2$, T, Py und X die oben angegebenen Bedeutungen besitzen,
reduziert und anschließend cyclisiert oder
c) eine Verbindung der Formel XXVI

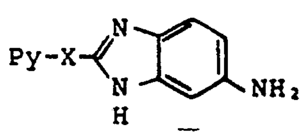

(XXVI)

mit einem reaktiven Carbonsäure-Derivat der Formel XXVII

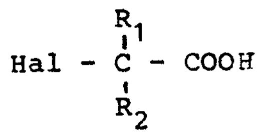 (XXVII)

acyliert und cyclisiert,
wobei $R_1$, $R_2$, Py, X und Hal die angegebenen Bedeutungen haben, oder
d) eine Verbindung der Formel XXVIII

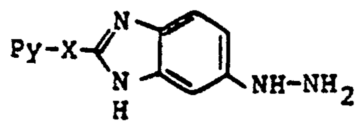 (XXVIII)

mit einem reaktiven Carbonsäure-Derivat der Formel XXIX

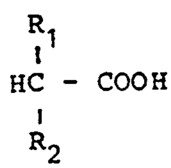 (XXIX)

zu entsprechenden Hydraziden umgesetzt und diese dann unter alkalischen Bedingungen cyclisiert, wobei $R_1$, $R_2$, Py und X die angegebenen Bedeutungen haben und anschließend gewünschtenfalls erhaltene Verbindungen der Formel I in andere Verbindungen der Formel I überführt sowie gewüschtenfalls die Verbindungen mit anorganischen oder organischen Säuren in physiologisch verträgliche Salze überführt.

6. Arzneimittel, enthaltend eine Verbindung gemäß Anspruch 1, 2 oder 3 neben üblichen Träger- und Hilfsstoffen.

7. Verwendung von Verbindungen gemäß Anspruch 1, 2, oder 3 zur Herstellung von Arzneimitteln zur Prophylaxe bzw. Behandlung von Herz- und Kreislauferkrankungen.

8. Verbindungen der Formel XXX

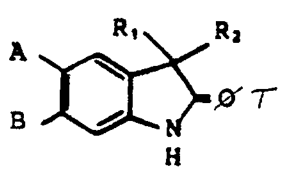 — (XXX)

in der

$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet

$R_2$ ein Wasserstoffatom, eine $C_1$-$C_6$-Alkyl-, $C_2$-$C_6$-Alkenyl-, oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino-oder Hydrazinogruppe substituierte Carbonylgruppe bedeutet oder mit $R_1$ zusammen eine $C_3$-$C_8$-Cycloalkylengruppe darstellt oder $R_1$ und $R_2$ zusammen eine $C_2$-$C_6$-Alkyliden- oder $C_3$-$C_8$-Cycloalkylidengruppe bilden,

A ein Wasserstoffatom, $NH_2$, $NO_2$ oder Py-X-CO-NH- und

B ein Wasserstoffatom, $NH_2$, $NO_2$ oder Py-X-CO-NH- bedeuten, wobei
X einen Valenzstrich, eine $C_1$-$C_4$-Alkylengruppe oder die Vinylengruppe bedeutet und
Py einen 2-, 3- oder 4-Pyridylrest darstellt, der gegebenenfalls am Ringheteroatom ein Sauerstoffatom trägt und/oder durch eine oder mehrere $C_1$-$C_6$-Alkyl-, $C_1$-$C_6$-Alkoxy-, Hydroxy-, Cyano- oder Nitrogruppen sowie durch Halogen substituiert sein kann, und
T gleich Sauerstoff oder Schwefel bedeutet,
mit der Maßgabe, daß A und B nur gleichzeitig $NH_2$ bedeuten dürfen, und
mit der Maßgabe, daß wenn $R_1$ Wasserstoff und $R_2$ Wasserstoff oder eine Alkylgruppe darstellt, oder wenn $R_1$ Wasserstoff, eine Alkyl- oder Alkenylgruppe und $R_2$ eine durch eine Hydroxy- oder Alkoxygruppe substituierte Carbonylgruppe darstellt, A nicht Wasserstoff oder eine Aminogruppe sein kann, wenn B Wasserstoff oder eine Aminogruppe ist.

**9.** Verfahren zur Herstellung von Verbindungen der Formel XXX in der

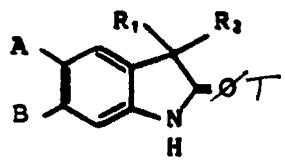
(XXX)

$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet
$R_2$ ein Wasserstoffatom, eine $C_1$-$C_6$-Alkyl-, $C_2$-$C_6$-Alkenyl-, oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe bedeutet oder mit $R_1$ zusammen eine $C_3$-$C_8$-Cycloalkylengruppe darstellt oder $R_1$ und $R_2$ zusammen eine $C_2$-$C_6$-Alkyliden- oder $C_3$-$C_8$-Cycloalkylidengruppe bilden,
A ein Wasserstoffatom, $NH_2$, $NO_2$ oder Py-X-CO-NH- und
B ein Wasserstoffatom, $NH_2$, $NO_2$ oder Py-X-CO-NH- bedeuten, wobei
X einen Valenzstrich, eine $C_1$-$C_4$-Alkylengruppe oder die Vinylengruppe bedeutet und
Py einen 2-, 3- oder 4-Pyridylrest darstellt, der gegebenenfalls am Ringheteroatom ein Sauerstoffatom trägt und/oder durch eine oder mehrere $C_1$-$C_6$-Alkyl-, $C_1$-$C_6$-Alkoxy-, Hydroxy-, Cyano- oder Nitrogruppen sowie durch Halogen substituiert sein kann, und
T gleich Sauerstoff oder Schwefel bedeutet,
mit der Maßgabe, daß A und B nur gleichzeitig $NH_2$ bedeuten dürfen, und
mit der Maßgabe, daß wenn $R_1$ Wasserstoff und $R_2$ Wasserstoff oder eine Alkylgruppe darstellt, oder wenn $R_1$ Wasserstoff, eine Alkyl- oder Alkenylgruppe und $R_2$ eine durch eine Hydroxy- oder Alkoxygruppe substituierte Carbonylgruppe darstellt, A nicht Wasserstoff oder eine Aminogruppe sein kann, wenn B Wasserstoff oder eine Aminogruppe ist,
dadurch gekennzeichnet, daß man in an sich bekannter Weise entweder

a) eine Verbindung der Formel IX

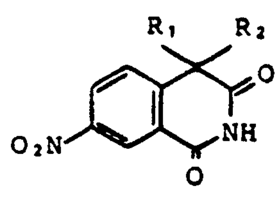
(IX),

in der $R_1$ und $R_2$ die oben genannten Bedeutungen haben, durch Hoffmann-Abbau in Verbindungen der allgemeinen Formel X

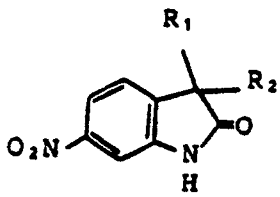

(X),

umwandelt, und gegebenenfalls die Nitrogruppe zur Aminogruppe reduziert, oder

b) entsprechend substituierte Derivate der 2,4-Diaminophenylessigsäure durch Ringschluß in Verbindungen der allgemeinen Formel XI,

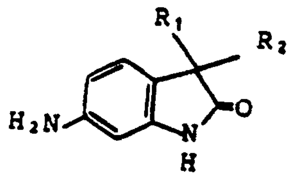

(XI),

in der $R_1$ und $R_2$ die oben genannten Bedeutungen haben, umwandelt, oder

c) eine Verbindung der allgemeinen Formel XI

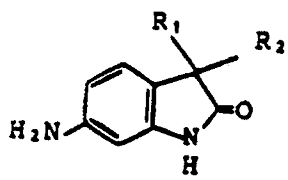

(XI),

in der $R_1$ und $R_2$ die oben genannten Bedeutungen haben, acetyliert, in 5-Stellung nitriert, die Schutzgruppe abspaltet und die Nitrogruppe zu einer Verbindung der allgemeinen Formel XII

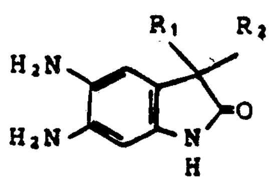

(XII),

reduziert, oder

d) eine Verbindung der allgemeinen Formel XIII

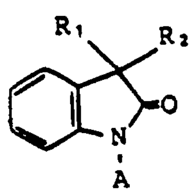

(XIII),

in der $R_1$ und $R_2$ Wasserstoff bedeuten und A eine Schutzgruppe darstellt, mit einer Verbindung der allgemeinen Formel III

$$R_1-Z \qquad (III),$$

oder IV

$$Z-R_3-Z \qquad (IV),$$

in denen $R_1$ die oben angegebene Bedeutung hat, $R_3$ eine $C_2$-$C_6$-Alkylengruppe ist und Z eine abspaltbare Gruppe darstellt, zu Verbindungen der allgemeinen Formel XIII, in der $R_1$ und $R_2$ die oben angegebene Bedeutung haben, jedoch nicht Wasserstoff sein können, alkyliert, die Schutzgruppe A abspaltet, und die so erhaltenen Verbindungen, in denen A Wasserstoff bedeutet, anschließend nitriert, die Nitrogruppe zur Aminogruppe reduziert, die Aminogruppe acetyliert, anschließend in 6-Stellung nitriert, die Schutzgruppe an der Aminofunktion abspaltet, und die Nitrogruppe durch Reduktion in eine Verbindung der Formel XII

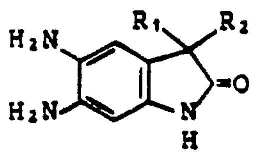

(XII),

in der $R_1$ und $R_2$ die oben genannte Bedeutung haben, überführt, oder

e) eine Verbindung der Formel XII

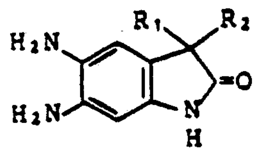

(XII),

in der $R_1$ und $R_2$ die oben angegebenen Bedeutungen haben, mit einer Verbindung der Formel XIV

Py-X-CO-Y (XIV),

in der Py und X die angegebene Bedeutung haben und Y entweder Wasserstoff oder ein leicht abspaltbarer Rest darstellt, umsetzt oder

f) eine Verbindung der Formel XV oder XVI

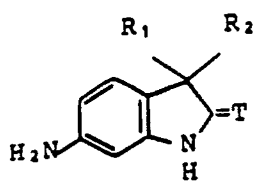

(XV)

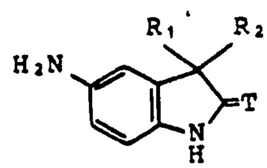

(XVI),

in denen $R_1$, $R_2$ und T die oben genannten Bedeutungen besitzen, mit einer Verbindung der allgemeinen Formel XIV

Py-X-CO-Y (XIV),

in der Py und X die oben genannten Bedeutungen haben und Y einen leicht abspaltbaren Rest darstellt, zu Verbindungen der Formel XVII bzw. XVIII

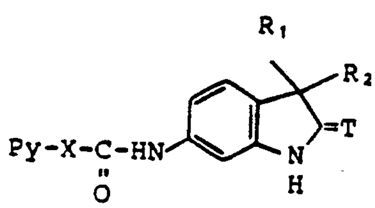

(XVII),

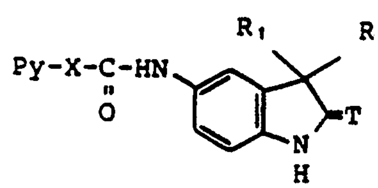

(XVIII),

umsetzt und diese gegebenenfalls in 5- bzw. 6-Stellung nitriert und gegebenenfalls anschließend die Nitrogruppe durch Reduktion in eine Aminogruppe umwandelt, oder
g) eine Verbindung der allgemeinen Formel XXIII

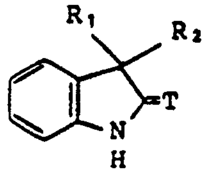
(XXIII),

in der $R_1$, $R_2$ und T die oben angegebenen Bedeutungen haben, in 5-Stellung nitriert und gegebenenfalls anschließend die Nitrogruppe zur Aminofunktion reduziert, oder
h) eine Verbindung der allgemeinen Formel XV bzw. XVI

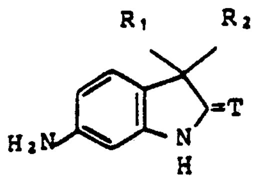
(XV)

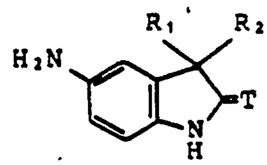
(XVI),

in der $R_1$, $R_2$ und T die oben angegebene Bedeutung haben, die 6- bzw. 5-ständige Aminogruppe acetyliert, in 5- bzw. 6-Stellung nitriert, die Schutzgruppen abspaltet, und die so erhaltene Verbindung mit einer Verbindung der allgemeinen Formel XIV

Py-X-CO-Y (XIV),

in der Py und X die oben angegebene Bedeutung haben und Y entweder Wasserstoff oder eine leicht abspaltbare Gruppe darstellt, zu Verbindungen der allgemeinen Formel XIX bzw. XX

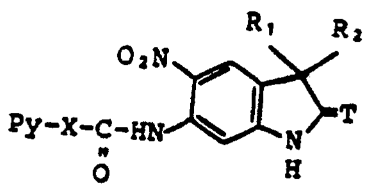
(XIX),

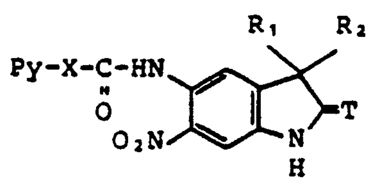
(XX),

umsetzt.

## Claims

1. Compounds of the formula I

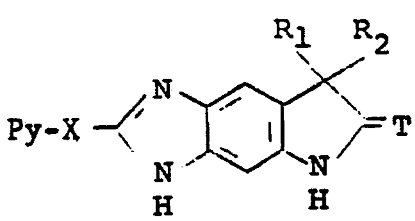
(I)

in which $R_1$ signifies a hydrogen atom, a $C_1$-$C_6$-alkyl, $C_2$-$C_6$-alkenyl or a $C_3$-$C_7$-cycloalkyl group, $R_2$

signifies a hydrogen atom, a $C_1$-$C_6$-alkyl, $C_2$-$C_6$-alkenyl or cyano group, a carbonyl group substituted by a hydroxyl, alkyl, alkoxy, amino, alkylamino, dialkylamino or hydrazino group, whereby the above-mentioned alkyl moieties contain 1 - 6 C-atoms, or together with $R_1$ represents a $c_3$-$C_8$-cycloalkylene group or $R_1$ and $R_2$ together form a $C_2$-$C_6$-alkylidene or $C_3$-$C_8$-cycloalkylidene group, X signifies a valency bond, a $C_1$-$C_4$-alkylene group or the vinylene group, T signifies oxygen or sulphur, Py represents a 2-, 3- or 4-pyridyl radical which optionally carries an oxygen atom on the ring hetero atom and/or can be substituted by one or more $C_1$-$C_6$-alkyl, $C_1$-$C_6$-alkoxy, hydroxyl, cyano or nitro groups, as well as by halogen; their tautomers and their physiologically acceptable salts of inorganic and organic acids.

2. Compounds according to claim 1, in which $R_1$ signifies hydrogen, methyl, ethyl, 2-propyl, 2-methylpropyl or cyclopentyl, $R_2$ hydrogen, methyl, ethyl, ethoxycarbonyl or hydrazinocarbonyl, $R_1$ and $R_2$, together with the C-atom to which they are attached, form a cyclopentane ring or together form an isopropylidene radical, X a valency bond, a methylene, ethylene or vinylene group, T oxygen or sulphur and Py a pyridylene-N-oxide radical or a pyridyl radical, which can be substituted one or more times by hydroxyl, methoxy, methyl or halogen, their tautomers and their physiologically acceptable salts.

3. Compounds according to claim 1 or 2, in which T represents oxygen, X a valency bond, $R_1$ hydrogen or methyl, $R_2$ methyl, ethyl or ethoxycarbonyl, and Py a pyridine-N-oxide radical or a pyridyl radical which can be substituted by hydroxyl or methyl, their tautomers and their physiologically acceptable salts.

4. Compounds according to claim 1 selected from 7,7-dimethyl-2-(4-pyridyl)-6,7-dihydro-3H,5H-pyrrolo-[2,3-f]benzimidazol-6-one; 7,7-dimethyl-2-(4-N-oxy-pyridyl)-6,7-dihydro-3H,5H-pyrrolo[2,3-f]-benzimidazol-6-one; 7-ethoxycarbonyl-7-methyl-2-(4-pyridyl)-6,7-dihydro-3H,5H-pyrrolo[2,3-f]-benzimidazol-6-one; 7,7-dimethyl-2-(4-(2-methylpyridyl)-6,7-dihydro-3H,5H-pyrrolo[2,3-f]benzimidazol-6-one.

5. Process for the preparation of compounds of the formula I

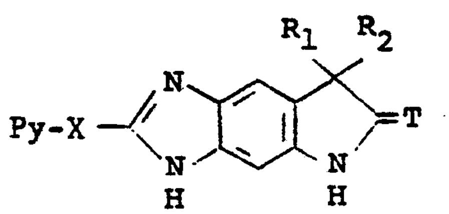

(I)

in which $R_1$ signifies a hydrogen atom, a $C_1$-$C_6$-alkyl, $C_2$-$C_6$-alkenyl or a $C_3$-$C_7$-cycloalkyl radical, $R_2$ signifies a hydrogen atom, a $C_1$-$C_6$-alkyl, $C_2$-$C_6$-alkenyl or cyano group, a carbonyl group substituted by a hydroxyl, alkyl, alkoxy, amino, alkylamino, dialkylamino or hydrazino group, whereby the above-mentioned alkyl moieties contain 1 - 6 C-atoms, or together with $R_1$ represents a $C_3$-$C_8$-cycloalkylene group or $R_1$ and $R_2$ together form a $C_2$-$C_6$-alkylidene or $C_3$-$C_8$-cycloalkylidene group, X signifies a valency bond, a $C_1$-$C_4$-alkylene radical or the vinylene group, T signifies oxygen or sulphur, Py is a 2-, 3- or 4-pyridyl radical which optionally carries an oxygen atom on the ring hetero atom and/or can be substituted by one or more $C_1$-$C_6$-alkyl, $C_1$-$C_6$-alkoxy, hydroxyl, cyano or nitro groups, as well as by halogen; of their tautomers and of their physiologically acceptable salts of inorganic and organic acids, characterised in that, in known manner, one

a) reacts a compound of the formula XII

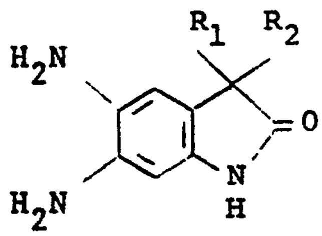

(XII)

in which $R_1$ and $R_2$ possess the above-given meaning, with a compound of the formula XIV

Py-X-CO-Y (XIV),

in which Py and X possess the given meaning and Y represents either hydrogen or a residue which can easily be split off; and cyclises the compound obtained in an acid medium, or

b) reduces a compound of the formula XIX

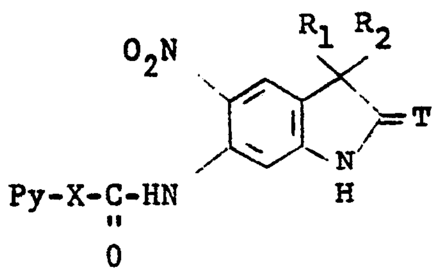

(XIX)

or of the formula XX

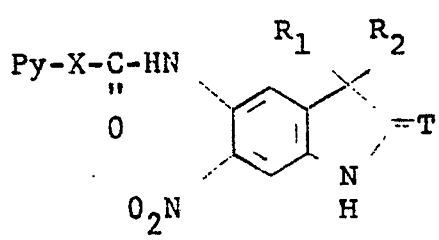

(XX)

in which $R_1$, $R_2$, T, Py and X possess the above-given meaning, and subsequently cyclised; or

c) acylates a compound of the formula XXVI

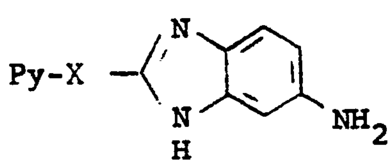

(XXVI)

with a reactive carboxylic acid derivative of the formula XXVII

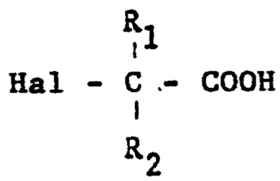

(XXVII)

whereby $R_1$ $R_2$, Py, X and Hal have the given meaning, and cyclises; or
d) reacts a compound of the formula XXVIII

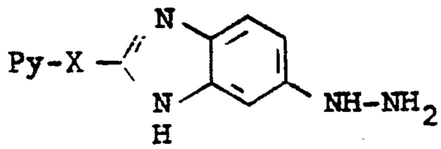

(XXVIII)

with a reactive carboxylic acid derivative of the formula XXIX

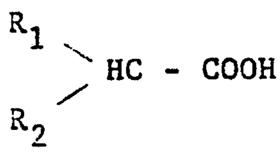

(XXIX)

to corresponding hydrazides and then cyclises these under alkaline conditions, whereby $R_1$, $R_2$, Py and X have the given meanings and subsequently, if desired, converts compounds obtained of the formula I into other compounds of the formula I, as well as, if desired, converts the compounds into physiologically acceptable salts with an inorganic or organic acid.

6. Medicaments containing a compound according to claim 1, 2 or 3, besides conventional carrier and adjuvant materials,

7. Use of compounds according to claim 1, 2 or 3 for the preparation of medicaments for the prophylaxis or treatment of heart or circulatory diseases.

8. Compounds of the formula XXX

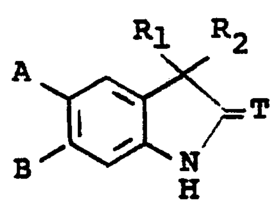

(XXX)

in which $R_1$ signifies a hydrogen atom, a $C_1$-$C_6$-alkyl, $C_2$-$C_6$-alkenyl or a $C_3$-$C_7$-cycloalkyl group, $R_2$ signifies a hydrogen atom, a $C_1$-$C_6$-alkyl, $C_2$-$C_6$-alkenyl or cyano group, a carbonyl group substituted by a hydroxyl, $C_1$-$C_6$-alkyl, $C_1$-$C_6$-alkoxy, amino, $C_1$-$C_6$-alkylamino, di-$C_1$-$C_6$-alkylamino or hydrazino group or together with $R_1$ represents a $C_3$-$C_8$-cycloalkylene group or $R_1$ and $R_2$ together form a $C_2$-$C_6$-alkylidene or $C_3$-$C_8$-cycloalkylidene group, A a hydrogen atom, $NH_2$, $NO_2$ or Py-X-NH- and B a hydrogen atom, $NH_2$, $NO_2$ or Py-X-NH-, whereby X signifies a valency bond, a $C_1$-$C_4$-alkylene group or the vinylene group and Py represents a 2-, 3- or 4-pyridyl radical which optionally carries an oxygen atom on the ring hetero atom and/or can be substituted by one or more $C_1$-$C_6$-alkyl, $C_1$-$C_6$-alkoxy, hydroxyl, cyano or nitro groups, as well as by halogen and T signifies oxygen or sulphur, with the proviso that A and B can only simultaneously signify $NH_2$ and with the proviso that when $R_1$ represents

hydrogen and $R_2$ hydrogen or an alkyl group or when $R_1$ represents hydrogen, an alkyl or alkenyl group and $R_2$ a carbonyl group substituted by a hydroxyl or alkoxy group, A cannot be hydrogen or an amino group when B is hydrogen or an amino group.

Claims for the following Contracting State: AT

1. Compounds of the formula I

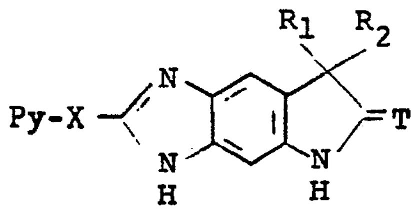

(I)

in which $R_1$ signifies a hydrogen atom, a $C_1$-$C_6$-alkyl, $C_2$-$C_6$-alkenyl or a $C_3$-$C_7$-cycloalkyl group, $R_2$ signifies a hydrogen atom, a $C_1$-$C_6$-alkyl, $C_2$-$C_6$-alkenyl or cyano group, a carbonyl group substituted by a hydroxyl, alkyl, alkoxy, amino, alkylamino, dialkylamino or hydrazino group, whereby the above-mentioned alkyl moieties contain 1 - 6 C-atoms, or together with $R_1$ represents a $C_3$-$C_8$-cycloalkylene group or $R_1$ and $R_2$ together form a $C_2$-$C_6$-alkylidene or $C_3$-$C_8$-cycloalkylidene group, X signifies a valency bond, a $C_1$-$C_4$-alkylene group or the vinylene group, T signifies oxygen or sulphur, Py represents a 2-, 3- or 4-pyridyl radical which optionally carries an oxygen atom on the ring hetero atom and/or can be substituted by one or more $C_1$-$C_6$-alkyl, $C_1$-$C_6$-alkoxy, hydroxyl, cyano or nitro groups, as well as by halogen; their tautomers and their physiologically acceptable salts of inorganic and organic acids.

2. Compounds according to claim 1, in which $R_1$ signifies hydrogen, methyl, ethyl, 2-propyl, 2-methylpropyl or cyclopentyl, $R_2$ hydrogen, methyl, ethyl, ethoxycarbonyl or hydrazinocarbonyl, $R_1$ and $R_2$, together with the C-atom to which they are attached, form a cyclopentane ring or together form an isopropylidene radical, X a valency bond, a methylene, ethylene or vinylene group, T oxygen or sulphur and Py a pyridylene-N-oxide radical or a pyridyl radical, which can be substituted one or more times by hydroxyl, methoxy, methyl or halogen, their tautomers and their physiologically acceptable salts.

3. Compounds according to claim 1 or 2, in which T represents oxygen, X a valency bond, $R_1$ hydrogen or methyl, $R_2$ methyl, ethyl or ethoxycarbonyl, and Py a pyridine-N-oxide radical or a pyridyl radical which can be substituted by hydroxyl or methyl, their tautomers and their physiologically acceptable salts.

4. Compounds according to claim 1 selected from 7,7-dimethyl-2-(4-pyridyl)-6,7-dihydro-3H,5H-pyrrolo-[2,3-f]benzimidazol-6-one; 7,7-dimethyl-2-(4-N-oxy-pyridyl)-6,7-dihydro-3H,5H-pyrrolo[2,3-f]-benzimidazol-6-one; 7-ethoxycarbonyl-7-methyl-2-(4-pyridyl)-6,7-dihydro-3H,5H-pyrrolo[2,3-f]-benzimidazol-6-one; 7,7-dimethyl-2-(4-(2-methylpyridyl)-6,7-dihydro-3H,5H-pyrrolo[2,3-f]benzimidazol-6-one.

5. Process for the preparation of compounds of the formula I

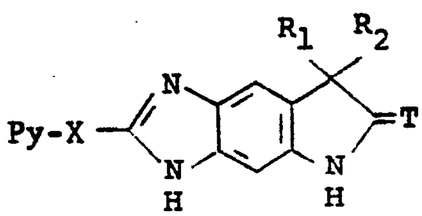

(I)

in which $R_1$ signifies a hydrogen atom, a $C_1$-$C_6$-alkyl, $C_2$-$C_6$-alkenyl or a $C_3$-$C_7$-cycloalkyl radical, $R_2$

signifies a hydrogen atom, a $C_1$-$C_6$-alkyl, $C_2$-$C_6$-alkenyl or cyano group, a carbonyl group substituted by a hydroxyl, alkyl, alkoxy, amino, alkylamino, dialkylamino or hydrazino group, whereby the above-mentioned alkyl moieties contain 1 - 6 C-atoms, or together with $R_1$ represents a $C_3$-$C_8$-cycloalkylene group or $R_1$ and $R_2$ together form a $C_2$-$C_6$-alkylidene or $C_3$-$C_8$-cycloalkylidene group, X signifies a valency bond, a $C_1$-$C_4$-alkylene radical or the vinylene group, T signifies oxygen or sulphur, Py is a 2-, 3- or 4-pyridyl radical which optionally carries an oxygen atom on the ring hetero atom and/or can be substituted by one or more $C_1$-$C_6$-alkyl, $C_1$-$C_6$-alkoxy, hydroxyl, cyano or nitro groups, as well as by halogen; of their tautomers and of their physiologically acceptable salts of inorganic and organic acids, characterised in that, in known manner, one

a) reacts a compound of the formula XII

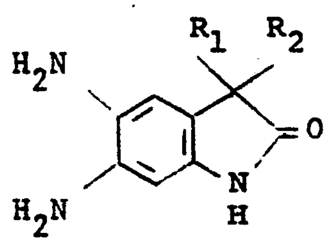

(XII)

in which $R_1$ and $R_2$ possess the above-given meaning, with a compound of the formula XIV

Py-X-CO-Y (XIV),

in which Py and X possess the given meaning and Y represents either hydrogen or a residue which can easily be split off; and cyclises the compound obtained in an acid medium, or

b) reduces a compound of the formula XIX

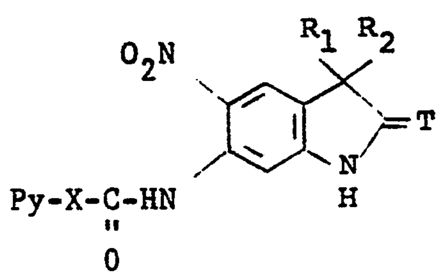

(XIX)

or of the formula XX

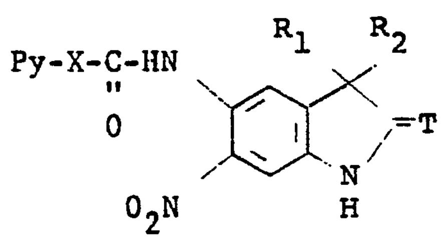

(XX)

in which $R_1$, $R_2$, T, Py and X possess the above-given meaning, and subsequently cyclised; or

c) acylates a compound of the formula XXVI

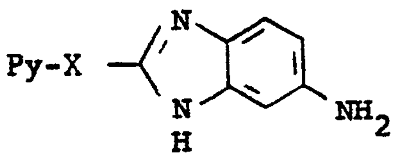

(XXVI)

with a reactive carboxylic acid derivative of the formula XXVII

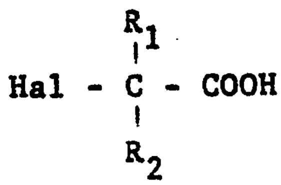

(XXVII)

whereby $R_1$, $R_2$, Py, X and Hal have the given meaning, and cyclises; or
d) reacts a compound of the formula XXVIII

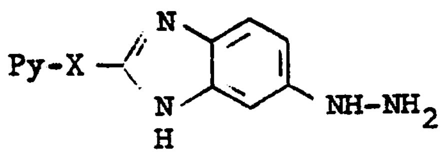

(XXVIII)

with a reactive carboxylic acid derivative of the formula XXIX

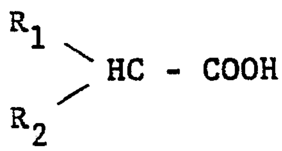

(XXIX)

to corresponding hydrazides and then cyclises these under alkaline conditions, whereby $R_1$, $R_2$, Py and X have the given meanings and subsequently, if desired, converts compounds obtained of the formula I into other compounds of the formula I, as well as, if desired, converts the compounds into physiologically acceptable salts with an inorganic or organic acid.

6. Medicaments containing a compound aocording to claim 1, 2 or 3, besides conventional carrier and adjuvant materials.

7. Use of compounds according to claim 1, 2 or 3 for the preparation of medicaments for the prophylaxis or treatment of heart or circulatory diseases.

8. Compounds of the formula XXX

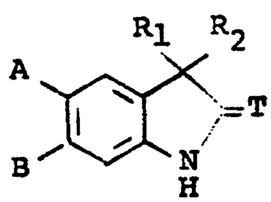

(XXX)

in which $R_1$ signifies a hydrogen atom, a $C_1$-$C_6$-alkyl, $C_2$-$C_6$-alkenyl or a $C_3$-$C_7$-cycloalkyl group, $R_2$ signifies a hydrogen atom, a $C_1$-$C_6$-alkyl, $C_2$-$C_6$-alkenyl or cyano group, a carbonyl group substituted by a hydroxyl, $C_1$-$C_6$-alkyl, $C_1$-$C_6$-alkoxy, amino, $C_1$-$C_6$-alkylamino, di-$C_1$-$C_6$-alkylamino or hydrazino

group or together with $R_1$ represents a $C_3$-$C_8$-cycloalkylene group or $R_1$ and $R_2$ together form a $C_2$-$C_6$-alkylidene or $C_3$-$C_8$-cycloalkylidene group, A a hydrogen atom, $NH_2$, $NO_2$ or Py-X-NH- and B a hydrogen atom, $NH_2$, $NO_2$ or Py-X-NH-, whereby X signifies a valency bond, a $C_1$-$C_4$-alkylene group or the vinylene group and Py represents a 2-, 3- or 4-pyridyl radical which optionally carries an oxygen atom on the ring hetero atom and/or can be substituted by one or more $C_1$-$C_6$-alkyl, $C_1$-$C_6$-alkoxy, hydroxyl, cyano or nitro groups, as well as by halogen and T signifies oxygen or sulphur, with the proviso that A and B can only simultaneously signify $NH_2$ and with the proviso that when $R_1$ represents hydrogen and $R_2$ hydrogen or an alkyl group or when $R_1$ represents hydrogen, an alkyl or alkenyl group and $R_2$ a carbonyl group substituted by a hydroxyl or alkoxy group, A cannot be hydrogen or an amino group when B is hydrogen or an amino group.

**9.** Process for the preparation of compounds of the formula XXX

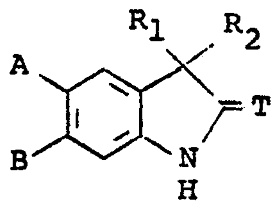

(XXX)

in which $R_1$ signifies a hydrogen atom, a $C_1$-$C_6$-alkyl, $C_2$-$C_6$-alkenyl or a $C_3$-$C_7$-cycloalkyl group, $R_2$ signifies a hydrogen atom, a $C_1$-$C_6$-alkyl, $C_2$-$C_6$-alkenyl or cyano group, a carbonyl group substituted by a hydroxyl, $C_1$-$C_6$-alkyl, $C_1$-$C_6$-alkoxy, amino, $C_1$-$C_6$-alkylamino, di-$C_1$-$C_6$-alkylamino or hydrazino group or together with $R_1$ represents a $C_3$-$C_8$-cycloalkyl group or $R_1$ and $R_2$ together form a $C_2$-$C_6$-alkylidene or $C_3$-$C_8$-cycloalkylidene group, A signifies a hydrogen atom, $NH_2$, $NO_2$ or Py-X-CO-NH- and B a hydrogen atom, $NH_2$, $NO_2$ or Py-X-Co-NH-, whereby X signifies a valency bond, a $C_1$-$C_4$-alkylene group or the vinylene group and Py represents a 2-, 3- or 4-pyridyl radical which optionally carries an oxygen atom on the ring heteroatom and/or can be substituted by one or more $C_1$-$C_6$-alkyl, $C_1$-$C_6$-alkoxy, hydroxyl, cyano or nitro groups, as well as by halogen, and T signifies oxygen or sulphur, with the proviso that A and B can only simultaneously signify $NH_2$ and with the proviso that when $R_1$ represents hydrogen and $R_2$ hydrogen or an alkyl group or when $R_1$ represents hydrogen, an alkyl or alkenyl group and $R_2$ a carbonyl group substituted by a hydroxyl or alkoxy group, A cannot be hydrogen or an amino group when B is hydrogen or an amino group, characterised in that, in per se known manner, one either

a) converts a compound of the formula IX

-8-

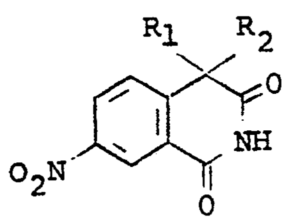

(IX)

in which $R_1$ and $R_2$ have the above-mentioned meanings, by Hoffmann decomposition into compounds of the general formula X

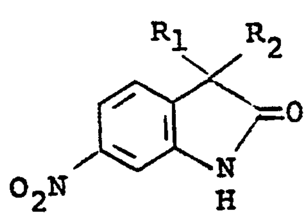

(X)

and optionally reduces a nitro group to the amino group, or
b) converts correspondingly substituted derivatives of 2,4-diaminophenylacetic acid by ring closure into compounds of the general formula XI

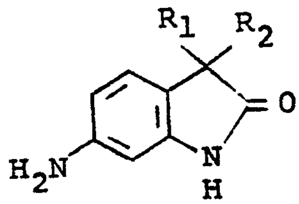

(XI)

in which $R_1$ and $R_2$ have the above-mentioned meanings, or
c) acetylates a compound of the general formula XI

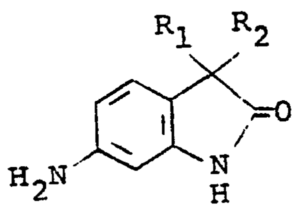

(XI)

in which $R_1$ and $R_2$ have the above-mentioned meanings, nitrates in the 5-position, splits off the protective group and reduces the nitro group to a compound of the general formula XII, or
d) alkylates a compound of the general formula XIII

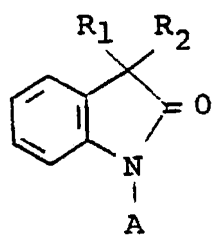

(XIII)

in which $R_1$ and $R_2$ signify hydrogen and A represents a protective group, with a compound of the general formula III

$R_1$-Z (III)

or IV

Z-$R_3$-Z (IV)

in which $R_1$ has the above-given meaning, $R_3$ is a $C_2$-$C_6$-alkylene group and Z is a group which can be split off, to compounds of the general formula XIII, in which $R_1$ and $R_2$ have the above-given meaning but cannot be hydrogen, splits off the protective group A and subsequently nitrates the so obtained compounds in which A signifies hydrogen, reduces the nitro group to the amino group, acetylates the amino group, subsequently nitrates in the 6-position, splits off the protective group on the amino function and converts the nitro group by reduction into a compound of the formula XII

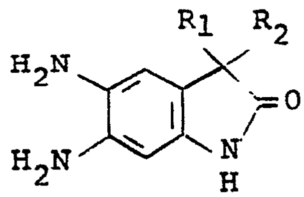

(XII)

in which $R_1$ and $R_2$ have the above-mentioned meaning, or

e) reacts a compound of the formula XII

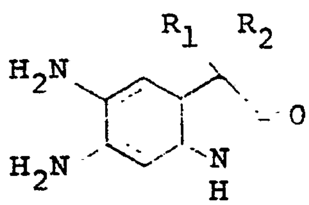

(XII)

in which $R_1$ and $R_2$ have the above-given meanings, with a compound of the formula XIV

Py-X-CO-Y (XIV),

in which Py and X have the given meaning and Y represents either hydrogen or a residue which is easily split off, or

f) reacts a compound of the formula XV or XVI

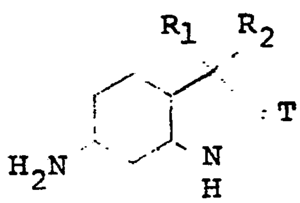

(XV)

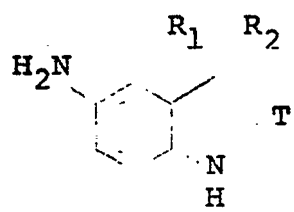

(XVI)

in which $R_1$, $R_2$ and T possess the mentioned meanings, with a compound of the general formula XIV

Py-Y-CO-Y (XIV),

in which Py and X have the above-mentioned meanings and Y represents a residue which is easily split off, to compounds of the formula XVII or XVIII

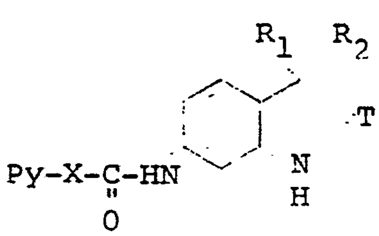

(XVII)

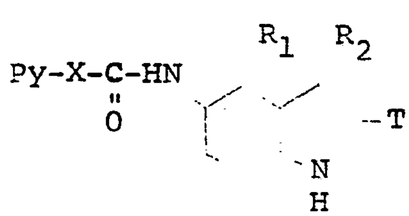

(XVIII)

and optionally nitrates these in the 5- or 6-position and optionally subsequently converts the nitro group by reduction into an amino group, or

g) nitrates a compound of the general formula XXIII

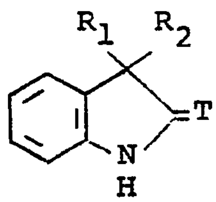

(XXIII),

in which $R_1$, $R_2$ and T have the above-given meaning, in the 5-position and optionally subsequently reduces the nitro group to the amino function, or

g) in a compound of the general formula XV or XVI

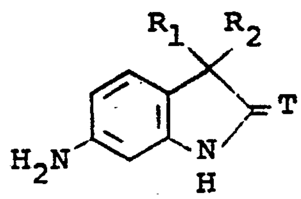

(XV)

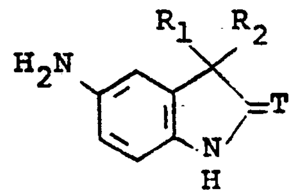

(XVI)

in which $R_1$, $R_2$ and T have the above-given meaning, acetylates the 6- or 5-positioned amino group, nitrates in the 5- or 6-position, splits off the protective groups and reacts the so obtained compound with a compound of the general formula XIV

Py-X-CO-Y (XIV)

in which Py and X have the above-mentioned meaning and Y represents either hydrogen or a group which is easily split off, to compounds of the general formula XIX or XX

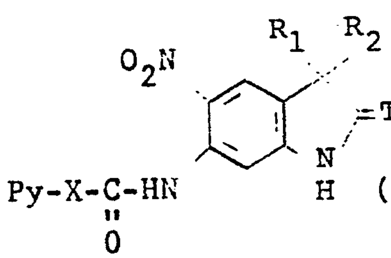

(XIX)

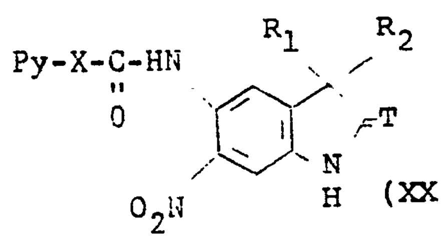

(XX)

## Revendications

**1.** Composés de Formule I

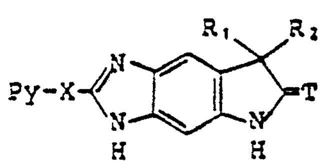

(I),

dans lesquels

$R_1$ représente un atome d'hydrogène, un groupe alkyle en $C_1$-$C_6$, un groupe alcényle en $C_2$-$C_6$ ou un groupe cycloalkyle en $C_3$-$C_7$,

$R_2$ représente un atome d'hydrogène, un groupe alkyle en $C_1$-$C_6$, un groupe alcényle en $C_2$-$C_6$ ou un groupe cyano, un groupe carbonyle substitué par un groupe hydroxy, alkyle, alcoxy, amino, alkylamino, dialkylamino ou hydrazino, les groupes alkyle mentionnés contenant 1 à 6 atomes de carbone, ou forme , en commun avec $R_1$, un groupe cycloalkylène en $C_3$-$C_8$, ou bien $R_1$ et $R_2$ forment en commun un groupe alkylidène en $C_2$-$C_6$ ou un groupe cycloalkylidène en $C_3$-$C_8$,

X représente une valence libre, un groupe alkylène en $C_1$-$C_4$ ou le groupe vinylène,

T représente un atome d'oxygène ou un atome de soufre,

Py représente un reste 2-, 3-, ou 4-pyridyle, qui porte éventuellement un atome d'oxygène sur l'hétéroatome du cycle et/ou peut être substitué par un ou plusieurs groupes alkyle en $C_1$-$C_6$, alcoxy en $C_1$-$C_6$, hydroxy, cyano ou nitro, ainsi que par un atome d'halogène, leurs tautomères et leurs sels d'acides minéraux et organiques physiologiquement acceptables.

2. Composés selon la Revendication 1, dans lesquels $R_1$ représente un atome d'hydrogène, un groupe méthyle, éthyle, propyle, 2-méthyl-propyle ou cyclopentyle, $R_2$ représente un atome d'hydrogène, un groupe méthyle, éthyle, éthoxycarbonyle ou hydrazinocarbonyle, $R_1$ et $R_2$ forment, en commun avec l'atome de carbone auquel ils sont liés, un cycle cyclopentane, ou en commun un reste isopropylidène, X représente une valence libre, un groupe méthylène, éthylène ou vinyle, T représente l'oxygène ou le soufre et Py représente un reste pyridylène-N-oxyde ou un reste pyridyle, qui peut être substitué une ou plusieurs fois par un groupe hydroxyle, méthoxy, méthyle ou un atome d'halogène, leurs tautomères et leurs sels physiologiquement acceptables.

3. Composés selon les Revendications 1 ou 2, dans lesquels T représente l'oxygène, X représente une valence libre, $R_1$ représente l'hydrogène ou un groupe méthyle, $R_2$ représente un groupe méthyle, éthyle, ou éthoxycarbonyle et Py représente un reste pyridine-N-oxyde ou un reste pyridyle, qui peut être substitué par un groupe hydroxy ou méthyle, leurs tautomères et leurs sels physiologiquement acceptables.

4. Composés selon la Revendication 1, choisis parmi les :
7,7-diméthyl-2-(4-pyridyl)-6,7-dihydro-3H,5H-pyrrolo[2,3-f]-benzimidazol-6-one
7,7-diméthyl-2-(4-N-oxy-pyridyl)-6,7-dihydro-3H,5H-pyrrolo-[2,3-f]benzimidazol-6-one
7-éthoxycarbonyl-7-méthyl-2-(4-pyridyl)-6,7-dihydro-3H,5H-pyrrolo[2,3-f]benzimidazol-6-one
7,7-diméthyl-2-(4-(2-méthyl-pyridyl))-6,7-dihydro-3H,5H-pyrrolo[2,3-f]benzimidazol-6-one

5. Procédé pour la préparation de composés de Formule I

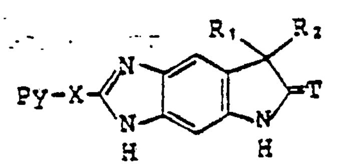

(I),

dans lesquels

$R_1$ représente un atome d'hydrogène, un groupe alkyle en $C_1$-$C_6$, un groupe alcényle en $C_2$-$C_6$ ou un groupe cycloalkyle en $C_3$-$C_7$,

$R_2$ représente un atome d'hydrogène, un groupe alkyle en $C_1$-$C_6$, un groupe alcényle en $C_2$-$C_6$ ou un groupe cyano, un groupe carbonyle substitué par un groupe hydroxy, alkyle, alcoxy, amino, alkylamino, dialkylamino ou hydrazino, les groupes alkyle mentionnés contenant 1 à 6 atomes de carbone, ou forme , en commun avec $R_1$, un groupe cycloalkylène en $C_3$-$C_8$, ou bien $R_1$ et $R_2$ forment en commun un groupe alkylidène en $C_2$-$C_6$ ou un groupe cycloalkylidène en $C_3$-$C_8$,

X représente une valence libre, un groupe alkylène en $C_1$-$C_4$ ou le groupe vinylène,

T représente un atome d'oxygène ou un atome de soufre,

Py représente un reste 2-, 3-, ou 4-pyridyle, qui porte éventuellement un atome d'oxygène sur l'hétéroatome du cycle et/ou peut être substitué par un ou plusieurs groupes alkyle en $C_1$-$C_6$, alcoxy en $C_1$-$C_6$, hydroxy, cyano ou nitro, ainsi que par un atome d'halogène,

leurs tautomères et leurs sels d'acides minéraux et organiques physiologiquement acceptables, caractérisés en ce que, d'une manière connue en soi, on fait réagir

a) un composé de Formule XII

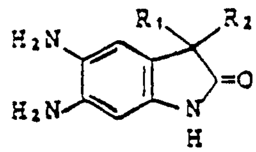
(XII),

dans laquelle $R_1$ et $R_2$ ont la signification mentionnée plus haut, avec un composé de Formule XIV où Py et X ont la signification mentionnée plus haut et Y représente soit un atome d'hydrogène soit un reste facilement séparable, et on cyclise le composé obtenu en milieu acide, ou

b) on réduit un composé de Formule XIX

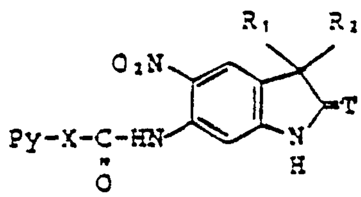
(XIX),

ou de Formule XX

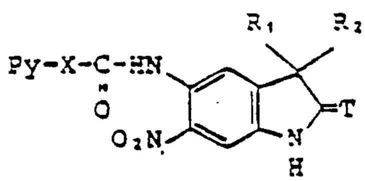
(XX),

où $R_1$, $R_2$, T, Py et X ont la signification mentionnée plus haut, et ensuite on cyclise ou

c) on acyle et cyclise un composé de Formule XXVI

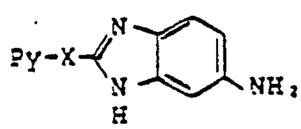
(XXVI)

avec un dérivé d'acide carboxylique réactif de Formule XXVII

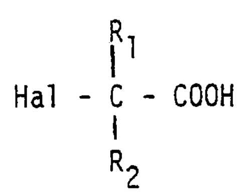
(XXVII)

où $R_1$, $R_2$, Py, X et Hal ont la signification mentionnée plus haut, ou on fait réagir

d) un composé de Formule XXVIII

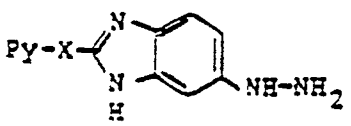 (XXVIII)

avec un dérivé d'acide carboxylique réactif de Formule XXIX

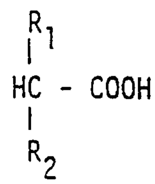 (XXIX)

en hydrazide correspondant, et on cyclise ensuite celui-ci dans des conditions alcalines, $R_1$, $R_2$, Py et X ayant la signification mentionnée plus haut, et ensuite, on transforme éventuellement les composés de Formule I obtenus en d'autres composés de Formule I, ainsi qu'éventuellement on transforme les composés avec des acides minéraux ou organiques en sels physiologiquement compatibles.

**6.** Médicament contenant un composé selon les Revendications 1, 2 ou 3, outre les supports et additifs habituels.

**7.** Utilisation des composés selon la Revendication 1, 2 ou 3, pour la preparation des médicaments pour la prophyllaxie ou le traitement des maladies cardiaques et circulatoires.

**8.** Composés de Formule XXX

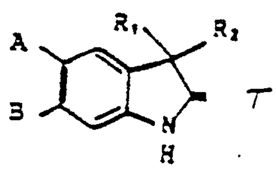 (XXX)

où

$R_1$ représente un atome d'hydrogène, un groupe alkyle en $C_1$-$C_6$, un groupe alcényle en $C_2$-$C_6$ ou un groupe cycloalkyle en $C_3$-$C_7$,

R2 représente un atome d'hydrogène, un groupe alkyle en $C_1$-$C_6$, alcényle en $C_2$-$C_6$ ou cyano, un groupe carbonyle substitué par un groupe hydroxy alkyle en $C_1$-$C_6$, alcoxy en $C_1$-$C_6$, amino, alkylamino en $C_1$-$C_6$, dialkylamino en $C_1$-$C_6$ ou hydrazino, ou forme en commun avec $R_1$ un groupe cycloalkylène en $C_3$-$C_8$, ou $R_1$ et $R_2$ forment en commun un groupe alkylidène en $C_2$-$C_6$ ou cycloalkylidène en $C_3$-$C_8$,

A représente un atome d'hydrogène, un groupe $NH_2$, $NO_2$ ou Py-X-CO-NH et

B représente un atome d'hydrogène, un groupe $NH_2$, $NO_2$ ou Py-X-CO-NH,

X représentant une valence libre, un groupe alkylène en $C_1$-$C_4$ ou un groupe vinylène et

Py représentant un reste 2-, 3-, ou 4-pyridyle qui porte éventuellement un atome d'oxygène sur l'hétéroatome du cycle et/ou peut être substitué par un ou plusieurs groupes alkyles en $C_1$-$C_6$, alcoxy en $C_1$-$C_6$, hydroxy, cyano ou nitro ainsi que par un atome d'halogène, et

T représentant l'oxygène ou le soufre,

étant entendu que A et B ne peuvent représenter à la fois que $NH_2$, et étant entendu que, lorsque $R_1$ représente l'hydrogène et $R_2$ représente l'hydrogène ou un groupe alkyle, ou lorsque $R_1$ représente l'hydrogène, un groupe alkyle ou un groupe alcényle et $R_2$ représente un groupe carbonyle substitué par un groupe hydroxy ou alcoxy, A ne peut représenter l'hydrogène ou un groupe amino lorsque B représente l'hydrogène ou un groupe amino.

Revendications pour l'Etat contractant suivant: AT

**1.** Composés de Formule I

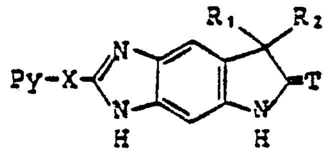

(I),

dans lesquels

- $R_1$ représente un atome d'hydrogène, un groupe alkyle en $C_1$-$C_6$, un groupe alcényle en $C_2$-$C_6$ ou un groupe cycloalkyle en $C_3$-$C_7$,
- $R_2$ représente un atome d'hydrogène, un groupe alkyle en $C_1$-$C_6$, un groupe alcényle en $C_2$-$C_6$ ou un groupe cyano, un groupe carbonyle substitué par un groupe hydroxy, alkyle, alcoxy, amino, alkylamino, dialkylamino ou hydrazino, les groupes alkyle mentionnés contenant 1 à 6 atomes de carbone, ou forme , en commun avec $R_1$, un groupe cycloalkylène en $C_3$-$C_8$, ou bien $R_1$ et $R_2$ forment en commun un groupe alkylidène en $C_2$-$C_6$ ou un groupe cycloalkylidène en $C_3$-$C_8$,
- X représente une valence libre, un groupe alkylène en $C_1$-$C_4$ ou le groupe vinylène,
- T représente un atome d'oxygène ou un atome de soufre,
- Py représente un reste 2-, 3-, ou 4-pyridyle, qui porte éventuellement un atome d'oxygène sur l'hétéroatome du cycle et/ou peut être substitué par un ou plusieurs groupes alkyle en $C_1$-$C_6$, alcoxy en $C_1$-$C_6$, hydroxy, cyano ou nitro, ainsi que par un atome d'halogène,

leurs tautomères et leurs sels d'acides minéraux et organiques physiologiquement acceptables.

**2.** Composés selon la Revendication 1, dans lesquels $R_1$ représente un atome d'hydrogène, un groupe méthyle, éthyle, propyle, 2-méthyl-propyle ou cyclopentyle, $R_2$ représente un atome d'hydrogène, un groupe méthyle, éthyle, éthoxycarbonyle ou hydrazinocarbonyle, $R_1$ et $R_2$ forment, en commun avec l'atome de carbone auquel ils sont liés, un cycle cyclopentane, ou en commun un reste isopropylidène, X représente une valence libre, un groupe méthylène, éthylène ou vinyle, T représente l'oxygène ou le soufre et Py représente un reste pyridylène-N-oxyde ou un reste pyridyle, qui peut être substitué une ou plusieurs fois par un groupe hydroxyle, méthoxy, méthyle ou un atome d'halogène, leurs tautomères et leurs sels physiologiquement acceptables.

**3.** Composés selon les Revendications 1 ou 2, dans lesquels T représente l'oxygène, X représente une valence libre, $R_1$ représente l'hydrogène ou un groupe méthyle, $R_2$ représente un groupe méthyle, éthyle, ou éthoxycarbonyle et Py représente un reste pyridine-N-oxyde ou un reste pyridyle, qui peut être substitué par un groupe hydroxy ou méthyle, leurs tautomères et leurs sels physiologiquement acceptables.

**4.** Composés selon la Revendication 1, choisis parmi les :

7,7-diméthyl-2-(4-pyridyl)-6,7-dihydro-3H,5H-pyrrolo[2,3-f]-benzimidazol-6-one
7,7-diméthyl-2-(4-N-oxy-pyridyl)-6,7-dihydro-3H,5H-pyrrolo-[2,3-f]benzimidazol-6-one
7-éthoxycarbonyl-7-méthyl-2-(4-pyridyl)-6,7-dihydro-3H,5H-pyrrolo[2,3-f]benzimidazol-6-one
7,7-diméthyl-2-(4-(2-méthyl-pyridyl))-6,7-dihydro-3H,5H-pyrrolo[2,3-f]benzimidazol-6-one

**5.** Procédé pour la préparation de composés de Formule I

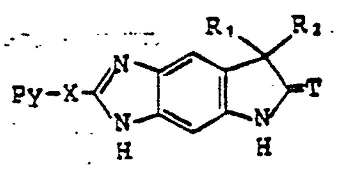

(I),

dans lesquels

- $R_1$ représente un atome d'hydrogène, un groupe alkyle en $C_1$-$C_6$, un groupe alcényle en $C_2$-$C_6$ ou un groupe cycloalkyle en $C_3$-$C_7$,

$R_2$ représente un atome d'hydrogène, un groupe alkyle en $C_1$-$C_6$, un groupe alcényle en $C_2$-$C_6$ ou un groupe cyano, un groupe carbonyle substitué par un groupe hydroxy, alkyle, alcoxy, amino, alkylamino, dialkylamino ou hydrazino, les groupes alkyle mentionnés contenant 1 à 6 atomes de carbone, ou forme , en commun avec $R_1$, un groupe cycloalkylène en $C_3$-$C_8$, ou bien $R_1$ et $R_2$ forment en commun un groupe alkylidène en $C_2$-$C_6$ ou un groupe cycloalkylidène en $C_3$-$C_8$,

X représente une valence libre, un groupe alkylène en $C_1$-$C_4$ ou le groupe vinylène,

T représente un atome d'oxygène ou un atome de soufre,

Py représente un reste 2-, 3-, ou 4-pyridyle, qui porte éventuellement un atome d' oxygène sur l'hétéroatome du cycle et/ou peut être substitué par un ou plusieurs groupes alkyle en $C_1$-$C_6$, alcoxy en $C_1$-$C_6$, hydroxy, cyano ou nitro, ainsi que par un atome d'halogène,

leurs tautomères et leurs sels d'acides minéraux et organiques physiologiquement acceptables, caractérisés en ce que, d'une manière connue en soi, on fait réagir

a) un composé de Formule XII

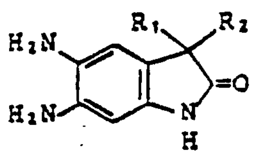

(XII),

dans laquelle $R_1$ et $R_2$ ont la signification mentionnée plus haut, avec un composé de Formule XIV

Py-X-CO-Y (XIV),

où Py et X ont la signification mentionnée plus haut et Y représente soit un atome d'hydrogène soit un reste facilement séparable, et on cyclise le composé obtenu en milieu acide, ou

b) on réduit un composé de Formule XIX

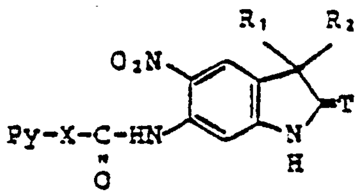

(XIX),

ou de Formule XX

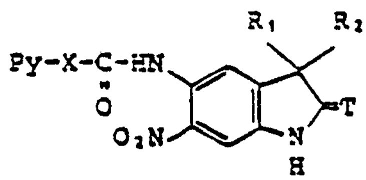

(XX),

où $R_1$, $R_2$, T, Py et X ont la signification mentionnée plus haut, et ensuite on cyclise ou

c) on acyle et cyclise un composé de Formule XXVI

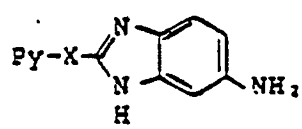

(XXVI)

avec un dérivé d'acide carboxylique réactif de Formule XXVII

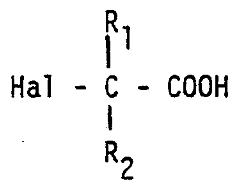 (XXVII)

où $R_1$, $R_2$, Py, X et Hal ont la signification mentionnée plus haut, ou on fait réagir
d) un composé de Formule XXVIII

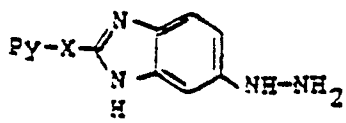 (XXVIII)

avec un dérivé d'acide carboxylique réactif de Formule XXIX

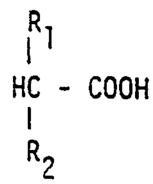 (XXIX)

en hydrazide correspondant, et on cyclise ensuite celui-ci dans des conditions alcalines, $R_1$, $R_2$, Py et X ayant la signification mentionnée plus haut, et ensuite, on transforme éventuellement les composés de Formule I obtenus en d'autres composés de Formule I, ainsi qu'éventuellement on transforme les composés avec des acides minéraux ou organiques en sels physiologiquement compatibles.

**6.** Médicament contenant un composé selon les Revendications 1, 2 ou 3, outre les supports et additifs habituels.

**7.** Utilisation des composés selon la Revendication 1, 2 ou 3, pour la preparation des médicaments pour la prophyllaxie ou le traitement des maladies cardiaques et circulatoires.

**8.** Composés de Formule XXX

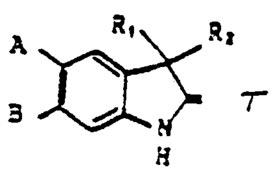 (XXX)

où
- $R_1$ représente un atome d'hydrogène, un groupe alkyle en $C_1$-$C_6$, un groupe alcényle en $C_2$-$C_6$ ou un groupe cycloalkyle en $C_3$-$C_7$,
- $R_2$ représente un atome d'hydrogène, un groupe alkyle en $C_1$-$C_6$, alcényle en $C_2$-$C_6$ ou cyano, un groupe carbonyle substitué par un groupe hydroxy alkyle en $C_1$-$C_6$, alcoxy en $C_1$-$C_6$, amino, alkylamino en $C_1$-$C_6$, dialkylamino en $C_1$-$C_6$ ou hydrazino, ou forme en commun avec $R_1$ un groupe cycloalkylène en $C_3$-$C_8$, ou $R_1$ et $R_2$ forment en commun un groupe alkylidène en $C_2$-$C_6$ ou cycloalkylidène en $C_3$-$C_8$,
- A représente un atome d'hydrogène, un groupe $NH_2$, $NO_2$ ou Py-X-CO-NH et
- B représente un atome d'hydrogène, un groupe $NH_2$, $NO_2$ ou Py-X-CO-NH,
- X représentant une valence libre, un groupe alkylène en $C_1$-$C_4$ ou un groupe vinylène et

Py représentant un reste 2-, 3-, ou 4-pyridyle qui porte éventuellement un atome d'oxygène sur l'hétéroatome du cycle et/ou peut être substitué par un ou plusieurs groupes alkyles en $C_1$-$C_6$, alcoxy en $C_1$-$C_6$, hydroxy, cyano ou nitro ainsi que par un atome d'halogène, et

T représentant l'oxygène ou le soufre,

étant entendu que A et B ne peuvent représenter à la fois que $NH_2$, et étant entendu que, lorsque $R_1$ représente l'hydrogène et $R_2$ représente l'hydrogène ou un groupe alkyle, ou lorsque $R_1$ représente l'hydrogène, un groupe alkyle ou un groupe alcényle et $R_2$ représente un groupe carbonyle substitué par un groupe hydroxy ou alcoxy, A ne peut représenter l'hydrogène ou un groupe amino lorsque B représente l'hydrogène ou un groupe amino.

**9.** Procédé pour la préparation de composés de Formule XXX

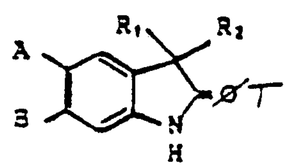

(XXX)

où

$R_1$ représente un atome d'hydrogène, un groupe alkyle en $C_1$-$C_6$, un groupe alcényle en $C_2$-$C_6$ ou un groupe cycloalkyle en $C_3$-$C_7$,

$R_2$ représente un atome d'hydrogène, un groupe alkyle en $C_1$-$C_6$, alcényle en $C_2$-$C_6$ ou cyano, un groupe carbonyle substitué par un groupe hydroxy, alkyle en $C_1$-$C_6$, alcoxy en $C_1$-$C_6$, amino, alkylamino en $C_1$-$C_6$, dialkylamino en $C_1$-$C_6$ ou hydrazino ou forme en commun avec $R_1$ un groupe cycloalkylène en $C_3$-$C_8$ où $R_1$ et $R_2$ forment en commun un groupe alkylidène en $C_2$-$C_6$ ou cycloalkylidène en $C_3$-$C_8$,

A représente un atome d'hydrogène, un groupe $NH_2$, $NO_2$ ou Py-X-CO-NH et

B représente un atome d'hydrogène, un groupe $NH_2$, $NO_2$ ou Py-X-CO-NH,

X représentant une valence libre, un groupe alkylène en $C_1$-$C_4$ ou un groupe vinylène et

Py représentant un reste 2-, 3- ou 4-pyridyle qui porte éventuellement un atome d'oxygène sur l'hétéroatome du cycle et/ou peut être substitué par un ou plusieurs groupes alkyles en $C_1$-$C_6$, alcoxy en $C_1$-$C_6$, hydroxy, cyano ou nitro ainsi que par un atome d'halogène, et

T représentant l'oxygène ou le soufre,

étant entendu que A et B ne peuvent représenter à la fois que $NH_2$, et étant entendu que, lorsque $R_1$ représente l'hydrogène et $R_2$ représente l'hydrogène ou un groupe alkyle, ou lorsque $R_1$ représente l'hydrogène, un groupe alkyle ou un groupe alcényle et $R_2$ représente un groupe carbonyle substitué par un groupe hydroxy ou alcoxy, A ne peut représenter l'hydrogène ou un groupe amino lorsque B représente l'hydrogène ou un groupe amino, caractérisés en ce que, d'une manière connue en soi,

a) on transforme un composé de Formule IX

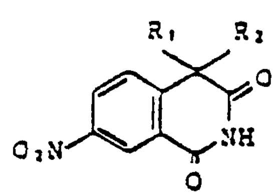

(IX),

où $R_1$ et $R_2$ ont la signification mentionnée plus haut, par décomposition de Hoffmann, en composés de Formule Générale X

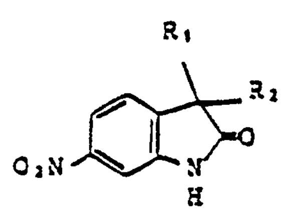

(X),

et éventuellement, on réduit le groupe nitro en groupe amino, ou
b) on transforme des dérivés de l'acide 2,4-diaminophénylacétique à substitution appropriée, par fermeture du cycle, en composés de Formule Générale XI,

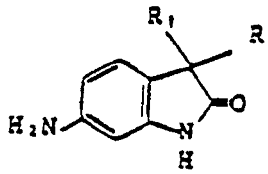

(XI),

où $R_1$ et $R_2$ ont la signification mentionnée plus haut, où
c) on acétyle un composé de Formule Générale XI

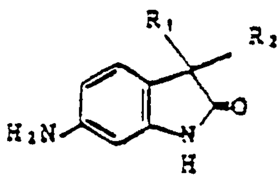

(XI),

où $R_1$ et $R_2$ ont la signification mentionnée plus haut, on effectue une nitration en position 5, sépare le groupe de protection et on réduit le groupe nitro en un composé de Formule Générale XII

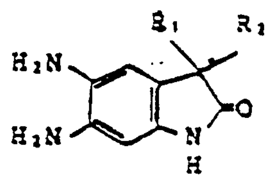

(XII),

ou
d) on alkyle un composé de Formule Générale XIII

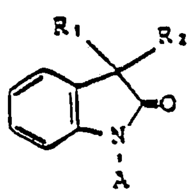

(XIII),

où $R_1$ et $R_2$ représentent l'hydrogène et A un groupe de protection, avec un composé de Formule Générale III

$$R_1\text{-}Z \qquad \text{(III)},$$

ou IV

$$Z\text{-}R_3\text{-}Z \qquad \text{(IV)},$$

où $R_1$ a la signification mentionnée plus haut, $R_3$ représente un groupe alkylène en $C_2$-$C_6$ et Z représente un groupe séparable, en composés de Formule Générale XIII, où $R_1$ et $R_2$ ont la signification mentionnée plus haut, mais ne peuvent représenter l'hydrogène, on sépare le groupe de protection A, et ensuite on effectue la nitration des composés obtenus dans lesquels A représente l'hydrogène, on réduit le groupe nitro en groupe amino, on acétyle le groupe amino, puis on effectue

la nitration en position 6, on sépare le groupe de protection de la fonction amino, et on transforme le groupe nitro par réduction en un composé de Formule XII

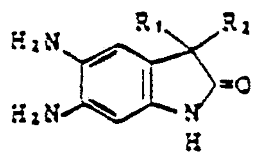 (XII),

où $R_1$ et $R_2$ ont la signification mentionnée plus haut, ou
e) on fait réagir un composé de Formule XII

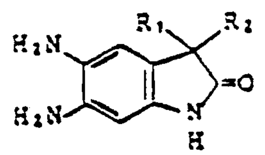 (XII),

où $R_1$ et $R_2$ ont la signification mentionnée plus haut, avec un composé de Formule XIV

Py-X-CO-Y (XIV),

où Py et X ont la signification mentionnée plus haut et Y représente soit l'hydrogène soit un reste facilement séparable, ou
f) on fait réagir un composé de Formules XV ou XVI

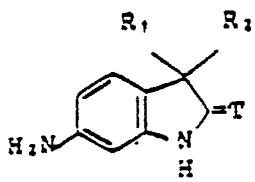 (XV) 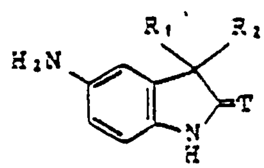 (XVI),

où $R_1$, $R_2$ et T ont la signification mentionnée plus haut, avec un composé de Formule Générale XIV

Py-X-CO-Y (XIV),

où Py et X ont la signification mentionnée plus haut et Y représente un reste facilement séparable, en composés de Formules XVII ou XVIII

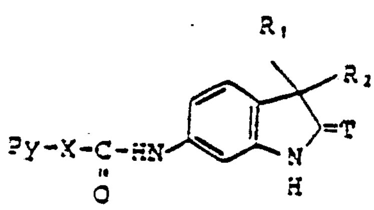 (XVII), 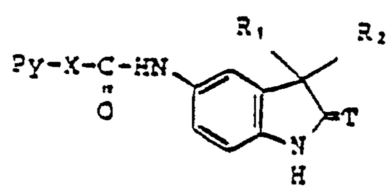 (XVIII),

et on effectue éventuellement la nitration de ces composés en positions 5 ou 6, et éventuellement, on réduit ensuite le groupe nitro en groupe amino, ou
g) on effectue la nitration d'un composé de Formule Générale XXIII

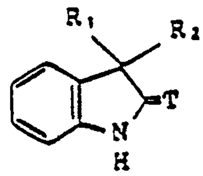

(XXIII),

où $R_1$, $R_2$ et T ont la signification mentionnée plus haut, en position 5, et éventuellement ensuite on réduit le groupe nitro en fonction amino, où

h) dans un composé de Formules Générales XV ou XVI

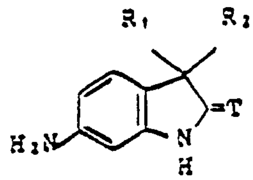

(XV)

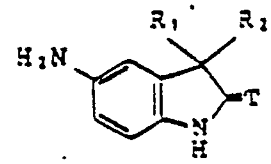

(XVI),

où $R_1$, $R_2$ et T ont la signification mentionnée plus haut, on acétyle le groupe amino en positions 6 ou 5, on effectue la nitration en positions 5 ou 6, on sépare le groupe de protection et on transforme le composé ainsi obtenu, avec un composé de Formule Générale XIV

Py-X-CO-Y (XIV),

où Py et X ont la signification mentionnée plus haut et Y représente soit l'hydrogène, soit un groupe facilement séparable, en composés de Formules Générales XIX ou XX

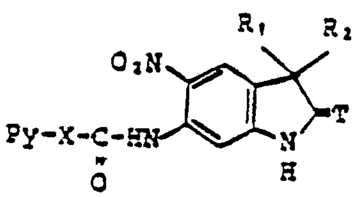

(XIX),

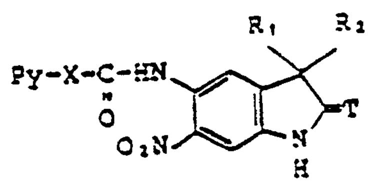

(XX),